# EUROPEAN PATENT APPLICATION

(11) **EP 1 932 907 A1**
(43) Date of publication of application: **18.06.2008**
(21) Application number: 06125973.5
(22) Date of filing: 12.12.2006
(51) Int. Cl.: C12N 9/50, C07K 14/435

(54) **Crystal structure of the catalytic domain of tolloid-like protease 1 and uses thereof**

(71) Applicant: Novartis AG, 4002 Basel (CH)
(72) Inventor: Gerhartz, Bernd, 79539 Lörrach (DE); Mac Sweeney, Aengus, 4059 Basel (CH); Bodendorf, Ursula, 4055 Basel (CH); Hommel, Ulrich, 79379 Müllheim (DE)
(74) Representative: Bourgarel, Denis Jacques Marie

(57) **Abstract**

The present invention relates to methods for cloning, expression, purification and crystallization of the catalytic domain of TLL1 (TLL1cd). The present inventors have solved the first human TLL1 catalytic domain structure alone and in complex with an inhibitor at a resolution of 1.80 A and 1.46 A, respectively. Surprisingly, the obtained structures reveal an unexpected arginine in the S1' binding pocket, which is displaced upon binding of the inhibitor. An other surprising and unexpected property of the crystal structures is that there is a disordered cysteine-rich loop in the S1 and S1' region of the active site, which loop is of great importance for ligand design. The present invention hence relates to a crystal comprising the tolloid-like protein 1 catalytic domain or a mutant thereof.

## Description

### Field of the Invention

The present invention relates to the crystal form of the catalytic domain of tolloid-like protease 1, the methods of such crystals and their use in drug discovery. The present invention also relates to the use of the three-dimensional structure of the catalytic domain of tolloid-like protease 1 for the design and/or identification of ligands (agonists and/or antagonists).

### Background of the Invention

In recent years, it has become evident that members of the M12a metalloproteinase family (bone morphogenic protein - BMP-1, mammalian tolloid - mTld, mammalian tolloid-like protein 1 - TLL1 and mammalian tolloid-like protein 2 - TLL2) are key players in morphogenesis through their ability to control and synchronize the processing of multiple extracellular substrates (Scott, et al 1999). Tolloid-like proteinases (also called Procollagen C-proteinases, PCP) trigger collagen fibril formation in the extracellular matrix by cleaving the C-propeptide regions from the major fibrillar procollagens (1, 11, and 111) (Kessler, et al 1996). In addition, these enzymes maturate the precursor form of lysyl oxidase (prolysyl oxidase) (Uzel, et al 2001), an enzyme responsible for the stabilization of collagen and elastin networks through the formation of covalent cross-links which seems to be essential for elastogenesis. BMP-1 and mTld result from the alternative splicing of the BMP1 gene and differ mainly in the number of CUB domains at the C-terminus of the protein, while the sequence identities of the catalytic domains of BMP-1, TLL1 and TLL2 are above 95 %.

A more recently reported target of these proteinases is pro-myostatin. Myostatin is a transforming growth factor β family member that is essential for proper regulation of skeletal muscle growth (McPherron and Lee 1997, McPherron, Lawler and Lee 1997). Myostatin knockout mice appear healthy and display a two- to threefold increase in skeletal muscle mass and decreased fat levels compared to their wild type littermates (Lin, et al 2002). The myostatin precursor requires two proteolytic steps for its activation, the second of which releases the active myostatin peptide (Wolfman, et al 2003) and has been linked to a tolloid-like protease. The necessity of this cleavage for the myostatin activation was confirmed by injecting a cleavage resistant variant into adult mice, which resulted in a muscle mass increase. Taken together, these results suggest that reducing myostatin processing through inhibition of tolloid-like proteases is a promising therapeutic approach for muscle-wasting related disorders, see also WO2004/024090 and WO2004/024092.

No crystal structure of a TLL family member is yet available. The closest homologue of known structure is the digestive enzyme astacin from the crayfish, which has a sequence identity of 36% with the catalytic domain of TLL1. Crystal structures of astacin in the absence of inhibitor (PDB code 1AST) and in complex with a hydroxamic acid-based (PDB code 1QJJ) and a phosphonic pseudopeptide (PDB code 1QJ1) inhibitor have been reported. Astacin displays a different substrate specificity to TLL, particularly in its preference for alanine or serine instead of aspartic acid for the P1' residue. For this reason, the astacin structure is considered to be too dissimilar to the tolloid-like proteases to be used as a basis for CADD.

In addition, it is yet not known exactly which of the TLL family members plays the most important role in pro-myostatin activation and which, if any, must be considered an anti-target. Potent inhibitors with medium or high selectivity for specific TLL family members would be of great use not only as starting points for a chemical program but also as tools to determine the desired inhibitor selectivity profile. Because of the high sequence similarity between the TLLs, especially in the active site region, structural information of inhibitor complexes will be useful for identifying any conformational differences that could be probed in order to gain inhibitor selectivity.

There is thus a need in the art for information design and/or identification of ligands (agonists and/or antagonists). Moreover, in view of the sequence identities between the catalytic domains of BMP1, TLL1 and TLL2 which are above 95%, the crystal structure of TLL1 provides useful information regarding opportunities to selectively inhibit members of this class of proteinases.

The present invention thus satisfies these needs by providing a surprising and advantageous crystal form of the catalytic domain of tolloid-like protease 1.

### Summary of the Invention

In order to fulfill the needs identified herein-above, the present inventors have established methods for cloning, expression, purification and crystallization of the catalytic domain of TLL1. In addition, the present inventors have solved the first human TLL1 catalytic domain structure alone and in complex with an inhibitor at a resolution of 1.80 A and 1.46 A, respectively. Surprisingly, the obtained structures reveal an unexpected arginine in the S1' binding pocket, which is displaced upon binding of the inhibitor. An other surprising and unexpected property of the crystal structures is that there is a disordered cysteine-rich loop in the S1 and S1' region of the active site, which loop is of great importance for ligand design. The present invention hence relates to a crystal comprising the tolloid-like protein 1 catalytic domain (TLL1cd) or a mutant thereof.

In one particular embodiment of the invention, said tolloid-like protein 1 is human TLL1cd (SEQ ID NO: 1). In another embodiment, the TLL1cd crystal has a cell size of 41 x 48 x 59 Angstroms and/or a three-dimensional structure characterized by the structure coordinates of Table 3.
In a further embodiment, the TLL1cd crystal of the invention further comprises a co-crystallised ligand.

Our invention also encompasses a method for making a crystal of the TLL1cd protein, said method comprising the steps of: (a) expressing a construct comprising a nucleotide sequence encoding for TLL1 cd; (b) purifying a sufficient amount of the TLL1cd protein produced at step (a) under appropriate conditions for crystallization; and, (c) crystallizing the TLL1cd protein purified at step (b). This method can further comprise the step of adding a ligand to the purified protein of step (b).

Another embodiment of our invention is a computer readable medium comprising data storage material encoded with computer readable data wherein said data comprises the structure coordinates of a TLL1cd crystal.

Moreover, an embodiment of our invention is the use of a TLL1cd crystal for the generation of crystal structure data, and the use of said generated crystal data in a method of identifying a ligand that binds to TLL1cd comprising the steps of: (i) using the three dimensional structure data generated according to our invention to select and/or design a potential ligand that binds to TLL1cd, (ii) identifying among the potential ligand selected in step (i), those ligands that bind to TLL1cd in an *in vitro, in vivo* or cell-based assay.

Our invention further encompasses a method for determining the three-dimensional structure of a complex comprising the TLL1cd protein, a fragment or homologue thereof comprising: (a) obtaining x-ray diffraction data for crystals of the complex, (b) utilizing the atomic coordinates generated according to our invention to define the three-dimensional structure of the complex.

In addition our invention further encompasses the use of an antagonist of TLL1cd for the manufacture of a medicament to treat muscle-wasting related disorders, and/or a method of treating a patient in need thereof with said antagonist of TLL1cd.

### Legends of the Figures

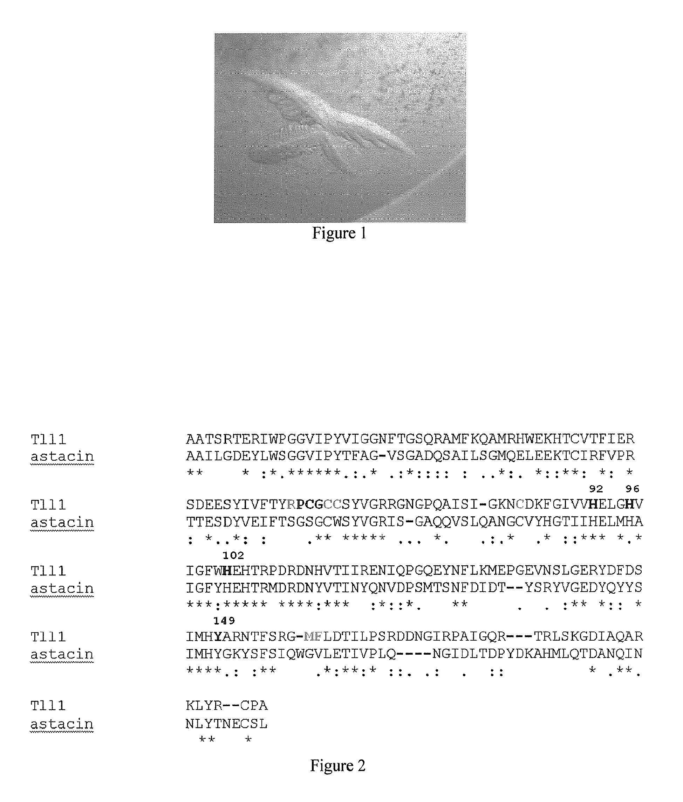

**Figure 1****:** Crystals of TLL1cd grown in 25% PEG3350, 0.2M Li2SO4, 0.1M Tris pH 8.0. The crystals diffract well despite their clustering and irregular appearance. The TLL1cd structure was solved by molecular replacement using the coordinates of crayfish astacin (pdb code 1QJJ) (Bode, et al 1992). The refined structure of TLL1cd was subsequently used to solve the structure of TLL1cd in complex with ligand. For ligand-free TLL1cd, 186 of the 201 amino acids could be traced in the electron density map and the backbone atoms of a further eight residues could be built. For the higher resolution ligand complex, 193 residues could be traced and the backbone atoms of a further five residues could be built.
**Figure 2**: A structure-based alignment of the TLL1cd and astacin sequences. Tyr149 and the three zinc-binding histidines are shown in bold. Arg176, which lies in the S1' pocket, is highlighted. Insertions are marked by a hyphen. Identical residues are underscored by an asterisk, and highly similar or similar residues are underscored by a colon or period, respectively.

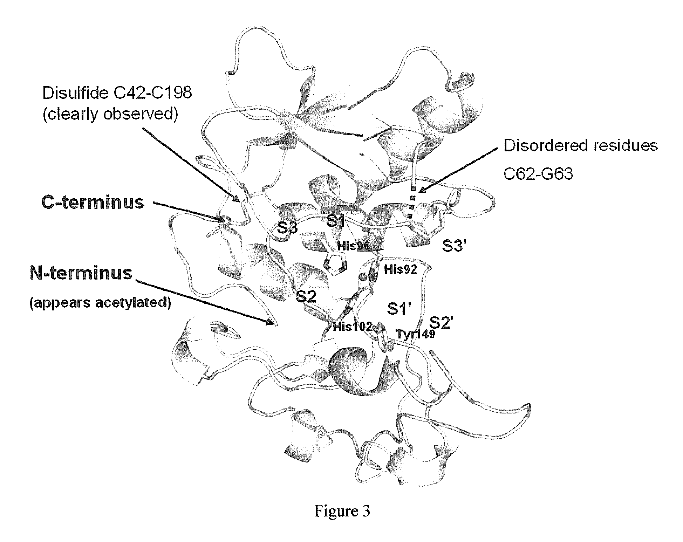

**Figure 3****:** Ribbon diagram of TLL1cd. The three zinc-binding histidines and the catalytic Glu93 are shown as stick models in grey, with oxygen and nitrogen atoms in red and blue, respectively. Disulfide bonds are shown in yellow. Disordered residues are indicated by a dotted line.

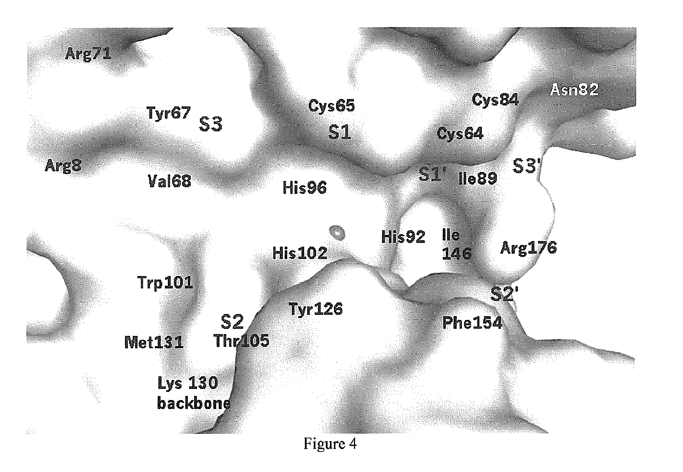

**Figure 4**: The active site of TLL1 cd is shown as a solvent-accessible-surface superimposed with key residues and binding pockets labeled. The zinc atom is shown as a sphere.

### Detailed Description of the Invention

The nucleotide and amino acid sequences of full-length sequence of TLL1cd are known (Scott, et al 1999). The amino acid sequence of human TLL1cd is: maatsrteriwpggvipyviggnftgsqramfkqamrhwekhtcvtfiersdeesyivftyrpcgccsyvgrrgngpqaisigknc dkfgivvhelghvigfwhehtrpdrdnhvtiireniqpgqeynflkmepgevnslgerydfdsimhyarntfsrgmfldtilpsrddn girpaigqrtrlskgdiaqarklyrcpa (SEQ ID NO:1).

The present invention provides TLL1cd in crystallized form. In particular, it provides crystals of TLL1cd in complex with ligands.

The crystals according to the invention preferably belong to space group P1 (triclinic). The crystals may also belong to the space group P2(1) and have 1 molecule per asymmetric unit.

Depending on the conditions used for crystallization, the parameters characterising the unit cell may vary with a limited range, at least within the range of the resolution. The resolution of the X-ray crystallography is typically ≤ 5 Angstroms and by means of the purification method described therein, it is possible to provide crystals of such high internal order that a resolution of ≤ 2A can be achieved. In particular, the soaking of one inhibitor induced a change of 6 degrees in the beta angle of the unit cell (from 90 degrees to 96 degrees).

The term "unit cell" according to the invention refers to the basic shape block. The entire volume of a crystal may be constructed by regular assembly of such blocks. Each unit cell comprises a complete representation of the unit of pattern, the repetition of which builds up the crystal.

The term "space group" according to the invention refers to the arrangement of symmetry elements of a crystal.

The term "structure coordinates" or "atomic coordinates" refers to mathematical coordinates derived from the mathematical equations related to the pattern obtained on diffraction of a monochromatic beam of X-rays by the atoms (scattering centers) of a crystal comprising TLL1cd. The diffraction data are used to calculate an electron density map of the repeating unit of the crystal. The electron density maps are used to establish the positions of the individual atoms within the unit cell of the crystal. The structure coordinates of hTLL1cd can be found in Table 3.

In a preferred embodiment of the invention, said TLL1cd is the catalytic domain of hTLL1 and comprises the sequence of SEQ ID NO: 1, or is a fragment, a homologue thereof or a mutant thereof.

A "fragment" of TLL1cd comprises more than 50% consecutive amino acids of the reference sequence of the TLL1cd according to SEQ ID NO:1, more preferably at least 80%, most preferably at least 90% of SEQ ID NO.1.

As used herein, a "homologue" of that sequence shares at least 70% identity, preferably 80% identity, more preferably 90%, and even more preferably 95% identity with the corresponding SEQ ID NO:1 when performing optimal alignment. Optimal alignment of sequences for determining a comparison window may be conducted by the local homology algorithm of Smith and Waterman (J. Theor. Biol., 91 (2) pgs. 370-380 (1981), by the homology alignment algorithm of Needleman and Wunsch, J. Miol. Biol., 48(3) pgs. 443-453 (1972), by the search for similarity via the method of Pearson and Lipman, PNAS, USA, 85(5) pgs. 2444-2448 (1988) or by computerized implementations of these algorithms (GAP, BESTFIT, FASTA and TFASTA in the Wisconsin Genetics Software Package Release 7.0, Genetic Computer Group, 575, Science Drive, Madison, Wisconsin).
The best alignment (i.e., resulting in the highest percentage of identity over the comparison window) generated by the various methods is selected for determining percentage identity.

The term "ligand" according to the invention, refers to a molecule or group of molecules that bind to one or more specific sites of TLL1cd. A ligand according to the invention can be an agonist or an antagonist. In addition, ligands according to the invention are preferably low molecular weight molecules.

The term "low molecular weight molecules" according to the invention refers to preferably organic compounds generally having a molecular weight less than about 1000, more preferably less than about 500.

More preferably, said ligand inhibits TLL1cd biological activity. A compound is considered as an TLL1 inhibitor if it has an IC50 ranging from 0.01 nM to 1.0 □M.

TLL1 inhibitor can also be a "peptide" or a "peptide derivative", which terms are intended to embrace a "peptidomimetic" or "peptide analogue" which complement the three-dimensional structure of the binding pocket of TLL 1cd or can be designed with improved physical or chemical properties to bind with the three-dimensional binding pocket of the TLL1cd as provided in the present invention.

The term "mutant" refers to a mutated sequence of SEQ ID NO:1 by deletion, insertion or preferably replacement of one or more selected amino acids, provided that such mutant sequence shares at least 90% identity, more preferably 95%, and even more preferably 99% identity with the corresponding fragment sequence of SEQ ID NO:1 when performing optimal alignment. Preferably, a mutant of TLL1 cd is a single mutant of TLL1cd.

Methods for the preparation of protein mutants are commonly known in the art. For example, TLL1cd mutants may be prepared by expression of TLL1cd DNA previously modified in its coding region by oligonucleotide directed mutagenesis.

As used herein, the term "binding pocket" refers to the region of TLL1cd that, as a result of its shape and physico-chemical properties favorably associates with another chemical entity or compound.

The TLL1cd protein to be used for crystallization may be biologically active or inactive. Such ability may be determined by morphological, biochemical or viability analysis well-known in the art.

Expression of recombinant TLL1cd or fragment thereof is achievable in eukaryotic or prokaryotic systems or *in vitro* expression systems.

According to a preferred embodiment, TLL1cd is bound to at least one ligand at any step prior to crystallization.

TLL1cd may be expressed as a fusion protein, e.g. a glutathione-S-transferase (GST) or histidine-tagged fusion protein. If desired, the fusion partner is removed before crystallization.

For carrying out the step of crystallization of the method for making a crystal, various methods can be used including vapour diffusion, dialysis or batch crystallization according to methods known in the art ("Crystallization of Biological Macromolecules", A. McPherson, Cold Spring Harbor Laboratory Press, Cold Spring Harbor, New York, USA).

In vapour diffusion crystallization, a small volume (i.e., a few microliters) of protein solution is mixed with a solution containing a precipitant. This mixed volume is suspended over a well containing a small amount, i.e. about 1 ml, of precipitant. Vapour diffusion from the drop to the well will result in crystal formation in the drop.

The dialysis method of crystallization utilizes a semipermeable size-exclusion membrane that retains the protein but allows small molecules (i.e. buffers and precipitants) to diffuse in and out. In dialysis, rather than concentrating the protein and the precipitant by evaporation, the precipitant is allowed to slowly diffuse through the membrane and reduce the solubility of the protein while keeping the protein concentration fixed.

The batch method generally involves the slow addition of a precipitant to an aqueous solution of protein until the solution just becomes turbid, at this point the container can be sealed and left undisturbed for a period of time until crystallization occurs. In the batch technique the precipitant and the target molecule solution are simply mixed. Supersaturation is achieved directly rather than by diffusion. Often the batch technique is performed under oil. The oil prevents evaporation and extremely small drops can be used. For this, the term "microbatch" is used. A modification of this technique is not to use paraffin oil (which prevents evaporation completely) but rather use silicone oil or a mixture of silicone and paraffin oils so that a slow evaporation is possible.

The claimed invention can encompass any and all methods of crystallization. One skilled in the art can choose any of such methods and vary the parameters such that the chosen method results in the desired crystals.

One preferred method of crystallization of TLL1 cd involves mixing a TLL1cd solution with a "reservoir buffer". For crystal formation, the concentration of the precipitating agent in the mixture has to be increased, e.g. by addition of precipitating agent, for example by titration, or by allowing the concentration of precipitating agent to balance by diffusion between the crystallization buffer and a reservoir buffer (not necessarily the same as the original reservoir buffer). Under suitable conditions such diffusion of precipitating agent occurs along the gradient of precipitating agent, e.g. from the reservoir buffer having a higher concentration of precipitating agent into the crystallization buffer having a lower concentration of precipitating agent. Diffusion may be achieved e.g. by vapour diffusion techniques allowing diffusion of water in the common gas phase. Known techniques are e.g. vapour diffusion methods, such as the "hanging drop" or the "sitting drop" method. In the vapour diffusion method a drop of crystallization buffer containing the protein is hanging above or sitting beside a much larger pool of reservoir buffer. Alternatively, the balancing of the precipitating agent can be achieved through a semipermeable membrane that separates the crystallization buffer from the reservoir buffer and prevents dilution of the protein into the reservoir buffer.

Formation of TLL1cd can be achieved under various conditions which are essentially determined by the following parameters: pH, presence of salts and additives, precipitating agent, protein concentration and temperature. The pH may range, for example, from about 4.0 to 9.0.

In another specific embodiment, the invention relates to a method for making a co-crystal of TLL1cd in complex with a ligand.

The crystal form of TLL1cd crystal may also be used for exchanging the ligand by soaking compounds of interest, for example, for compound optimization, or for the discovery of novel scaffolds in fragment based screening approaches.

The present invention also relates to a crystal comprising the TLL1 cd obtainable by the above methods.

Structure coordinates of a crystalline composition of this invention may be stored in a machine-readable form on a machine-readable storage medium, e.g. a computer hard drive, diskette, DAT tape, etc., for display as a three-dimensional shape or for other uses involving computer-assisted manipulation of, or computation based on, the structural coordinates or the three-dimensional structures they define. For example, data defining the three dimensional structure of a protein of the TLL family, or portions or structurally similar homologues of such proteins, may be stored in a machine-readable storage medium, and may be displayed as a graphical three-dimensional representation of the protein structure, typically using a computer capable of reading the data from said storage medium and programmed with instructions for creating the representation from such data.

According to the present invention, a three-dimensional TLL1cd model is obtainable from a TLL1cd crystal comprising the TLL1cd, fragment or homologue thereof.

The present invention also relates to a computer readable medium having stored a model of the TLL1cd crystal structure. In a preferred embodiment, said model is built from all or a selected portion of it, of the atomic coordinates of Table 3 derived from the X-ray diffraction data.

By "selected portion", it is meant the structure coordinates of at least 10 consecutive amino acids shown in Table 3 and preferably at least 50 amino acids, and more preferably at least 100 consecutive amino acids.

In a preferred embodiment, a selected portion corresponds to the structure coordinates of the amino acids forming at least the substrate binding pocket of TLL1cd, more preferably the catalytic site of TLL1.

The knowledge obtained from the three-dimensional model of TLL1cd can be used in various ways. For example, it can be used to identify chemical entities, for example, small organic and bioorganic molecules such as peptidomimetics and synthetic organic molecules that bind to TLL1 and preferably block or prevent a TLL1-mediated or associated process or event, or that act as TLL1 agonists. Furthermore, this information can be used to design and prepare TLL1cd mutants, e.g. mutants with altered catalytic activity, model the three-dimensional structure and solve the crystal structure of proteins, such as TLL1cd homologues, TLL1cd mutants or TLL1cd co-complexes, involving e.g. molecular replacement or homology modeling.

The term "molecular replacement" refers to a method that involves generating a preliminary structural model of a crystal whose structural coordinates are unknown, by orienting and positioning a molecule whose structural coordinates are known, e.g., the TLL1cd coordinates within the unit cell of the unknown crystal, so as to best account for the observed diffraction pattern of the unknown crystal. Phases can then be calculated from this model, and combined with the observed amplitudes to give an approximated Fourier synthesis of the structure whose coordinates are unknown. This in turn can be subject to any of the several forms of refinement to provide a final accurate structure of the unknown crystal. Using the structural coordinates provided by this invention, molecular replacement may be used to determine the structural coordinates of a crystalline co complex, unknown ligand, mutant, or homolog, or of a different crystalline form of TLL1 cd. Additionally, the claimed crystal and its coordinates may be used to determine the structural coordinates of a chemical entity that associates with TLL1cd.

"Homology modeling" according to the invention involves constructing a model of an unknown structure using structural coordinates of one or more related proteins, protein domains and/or one subdomains such as TLL1cd. Homology modeling may be conducted by fitting common or homologous portions of the protein or peptide whose three dimensional structure is to be solved to the three dimensional structure of homologous structural elements. Homology modeling can include rebuilding part or all of a three dimensional structure with replace of amino acids or other components by those of the related structure to be solved.

Based on the three-dimensional structure of TLL1 cd as provided in the present invention and using the atomic coordinates of Table 3, or a selected portion of it, the effects of site-specific mutations can be predicted. More specifically, the structural information provided herein permits the identification of desirable sites for amino acid modification, particularly amino acid mutation resulting in substitutional, insertional or deletional variants. Such variants may be designed to have special properties, particularly properties distinct from wild-type TLL1cd, such as altered catalytic activity. Substitutions, deletions and insertions may be combined to arrive at a desired variant. Such variants can be prepared by methods well-known in the art, e.g. starting from wild-type TLL1 cd or by de novo synthesis.

The TLL1cd structural information provided herein is useful for the design of ligands which are capable of selectively interacting with TLL1 cd, but not other proteases other than TLL1cd, and thereby specifically modulating the biological activity of TLL1 and not other non-TLL1 proteases.

Chemical entities that have a surface that mimics the accessible surface of the binding pocket of TLL1cd can be constructed by those skilled in the art. By way of example, the skilled artisan can screen three-dimensional structural databases of compounds to identify those compounds that position appropriate functional groups in similar three dimensional structural arrangement, then build combinatorial chemistry libraries around such chemical entities to identify those with high affinity to the binding pocket of TLL1cd.

In a specific embodiment of the invention, a cell-based assay is designed to identify ligands which inhibit the biological activity of TLL1 cd.

Ligands, such as small molecular weight compounds can be identified from screening compound databases or libraries and using a computational means to form a fitting operation to a binding site on the TLL1cd. The three dimensional structure of TLL1cd as provided in the present invention by the structure coordinates of Table 3 or a selected portion of it, can be used together with various docking programs.

The potential inhibitory or binding effect of a chemical entity on TLL1cd may be analyzed prior to its actual synthesis and testing by the use of computer-modeling techniques. If the theoretical structure of the given chemical entity suggests insufficient interaction and association between it and TLL1cd, the need for synthesis and testing of the chemical entity is obviated. However, if computer modeling indicates a strong interaction, the molecule may then be synthesized and tested for its ability to bind to TLL1cd. Thus, expensive and time-consuming synthesis of inoperative compounds may be avoided.

This *"in silico"* analysis may begin by visual inspection of, for example, the binding pocket on a computer screen based on the structural coordinates of Table 3 in whole or in part. Selected fragments or chemical entities may then be positioned in a variety of orientations, or "docked," within the binding pocket of TLL1cd. Docking may be accomplished using software such as Quanta and SYBYL, followed by energy minimization and molecular dynamics with standard molecular mechanics force fields, such as CHARMM and AMBER. Specialized computer programs may be of use for selecting interesting fragments or chemical entities. These programs include, for example, GRID, available from Oxford University, Oxford, UK; 5 MCSS or CATALYST, available from Molecular Simulations, Burlington, MA; AUTODOCK, available from Scripps Research Institute, La Jolla, CA; DOCK, available from University of California, San Francisco, CA, and XSITE, available from University College of London, UK.

Preferred is a method for designing a TLL1 inhibitor which interacts at the substrate binding site of TLL1 cd or any other binding sites. One approach enabled by this invention is the use of the structure coordinates of TLL1cd to design chemical entities that bind to or associate with TLL1 cd and alter the physical properties of the chemical entities in different ways. Thus, properties such as, for example, solubility, affinity, specificity, potency, on/off rates, or other binding characteristics may all be altered and/or maximized. One may design desired chemical entities by probing a TLL1cd comprising the catalytic domain with a library of different entities to determine optimal sites for interaction between candidate chemical entities and TLL1cd. For example, high-resolution X-ray diffraction data collected from crystals saturated with solvent allows the determination of where each type of solvent molecule adheres. Small molecules that bind tightly to those sites can then be designed and synthesized and tested for the desired activity. Once the desired activity is obtained, the molecules can be further altered to maximize desirable properties.

Once a compound has been designed or selected by the above methods, the efficiency with which that compound may bind to TLL1cd may be tested and modified for the maximum desired characteristic(s) using computational or experimental evaluation. Various parameters can be maximized depending on the desired result. These include, but are not limited to, specificity, affinity, on/off rates, hydrophobicity, solubility, and other characteristics readily identifiable by the skilled artisan.

In a preferred embodiment, the structure coordinates of Table 3 of TLL1cd is used in the above computer-based design step.

The invention further relates to a method for selecting a ligand that binds to TLL1cd, comprising:
a. co-crystallizing or incubating a candidate compound or mix of compounds with TLL1cd under appropriate conditions,
b. determining by X-ray or NMR methods the amino acids of TLL1cd which interacts with the candidate compound,
c. selecting the compound which interacts at least with one or more amino acids of the binding pocket.

For carrying out step b., mapping of the binding site of ligand is usually performed by recording NMR spectra with and without the candidate compound, and identifying those resonances of the protein that are affected by ligand binding. This requires assignment of the protein resonance prior to the analysis, or comparison with the pattern of chemical shift changes that occur upon binding of ligands with known binding sites. Alternatively, competition experiments using said ligands with known binding sites can yield equivalent information.

The present invention further provides methods to design novel ligands of TLL1 cd, using fragment linking approaches. Compounds binding to different binding regions of TLL1 cd are first selected. The ligands are linked together based on the spatial orientation, so that the designed novel compounds fits within the two binding sites.

The invention thus relates to a method to design ligands to TLL1cd, wherein said method comprises:
a. providing a first ligand that binds to one or more amino acids of a first binding region of TLL1cd,
b. providing a second ligand that binds to one or more amino amino acids of a second binding region of TLL1cd, and,
c. linking said first ligand to said second ligand to design a ligand that binds to the first and second binding pockets of TLL1cd.

The selection of an appropriate linking group is made by maintaining the spatial orientation of the ligands to one another and to TLL1cd based upon principles of bond angle and bond length information well known in the organic chemical art.

In addition, antagonists of TLL1 cd and the other members of the tolloid family can be used to treat patients, or for the manufacture of a medicament, to treat muscle-wasting related disorders. Examples of muscle-wasting related disorders are muscular dystrophy, catechxia and age-related sarcopenia. (Bogdanovich,et al 2002). The tolloid family of proteases is also linked to fibrosis, therefore antagonists of these enzymes could be used to treat excessive scar formation and other fibrosis-related diseases. The tolloids are also responsible for the activation of lysyl oxidase, a target for the prevention of metastasis.

The following examples serve to illustrate the present invention but should not be construed as a limitation thereof. The invention particularly relates to the specific embodiments described in these examples.

### Examples

### Cloning, expression, refolding and purification, crystallization

TLL1cd was expressed in *E. coli* in the form of insoluble inclusion bodies. The protein was dissolved in 7M guanidine-HCl, 100mM DTT, 1mM CaCl2, 250 mM Tris pH8.0, 10µM ZnCl2 to a concentration of 5 mg/ml. The dissolved inclusion bodies were purified by HPLC (high performance liquid chromatography). The protein was refolded by rapid dilution (1:100) into 0.8M arginine-HCl, 1mM CaCl2, 10µM ZnCl2, pH 8.0 containing 1mM oxidized glutathione and 0.25mM reduced glutathione. The refolding generated active monomeric TLL1cd. The final purification step (gel filtration) led to >80 % pure protein as judged by SDS polyacrylamide gel electrophoresis. The fractions containing enzymatic activity were pooled and stored frozen at - 80°C. The total yield was about 1 mg TLL1cd per liter of *E*. *coli* cell culture.
TLL1cd was concentrated to 11 mg/ml in 25mM Tris-HCl, pH 8.0, 125 mM NaCl, 25 µM ZnCl2 and crystallized at 20 °C by the vapour diffusion method in sitting drops. 0.3 µl protein solution containing 25 mM Tris-HCl pH 8.0, 125 mM NaCl and 10 µM ZnCl2 was mixed with 0.3 µl reservoir solution composed of 25 % (w/v) PEG 3350, 200 mM lithium sulphate and 100 mM Tris-HCl, pH 8.5. The drops were equilibrated against 200 µl of the reservoir solution. Small crystals appeared within 1-3 days. Subsequent optimization of crystallization conditions was carried out using the hanging drop method. Co-crystallization of ligands was carried out using the conditions described above.

### Data collection and processing

For X-ray data collection crystals were flash cooled in liquid nitrogen with the reservoir solution plus an additional 10% glycerol as cryoprotectant. The X-ray diffraction data were collected from a single crystal for each structure at 95 K at the beamline X10SA of the Swiss Light Source, operating at 300 mA with a MAR225 mosaic CCD detector and a monochromatic X-ray beam. For the crystals of TLL1cd alone and in complex with a ligand, 360 images were collected with 0.5° oscillation each. The exposure time was 0.5 s per image. The crystal-to-detector distance was 200, 160 and 120 mm for the ligand-free TLL1cd wild-type, ligand-free TLL1cd double mutant (C63A, C66A numbering from the N-terminal alanine; C62A, C65A using the astacin numbering scheme shown in figure 2) and the TLL1cd complex with ligand, respectively. The raw diffraction data were processed and scaled with XDS/XSCALE (Kabsch 1993) using the APRV (Kroemer, Dreyer and Wendt 2004) interface. The crystal data and data collection statistics are summarized in Table 1.

### Structure determination and structure refinement

The TLL1cd structure was solved by molecular replacement with the program Phaser version 1.3.1 (Vagin and Teplyakov 1997), using the coordinates of crayfish astacin (pdb code 1QJJ, refined to 1.86 A resolution (Bode, et al 1992)) as a search model. With a high resolution data cut-off of 3.0 A an unambiguous solution was found in space group P1 with two protein molecules in the asymmetric unit (rotational Z-scores of 10.0 and 9.0 for molecules 1 and 2, respectively). The TLL1cd structure was built and refined by alternating cycles of manual model (re)building using the program COOT version 0.1.0-pre-4 (Emsley and Cowtan 2004) and automated refinement using the bindividual protocols of the program REFMAC version 5.2.0019. Subsequently, water molecules were added using the program ARP version 5. (Vagin and Teplyakov 1997)

The refinement target was the maximum likelihood function with the parameters described by Engh and Huber (Engh and Huber 1991) using amplitudes. Cross validation was used throughout the refinement process, using 5.0 % of the reflections which were excluded from the refinement. The quality of the final model was assessed with the program PROCHECK (Laskowski, et al 1993). The refinement statistics are summarized in Table 2.

The structure of the TLL1 cd mutant and TLL1 cd in complex with a ligand was built and refined as described above for the enzyme without ligand, but the coordinates of the refined structure of TLL1 cd were used as a starting model without water molecules. For the ligand complex, anisotropic displacement parameters were included for the final refinement cycles at 1.56 A resolution using the program REFMAC. (Murshudov, Vagin and Dodson 1997, Murshudov, et al 1999) The refinement statistics are summarized in Table 2.

### Crystallization, data collection and structure determination

The crystallization and structure determination of TLL1cd has not been described to date. Screening for crystallization conditions for recombinant TLL1 cd using the commercial screening kits Index and Crystal Screen I (Hampton Research) yielded very small TLL1cd crystals (figure 1) which showed very weak diffraction using on an in-house FRE generator. The largest crystals were grown using the hanging drop method in the presence of 25% PEG 3350, 0.2M Li2SO4, 0.1 M Tris pH 8.0. The crystals grew in the triclinic spacegroup P1 with two molecules of TLL1cd in the asymmetric unit. Cocrystallization with ligands was carried out by adding the ligand at a concentration of 2-10 mM to the protein 30 minutes before crystallization. Soaking of ligands was carried out at ligand concentrations of 2-20mM in the reservoir buffer for a duration of 5 min to 24 hours. The ligand complex structure was obtained by cocrystallization with 10mM ligand. For data collection crystals could be flash cooled in the reservoir buffer prepared with an additional 10% glycerol or with an increased PEG3350 concentration of 35%. The largest crystals have dimensions of roughly 10 x 40 x 100 µm and diffract to a resolution of 1.5 A at the Swiss Light Source. The data collection statistics are summarized in Table 1.

### Structure of TLL1cd

The overall architecture of TLL1cd is similar to that of crayfish astacin, consisting of a V-shaped molecule that is divided by a long, deep active site cleft into two domains. The N-terminal domain comprises the first 100 residues and consists of a four-stranded pleated β-sheet and two long α-helices. The C-terminal domain consists mainly of irregular loops with several turns, in addition to an α-helix and a short two-stranded β-sheet. For ease of comparison, the astacin numbering scheme has been used for TLL1cd. The N-terminus is buried while the C-terminus is linked to the N-terminal domain by a disulfide bond between cysteines 42 and 198. The catalytic zinc is anchored by histidines 91, 96 and 102, and a water molecule liganded by Glu92. Unlike astacin, Tyr149 of TLL1cd does not function as additional zinc ligand. It has been proposed that the role of Tyr149 in astacin is to prevent loss of the catalytic metal from the free enzyme (Bode, et al 1992).

Despite the relatively high sequence similarity (36%) between astacin and TLL1 cd (figure 2), there are some surprising structural differences between the substrate binding sites of the two enzymes. In particular, a shift of up to 7 A in residues 165-170 places the side chain of Arg176 in the S1' pocket. The presence of a basic residue in S1' fits well with the substrate specificity of TLL1cd, which requires an aspartic acid in P1' for efficient substrate cleavage.

### Disordered cysteine-rich loop

In all TLL1cd structures solved to date, the cysteine rich loop comprising R60-P-C-G-C-C-S66 is partially or completely disordered (figure 3). The corresponding loop S60-G-S-G-C-W-S66 of astacin is well ordered in structures of both the free enzyme and inhibitor complexes. This loop forms part of the S1 and S1' binding pockets and is therefore of great importance for inhibitor design. In our structure, partial density is observed for Cys64 and Cys65. Cys64 makes up part of the S1' pocket and forms a disulphide bond with Cys84, while Cys65 forms part of the S1 pocket. Both residues occupy similar positions to the corresponding residues in astacin, namely Cys64 and Trp65. The presence of two extra cysteines in the loop region of TLL1cd has led to speculation that a disulphide may form between Cys62 and Cys65, which would mimic the hydrophobic side chain of Trp65 of astacin (Garrigue-Antar, Barker and Kadler 2001). In the TLL1 cd structure, Cys62 is completely disordered and there is no extension of the electron density surrounding the side chain to suggest the presence of a disulphide bond. We speculate that the formation of a disulfide bond between Cys62 and Cys64 is unlikely, as disulphide bonds between residues that are separated by only one residue are energetically unstable and seldom observed (Bhattacharyya, Pal and Chakrabarti 2004, Gross, et al 2002).

Several experiments were carried out in order to test different possible causes of disordering of the cysteine-rich loop. The TLL1cd double mutant Cys62Ala, Cys65Ala was produced in order to address the possibility that improper disulfide bond formation was responsible for disordering of the loop. The mutant was crystallized and the structure revealed a similar degree of disorder as observed for the wild-type enzyme. This excludes the possibility that incorrect or "scrambled" disulphide formation is necessary for a disordered conformation of the cysteine-rich loop. However, it does not exclude the possibility that the correct formation of all three disulphides is necessary for an ordered loop conformation.

The second hypothesis to be tested was that the cysteine-rich loop is inherently disordered in the absence of bound substrate or inhibitor. As the partial binding of a ligand failed to induce an ordered conformation, a peptidic inhibitor binding to the alternative (non-primed) side of the active site was designed. The inhibitor was designed on the basis of the TLL1 cd substrates listed in the Merops database (Rawlings, Morton and Barrett 2006). Finally, the hypothesis that N-terminal acetylation of TLL1 cd, described below, could be indirectly related to the disorder of the cysteine-rich loop will be tested by mutagenesis of the N-terminal alanine residue to threonine in order to prevent acetylation.

### Apparent N-terminal acetylation

The first crystal structure of astacin revealed that its partially buried N-terminal alanine formed a water-mediated interaction with Glu103, which is directly adjacent to the catalytic zinc-binding residue His102. On this basis, it was proposed that upon proteolytic activation, the insertion of the newly formed N-terminus of astacin of the proenzyme might play a role in stabilizing the active site. The proposed activation mechanism is thus analogous to that observed for the trypsin-like serine proteases.

By contrast with astacin which was isolated from its natural source, the N-terminal alanine residue of TLL1 cd appears to be acetylated and the interaction with Glu103 is mediated by a metal ion such as sodium or calcium. The N-terminal methionine shown in the sequence of TLL1cd is removed by E. coli during expression and thus is not present in the crystallized protein. No methionine is present in this position of the sequence of human TLL1, which contains additional residues N-terminal to the protease domain. The alanine acetylation is clearly an artefact caused by recombinant expression of the catalytic domain of TLL1cd in E. coli. Although the electron density strongly indicated acetylation of the N-terminus of TLL1cd, mass spectrometry analysis revealed only the mass of the unacetylated protein.

### Binding mode of ligand

The crystal structure of TLL1cd was solved in complex with a partially bound (occupancy approximately 50%) ligand. The ligand binds to the primed side of the active site, from S1' to S3'. A group binds in S1' and displaces Arg176 from the base of the pocket. A substituent lies adjacent to Thr153 and Phe154 in the S2' pocket and another group forms a hydrogen bond with the side chain of the displaced Arg176. A further group is poorly ordered in the structure, with weak electron density to suggest that it binds in the S3' region close to Asn82. While the structure of a non-prime side hydroxamic acid inhibitor of astacin is available (pdb code 1 QJJ), our ligand complex structure provides the first evidence of a primed side inhibitor of the astacin class of enzymes. A surface representation of the binding site is shown in fugure 4.

Interestingly, the structural rearrangement that accompanies inhibitor binding to astacin (Grams, et al 1996) was not observed for our complex. Briefly, an overall hinge movement in astacin brings the N- and C-terminal domains ~1 Å closer. Similar hinge movements have been observed for the structurally related thermolysins.

### Conclusion

Thus, our results demonstrate that the cloning, expression, purification and crystallization of the catalytic domain of TLL1 cd is well suitable for ligand design. Our results show for the first time that the obtained structures reveal an unexpected arginine in the S1' binding pocket, which is displaced upon binding of the inhibitor. An other surprising and unexpected property of the crystal structures is that there is a disordered cysteine-rich loop in the S1 and S1' region of the active site, which loop is of great importance for ligand design.

**Table 1 Crystal data and data collection statistics**

| Dataset | TLL1cd wild type | TLL1cd double mutant | Ligand complex |
|---|---|---|---|
| X-ray source | SLS / X10SA | SLS/X10SA | SLS/X10SA |
| Date of data collection | 22/06/2006 | 16/10/2006 | 20/8/2006 |
| Wavelength | 1.0077 | 1.0002 | 1.0332 |
| Detector | MARCCD | MARCCD | MARCCD |
| Temperature (K) | 100 | 100 | 100 |
| No. of crystals | 1 | 1 | 1 |
| Spacegroup | 1 | 1 | 1 |
| Unit cell (a,b,c) (Å) | 41.2, 47.8, 58.6 | 41.4, 48.6, 58.8 | 41.3, 48.0, 59.0 |
| Unit cell (α,β,γ) (°) | 90.1, 90.0, 74.6 | 90.0, 90.0, 73.5 | 90.0, 90.3, 74.2 |
| No. of monomers/ a.u. | 2 | 2 | 2 |
| Solvent content | 2.46 | 2.64 | 2.11 |
| Resolution range | 49.7 | 53.1 | 41.3 |
| No. of observations | 63833 | 124440 | 95812 |
| No. of unique | 33529 | 64744 | 47998 |
| reflections | | | |
| | | | |
| Overall | | | |
| Redundancy | 1.9 | 1.9 | 2.0 |
| Completeness | 92.5 | 90.8 | 95.7 |
| <I/σ (I)> | 11.2 | 11.1 | 14.9 |
| Rmerge | 0.044 | 0.042 | 0.039 |
| | | | |
| Highest resolution shell | | | |
| Resolution range | 1.92-1.86 | 1.54-1.49 | 1.73-1.68 |
| Completeness (%) | 63.2 | 65.5 | 94.4 |
| <I/σ (I)> | 3.1 | 4.2 | 3.9 |

| | | | |
|---|---|---|---|
| Rmerge = Σ \|Io -<I>\| / Σ<I> | | | |

**Table 2 Refinement statistics**

| Structure | TLL 1cd wild type | TLL1 cd double mutant | Ligand complex |
|---|---|---|---|
| Data used in refinement (A) | | | |
| resolution range | 58.5-1.86 | 35.7-1.68 | 58.9-1.46 |
| intensity cutoff (Sigma(F)) | 0 | 0 | 0 |
| no. of reflections (working/test) | 30174/1677 | 43197/2400 | 64139/3564 |
| completeness (%) | 92.5 | 95.7 | 93.7 |
| | | | |
| Fit to data used in refinement | | | |
| overall Rcryst | 0.199 | 0.217 | 0.173 |
| overall Rfree | 0.232 | 0.261 | 0.201 |
| Fit in the highest resolution bin | | | |
| resolution range | 1.91-1.86 | 1.72-1.68 | 1.50-1.46 |
| bin completeness (%) | 61.8 | 94.7 | 92.5 |
| bin Rcryst | 0.223 | 0.260 | 0.181 |
| bin Rfree | 0.299 | 0.310 | 0.235 |
| Number of non-hydrogen atoms | 3373 | 3474 | 3826 |
| protein atoms | 3068 | 3068 | 3300 |
| inhibitor atoms | 0 | 0 | 60 |
| waters | 332 | 406 | 429 |
| Overall B value from Wilson plot | 29.2 | 25.6 | 21.9 |
| Overall mean B value | 29.3 | 20.8 | 17.9 |
| protein atoms | 28.3 | 19.7 | 16.7 |
| inhibitor atoms | - | - | 20.2 |
| water molecules | 37.9 | 28.9 | 26.4 |
| | | | |
| Estimated coordinate error | | | |
| from Free R-factor | 0.147 | 0.125 | 0.076 |
| from maximum likelihood | 0.098 | 0.085 | 0.041 |
| | | | |
| Rms deviations from ideal values | | | |
| bond lengths | 0.016 | 0.014 | 0.013 |
| bond angles | 1.56 | 1.44 | 1.46 |
| | | | |
| Ramachandran plot | | | |
| residues in most favorable regions | 90.7 | 90.7 | 87.1% |
| residues in additional allowed regions | 9.3 | 9.0 | 12.0% |
| residues in generously allowed regions | 0.0 | 0.3 | 0.9% |

| | | | |
|---|---|---|---|
| Rcryst = Σ \| Fo - Fc I /ΣFo | | | |

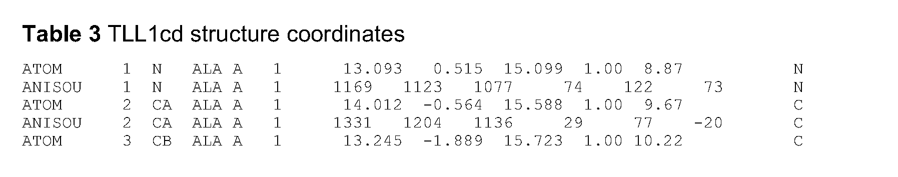

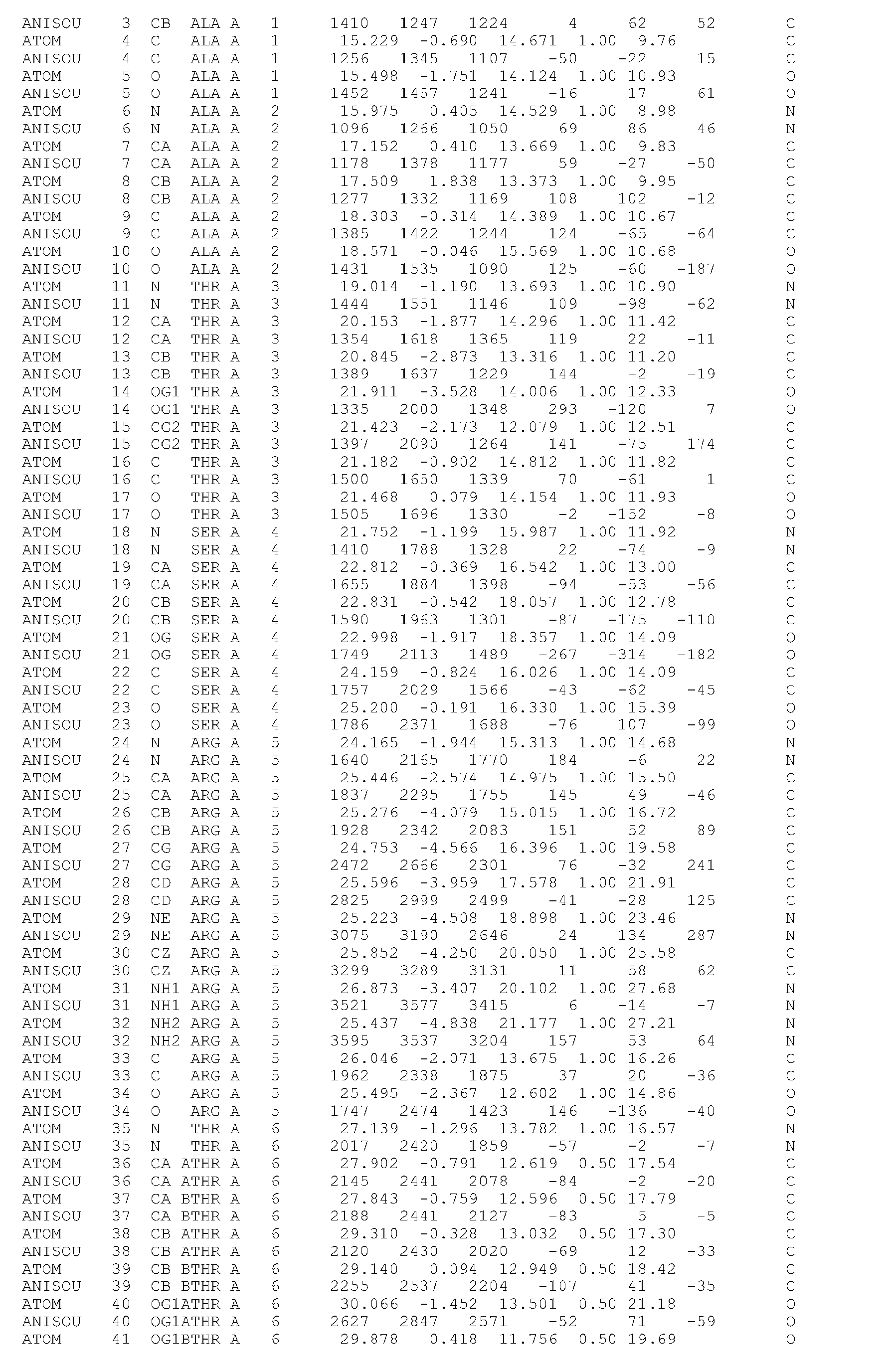

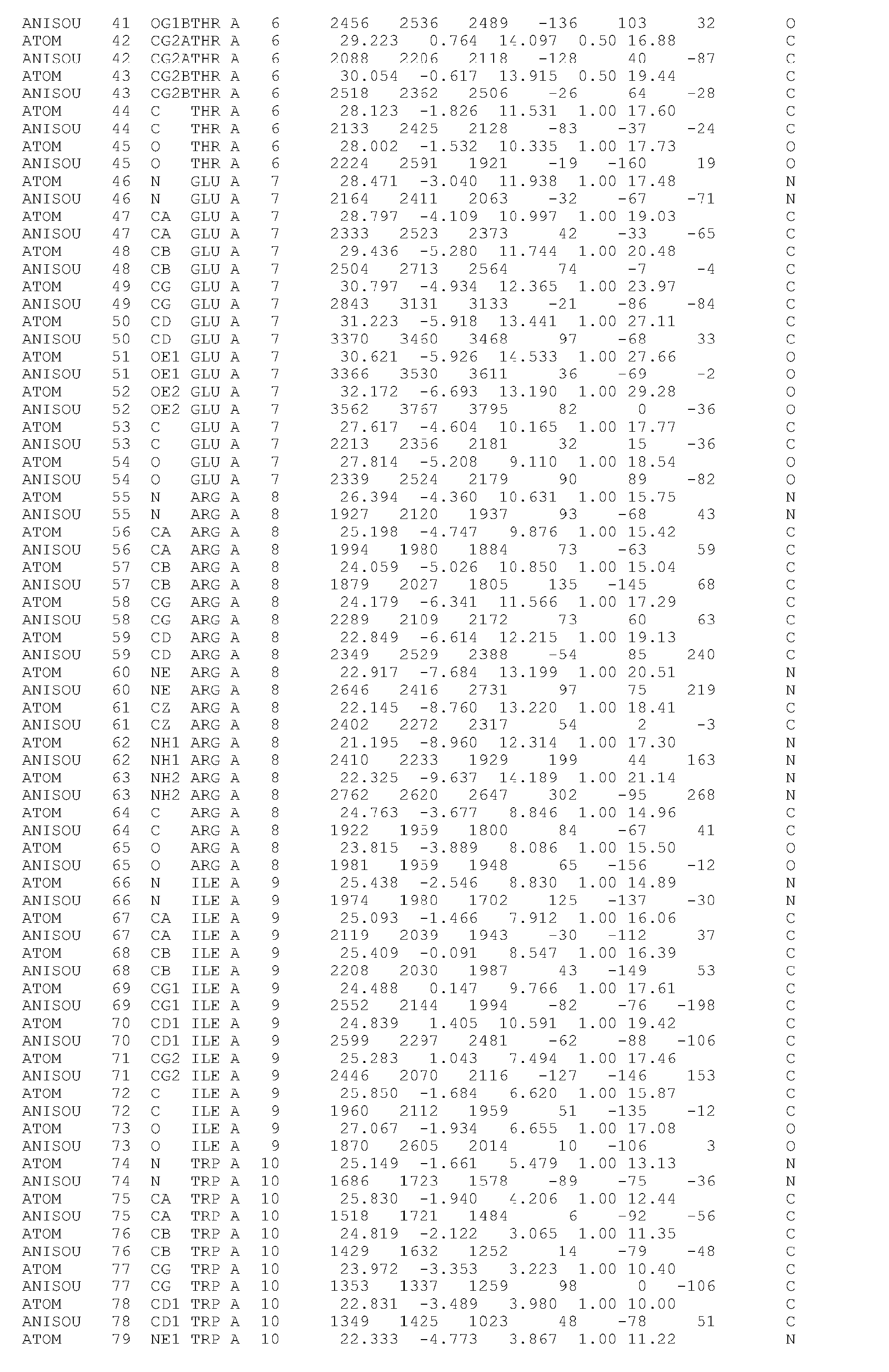

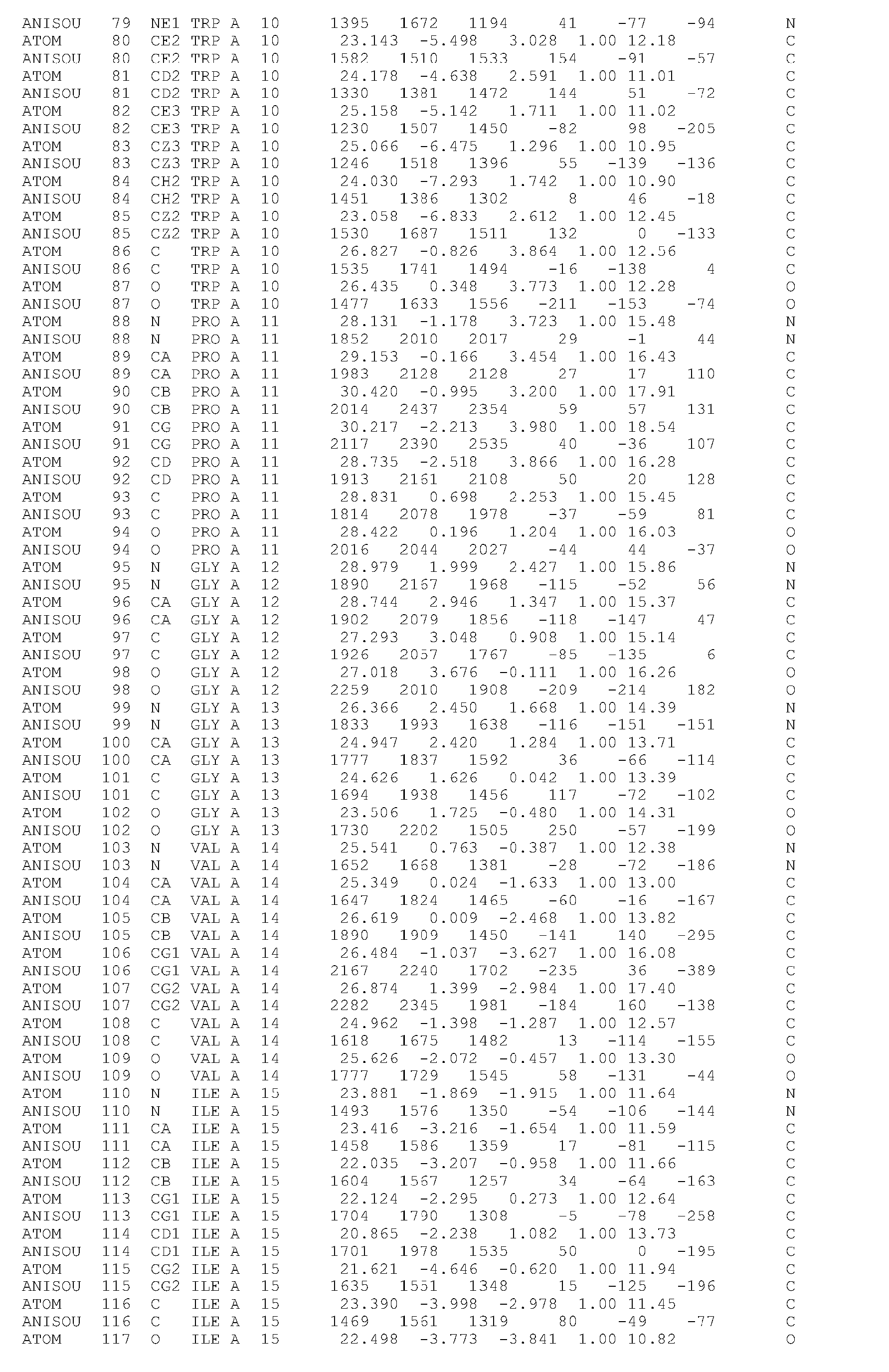

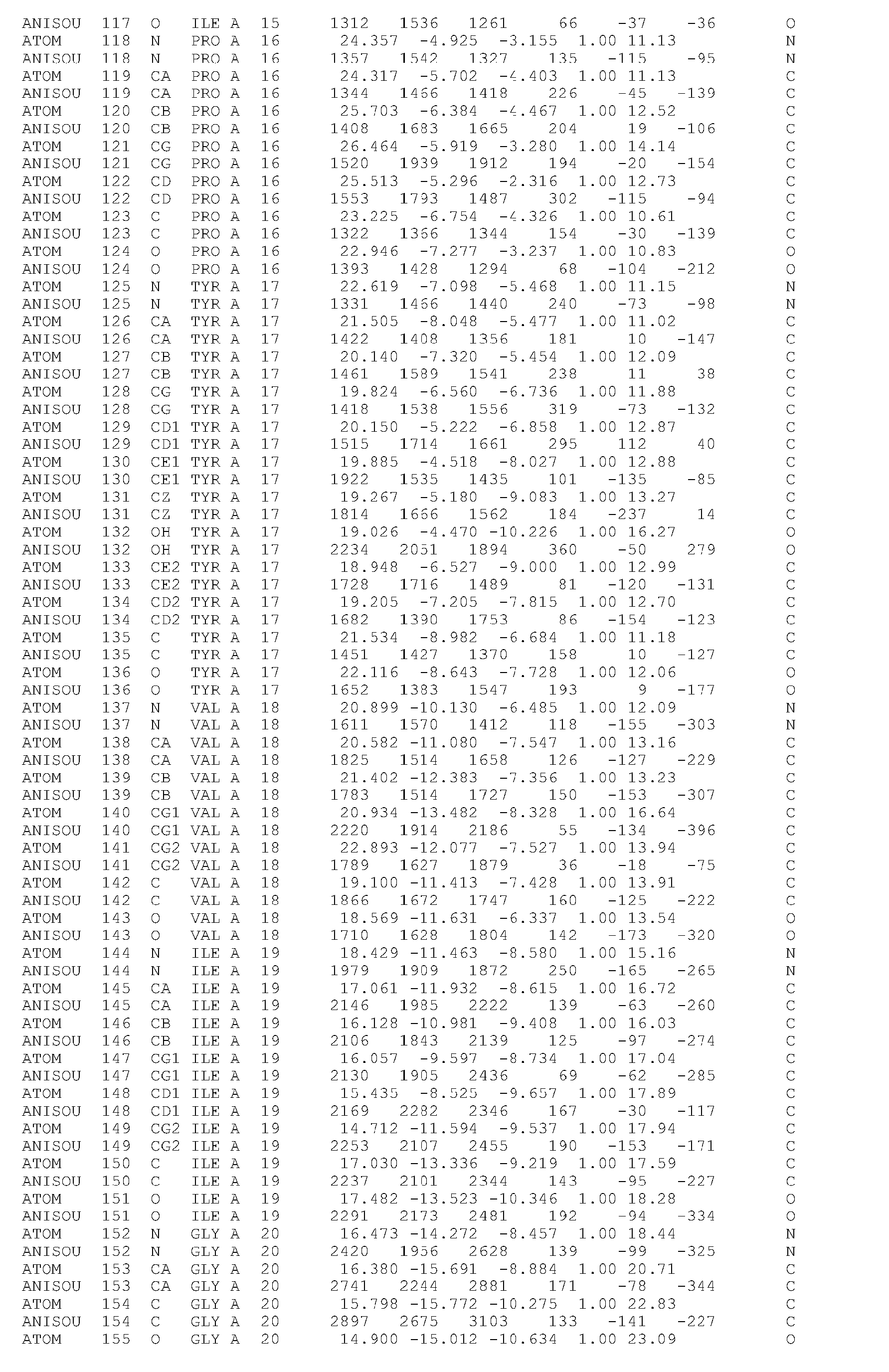

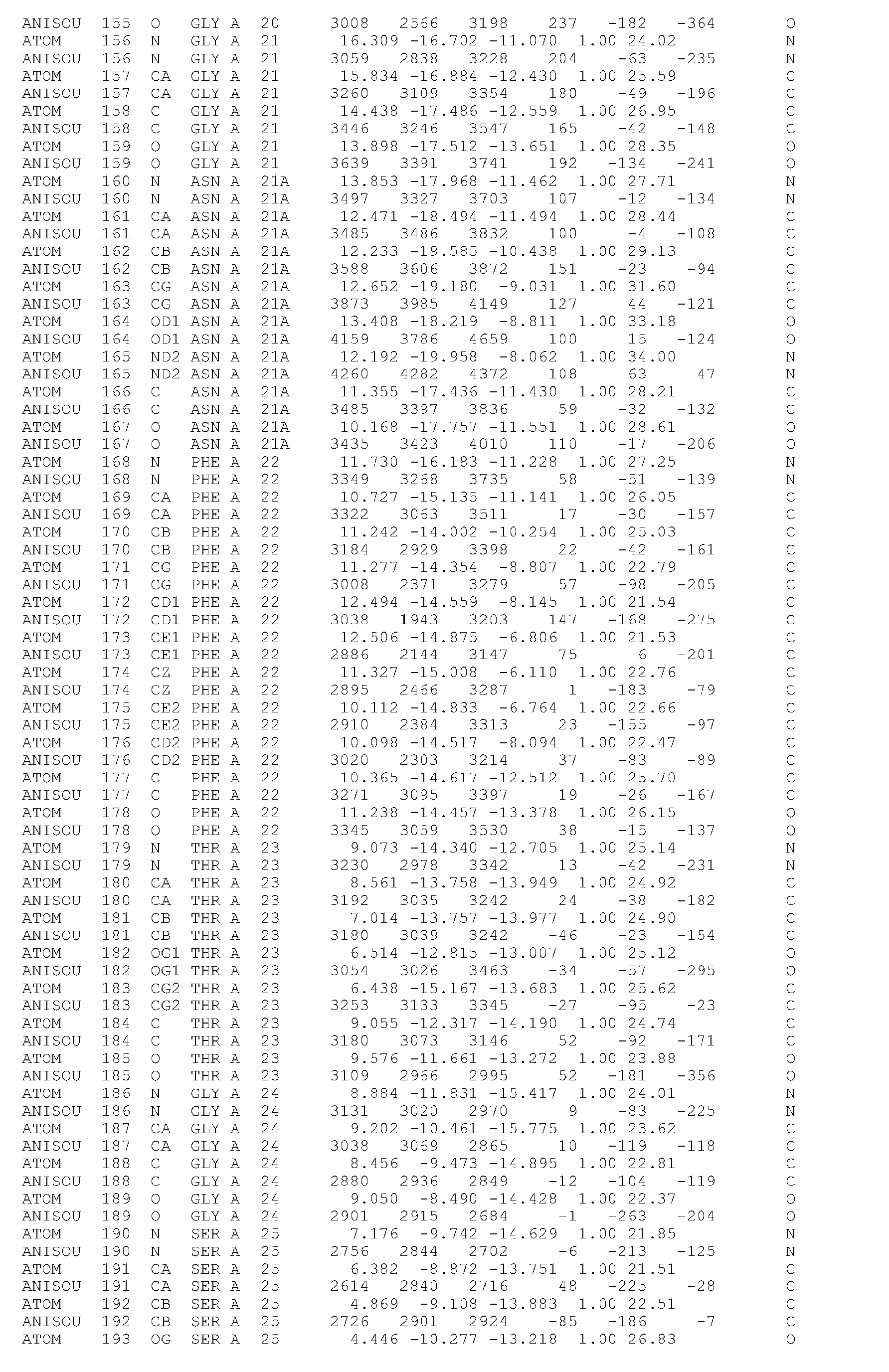

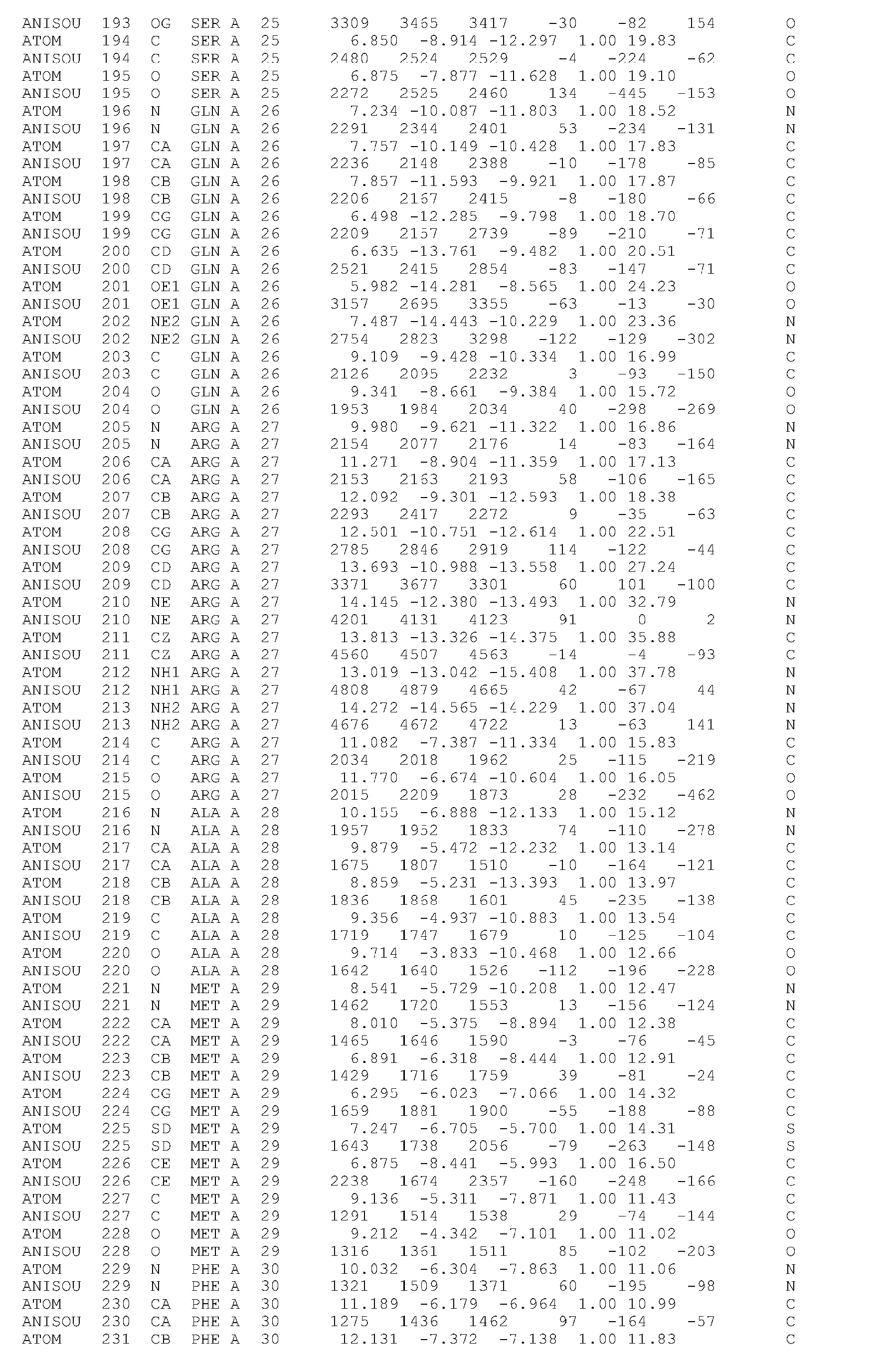

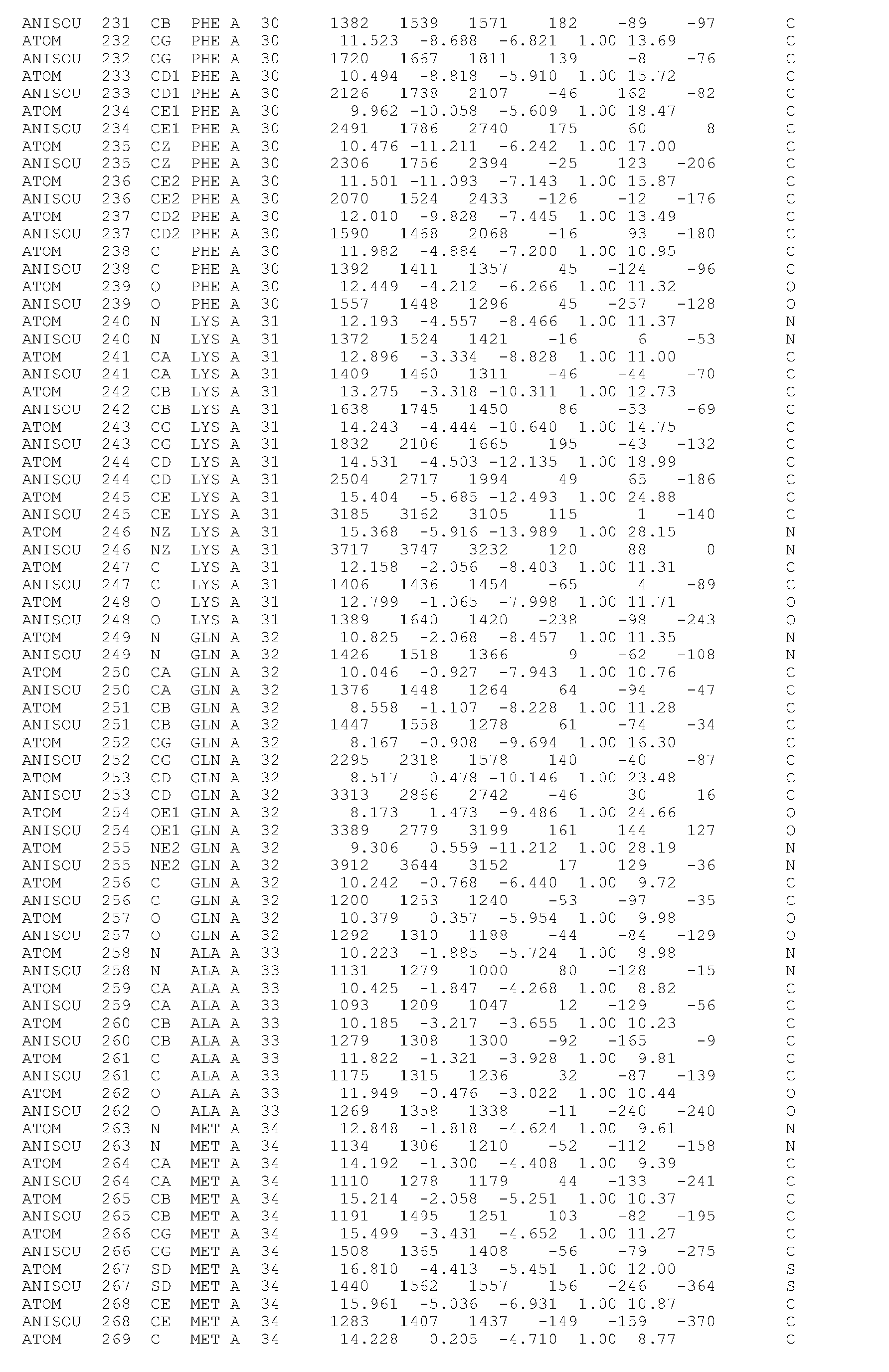

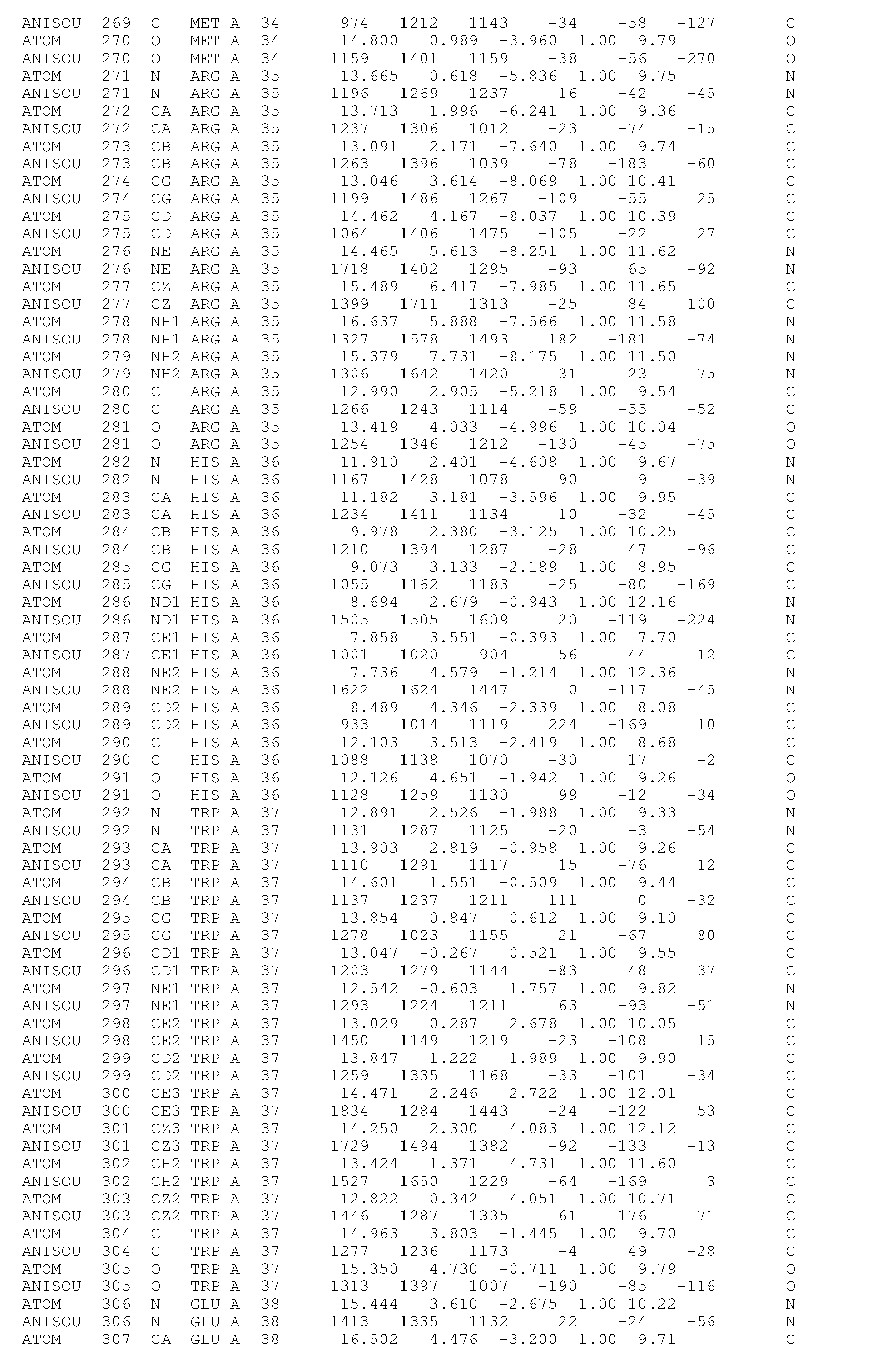

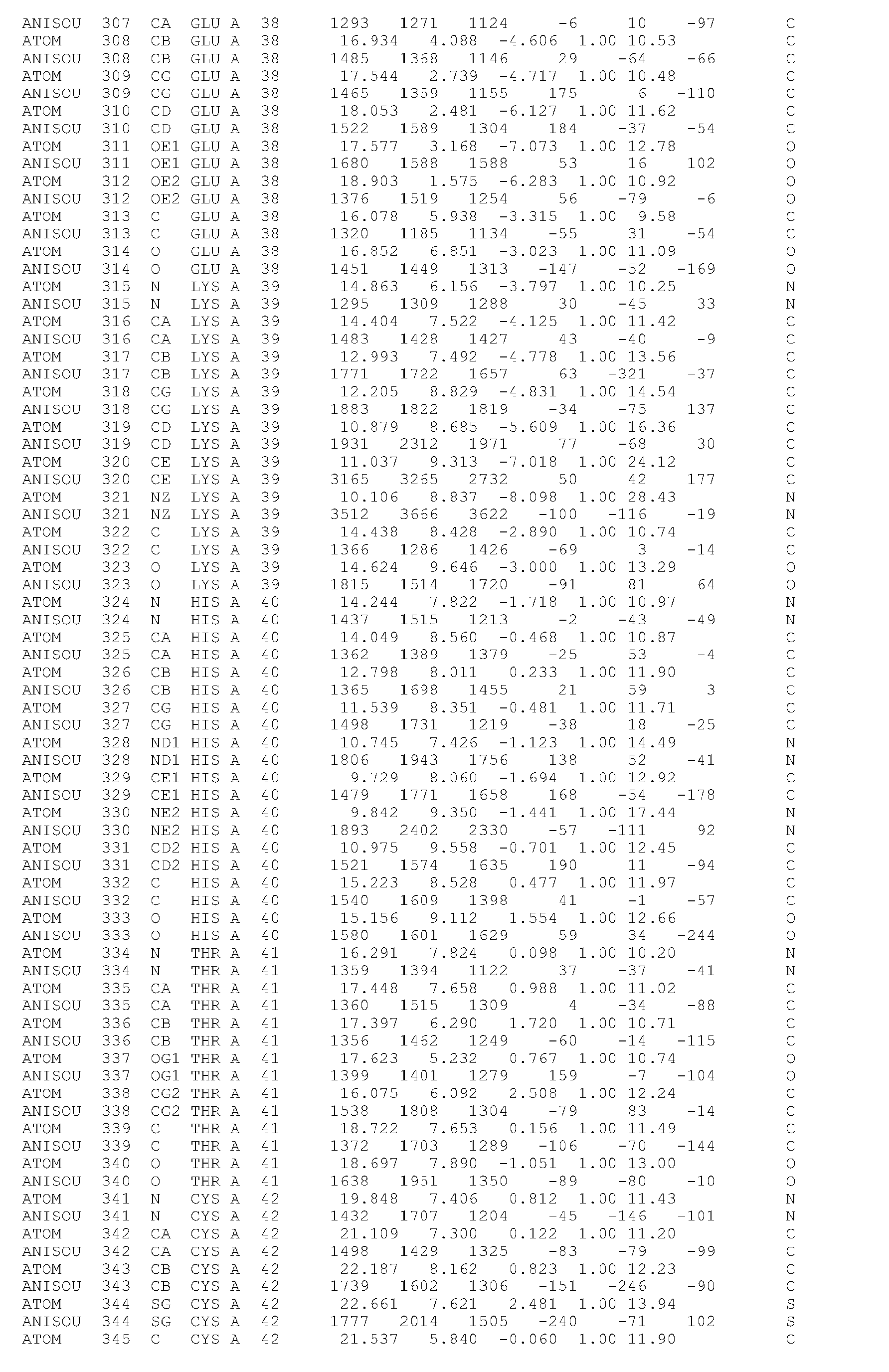

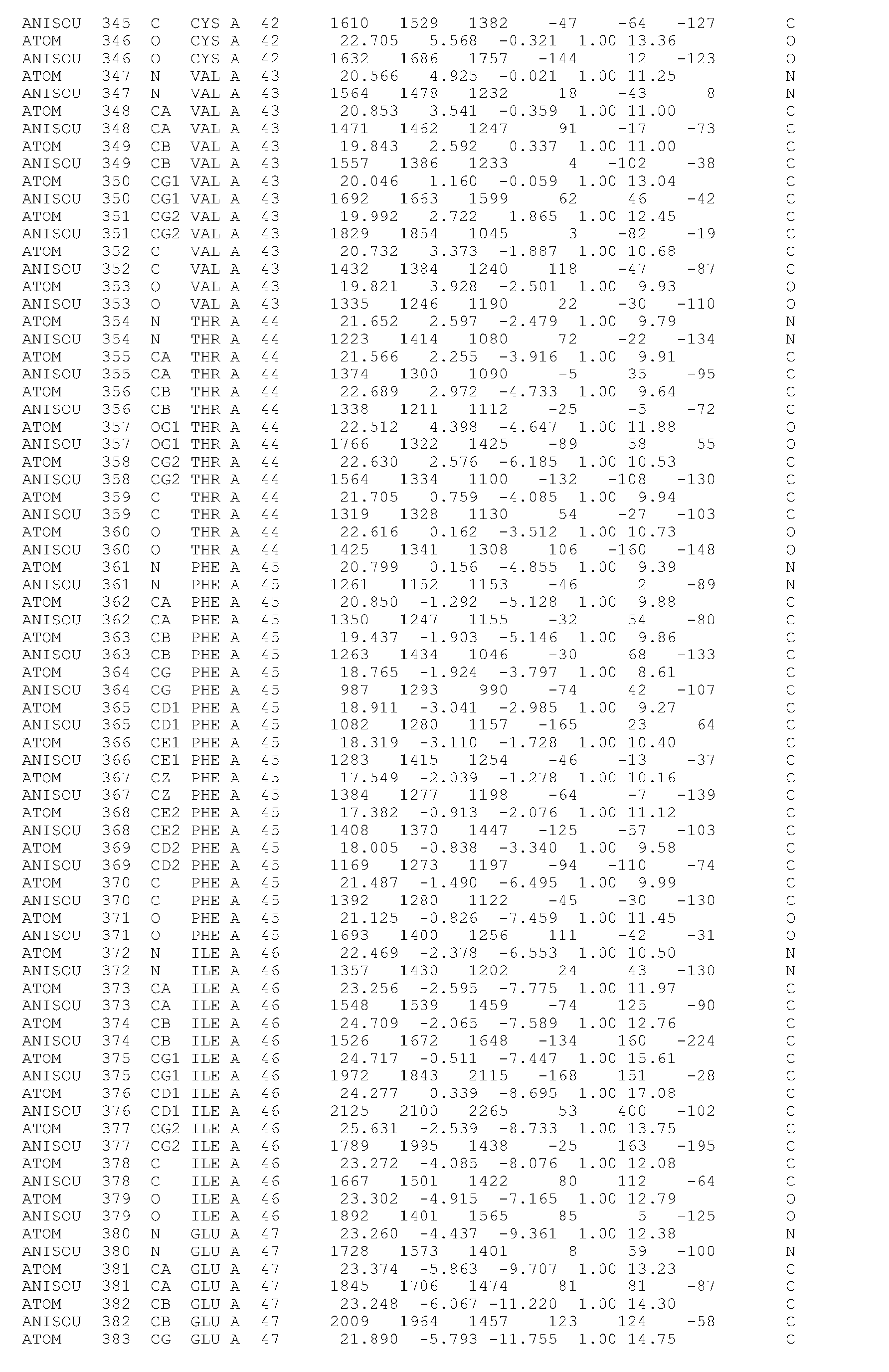

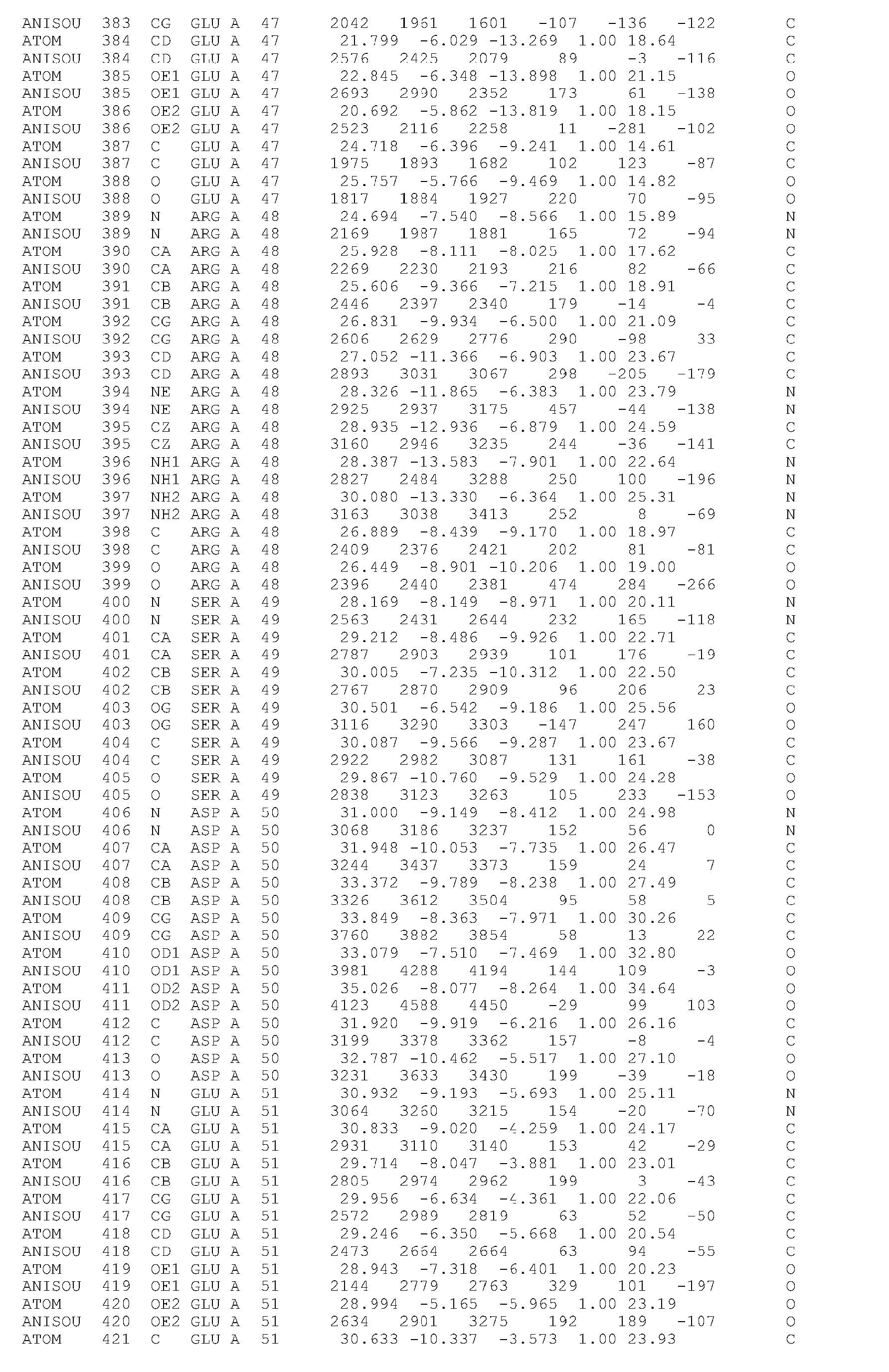

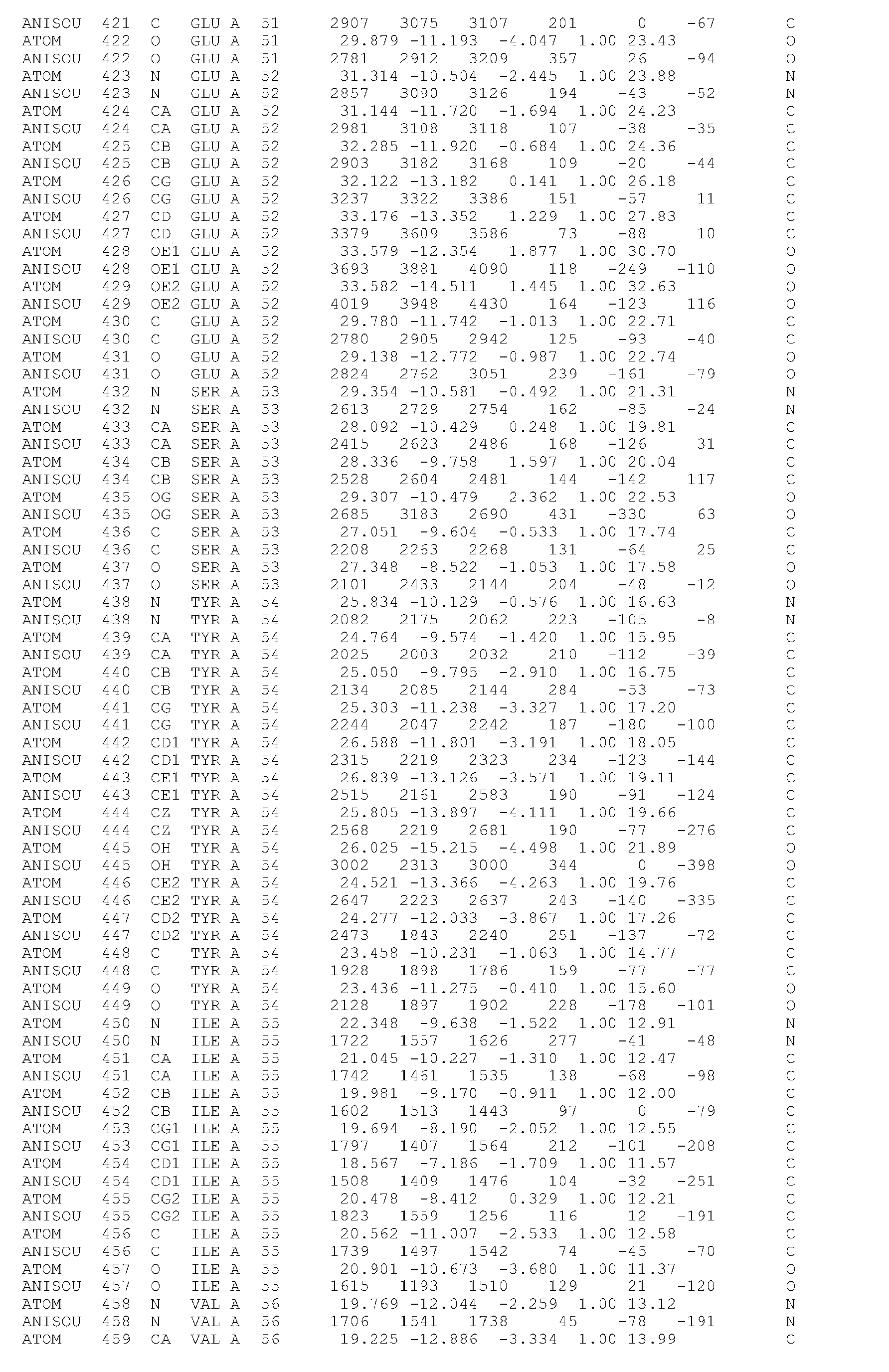

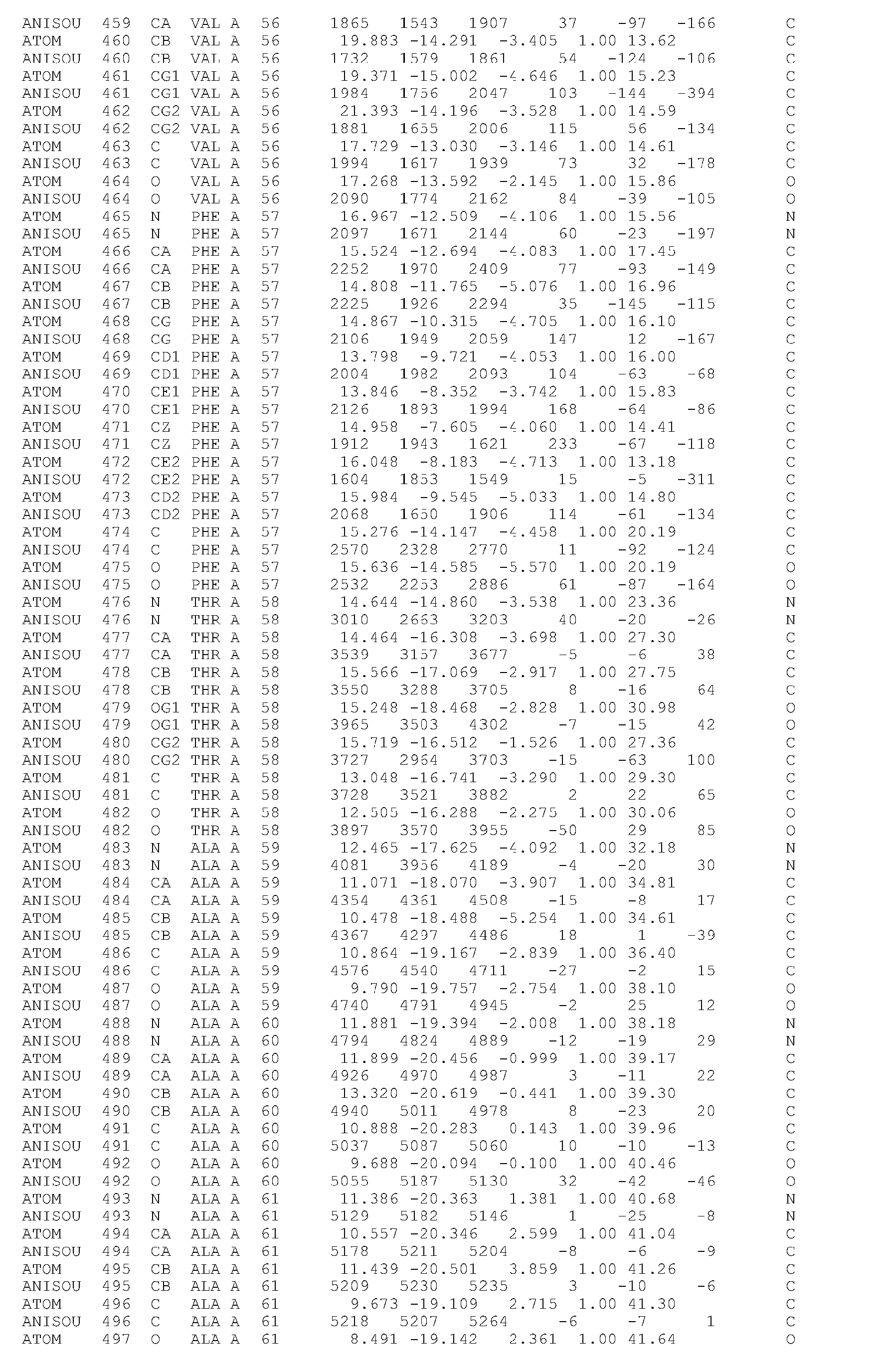

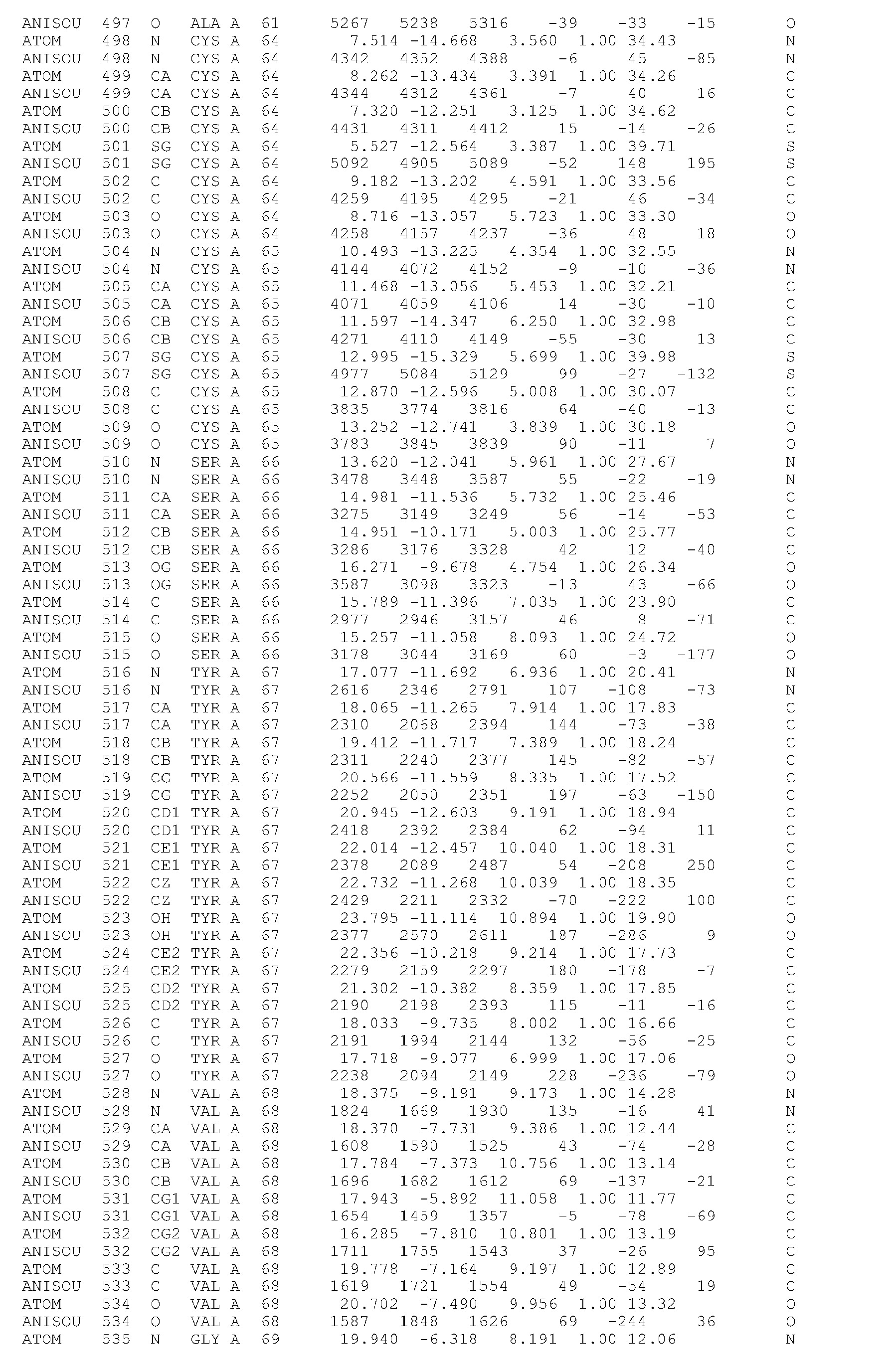

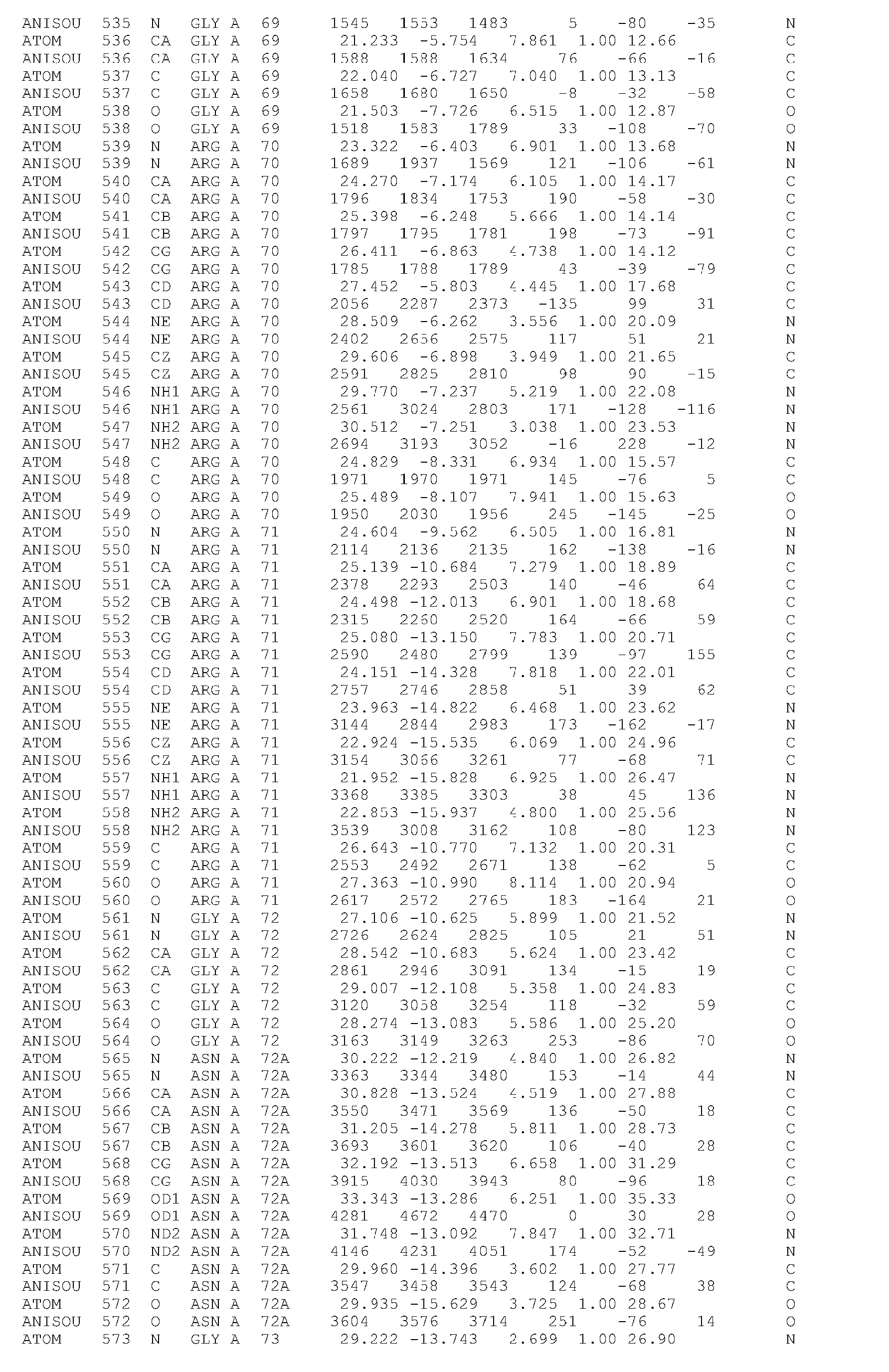

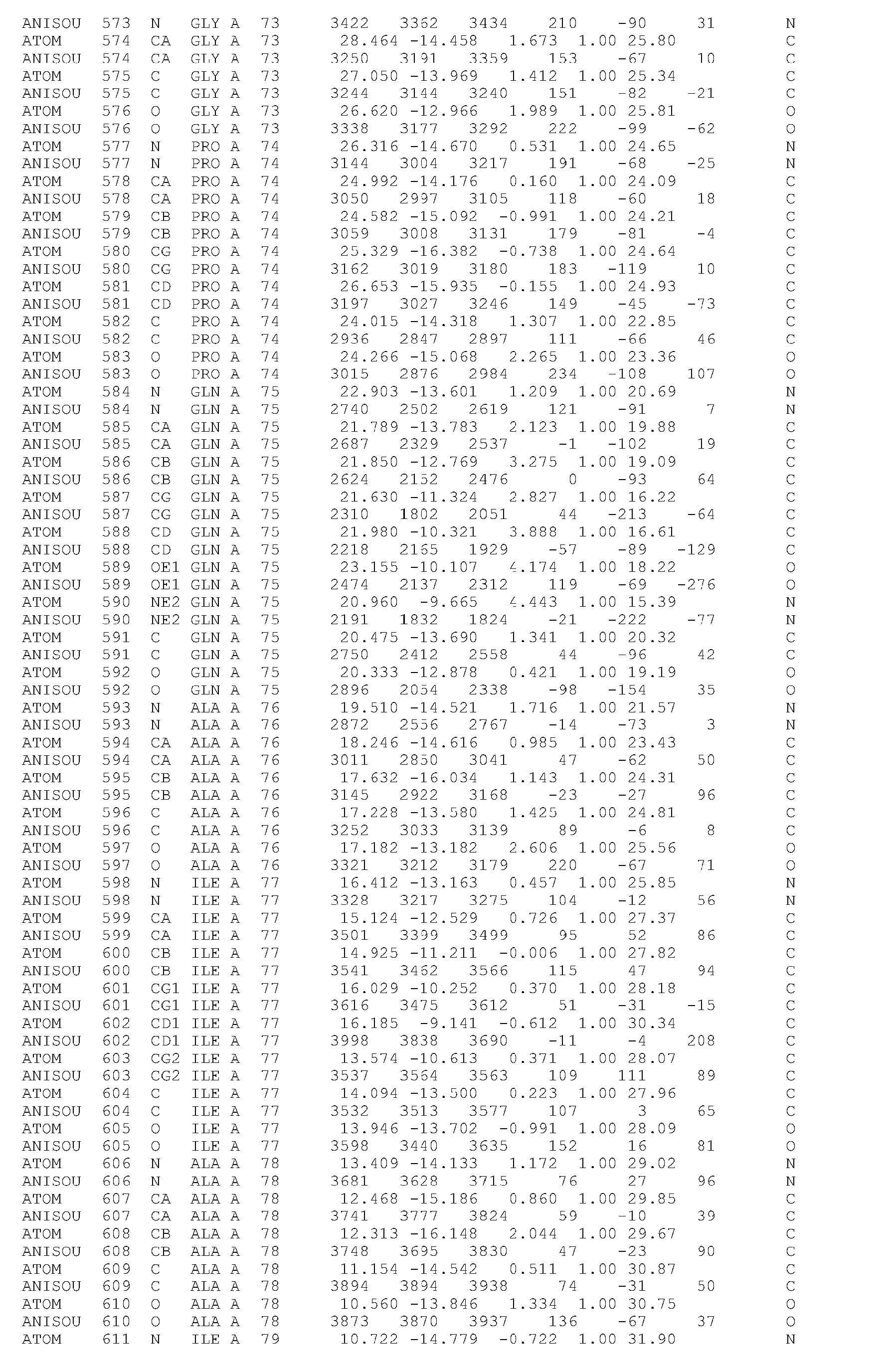

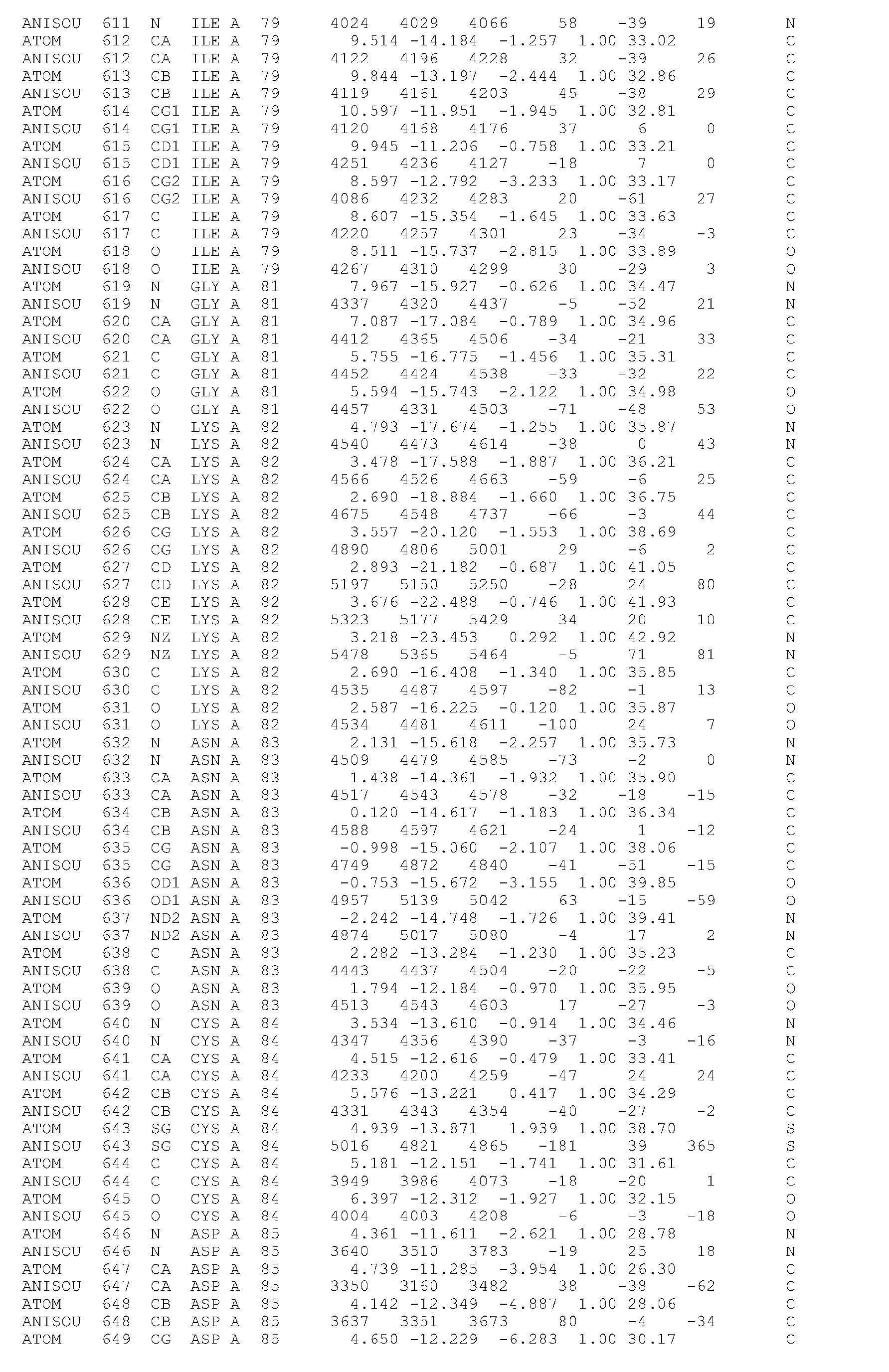

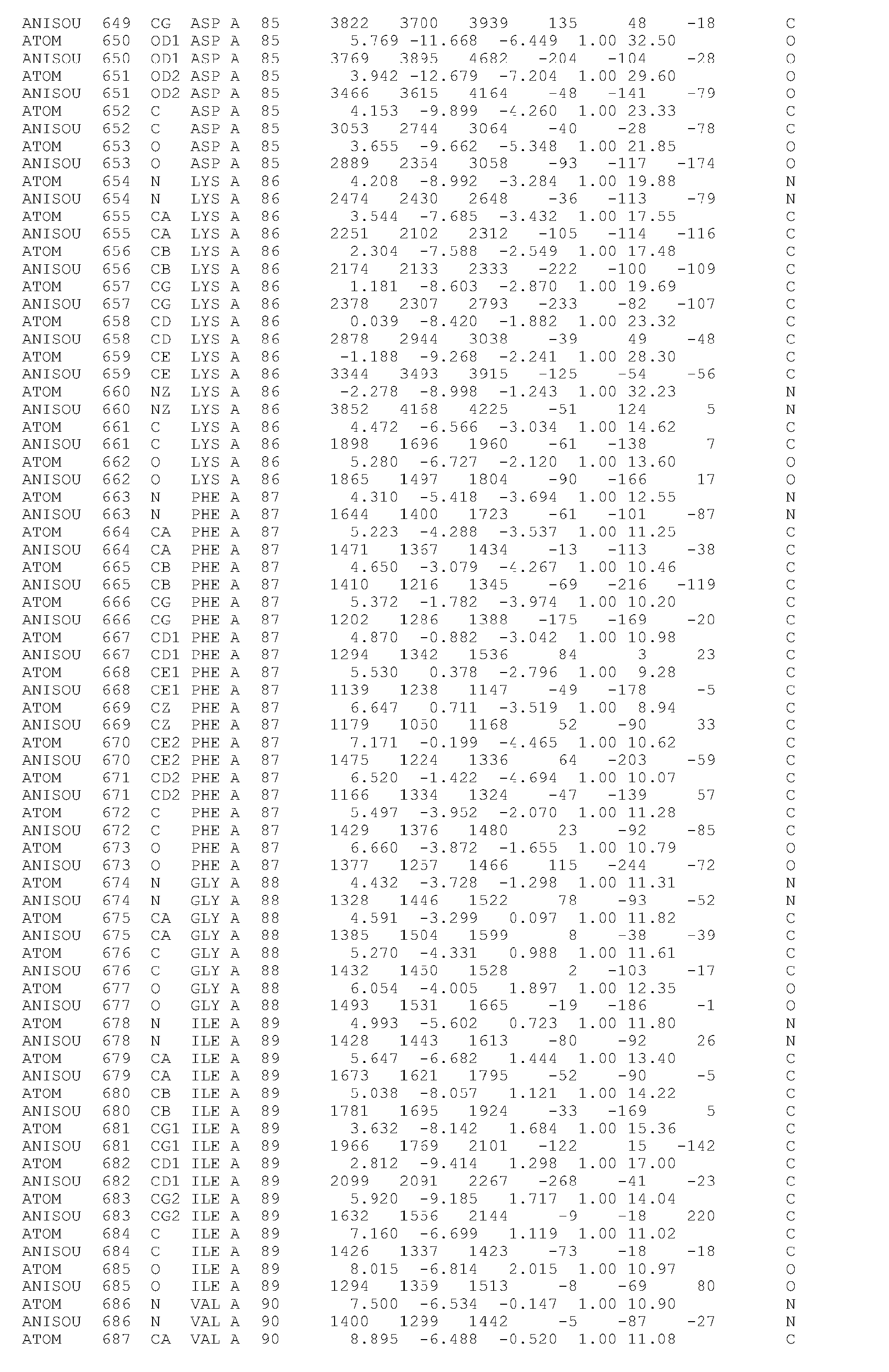

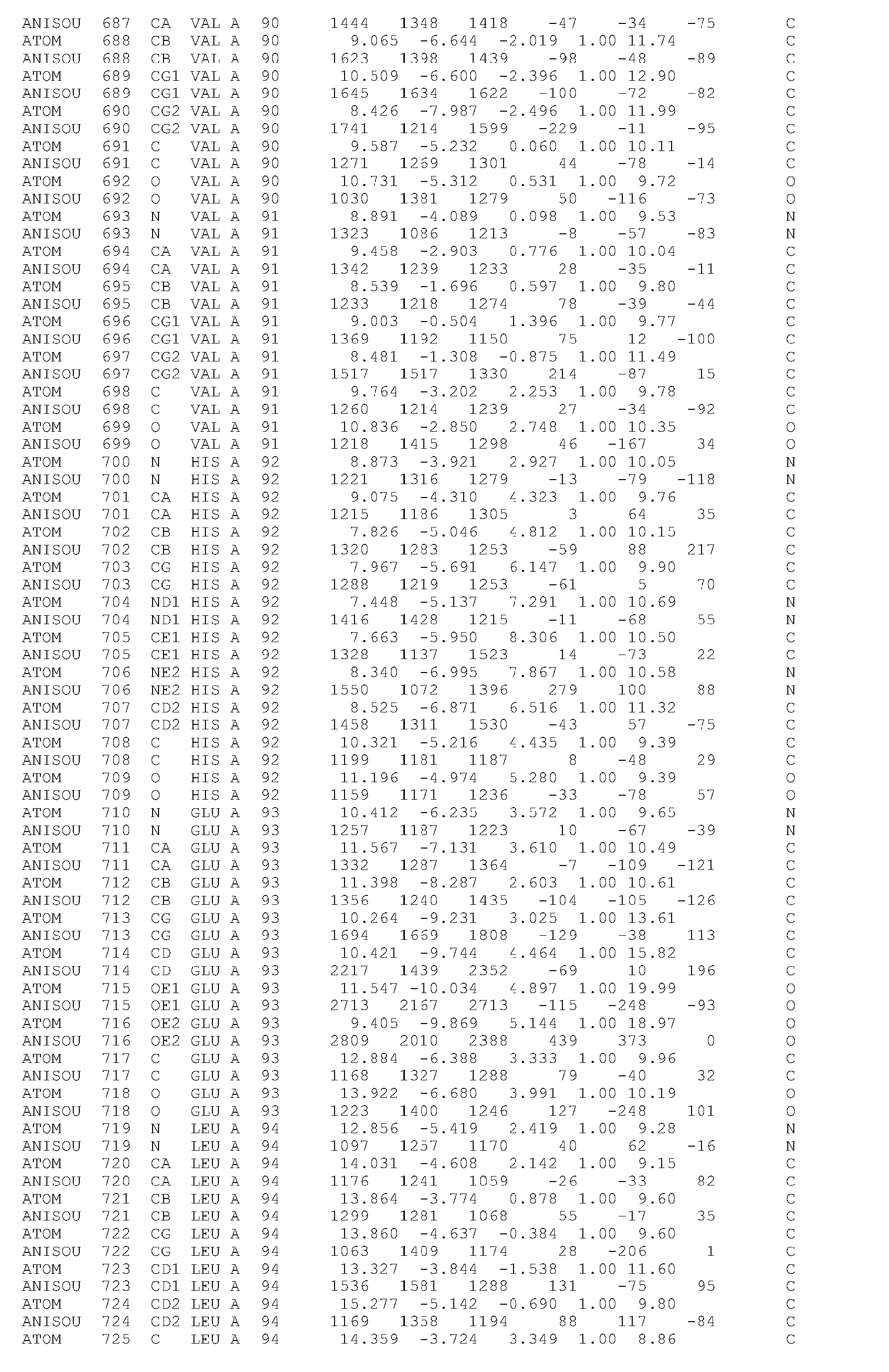

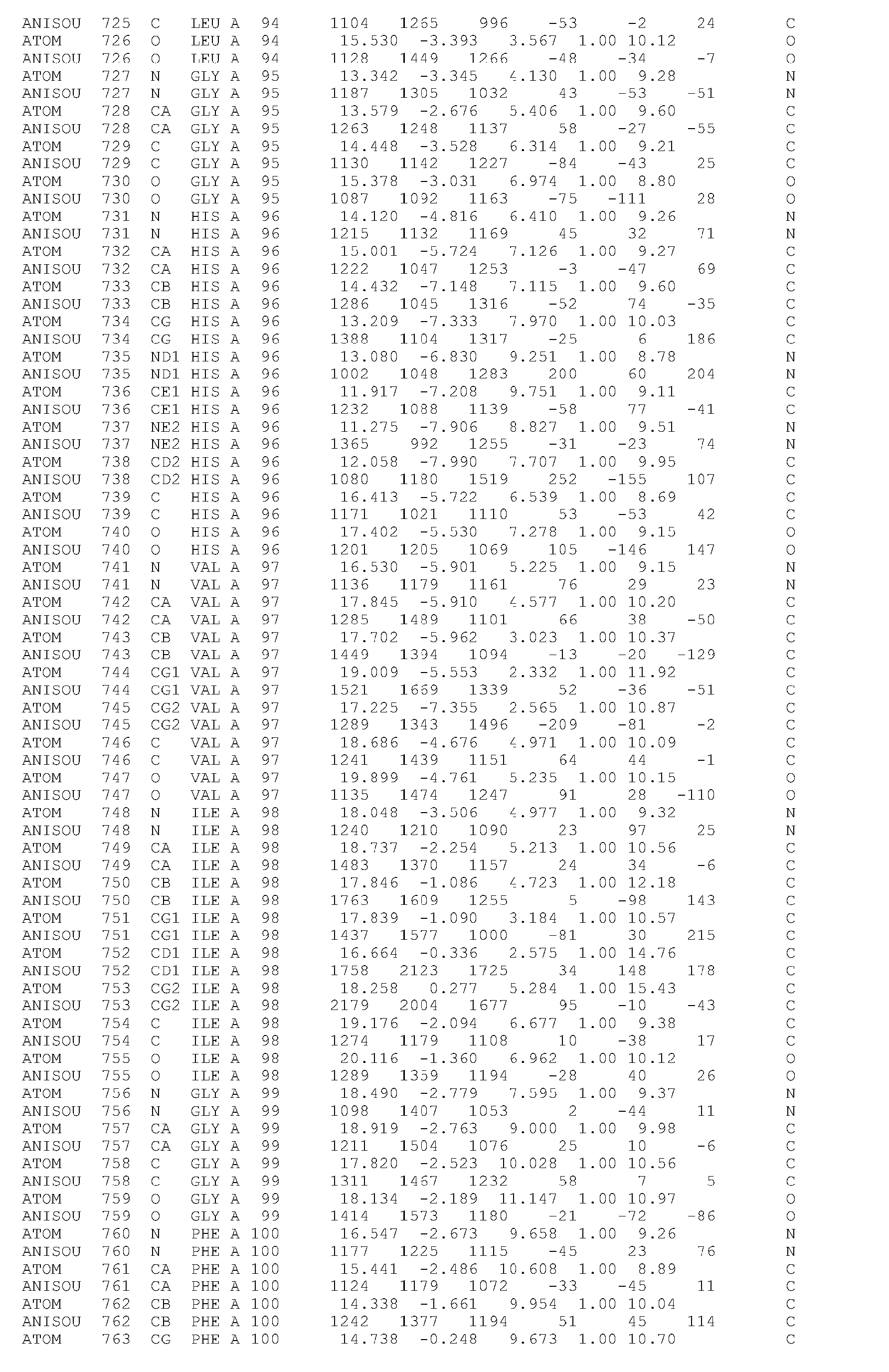

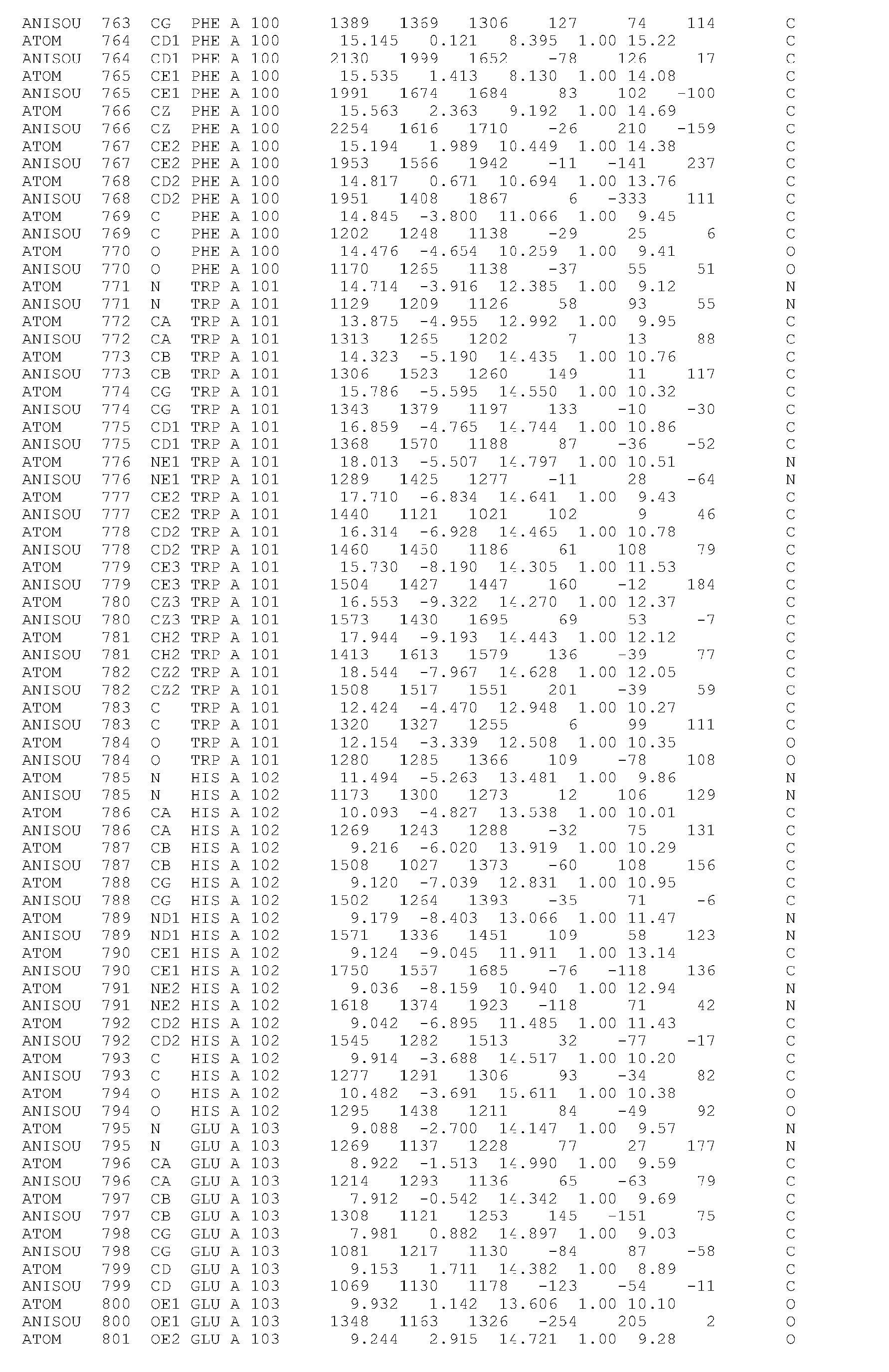

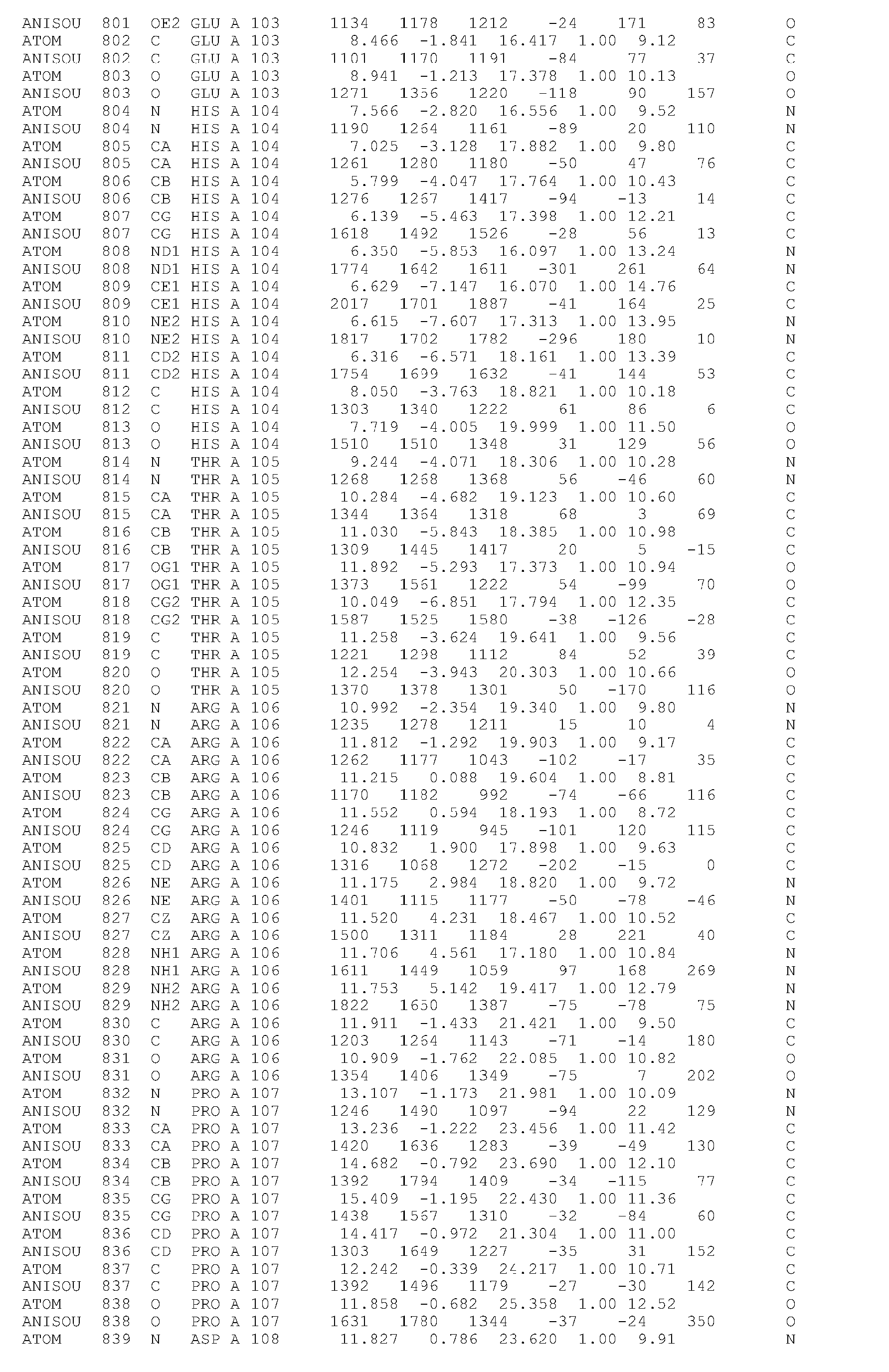

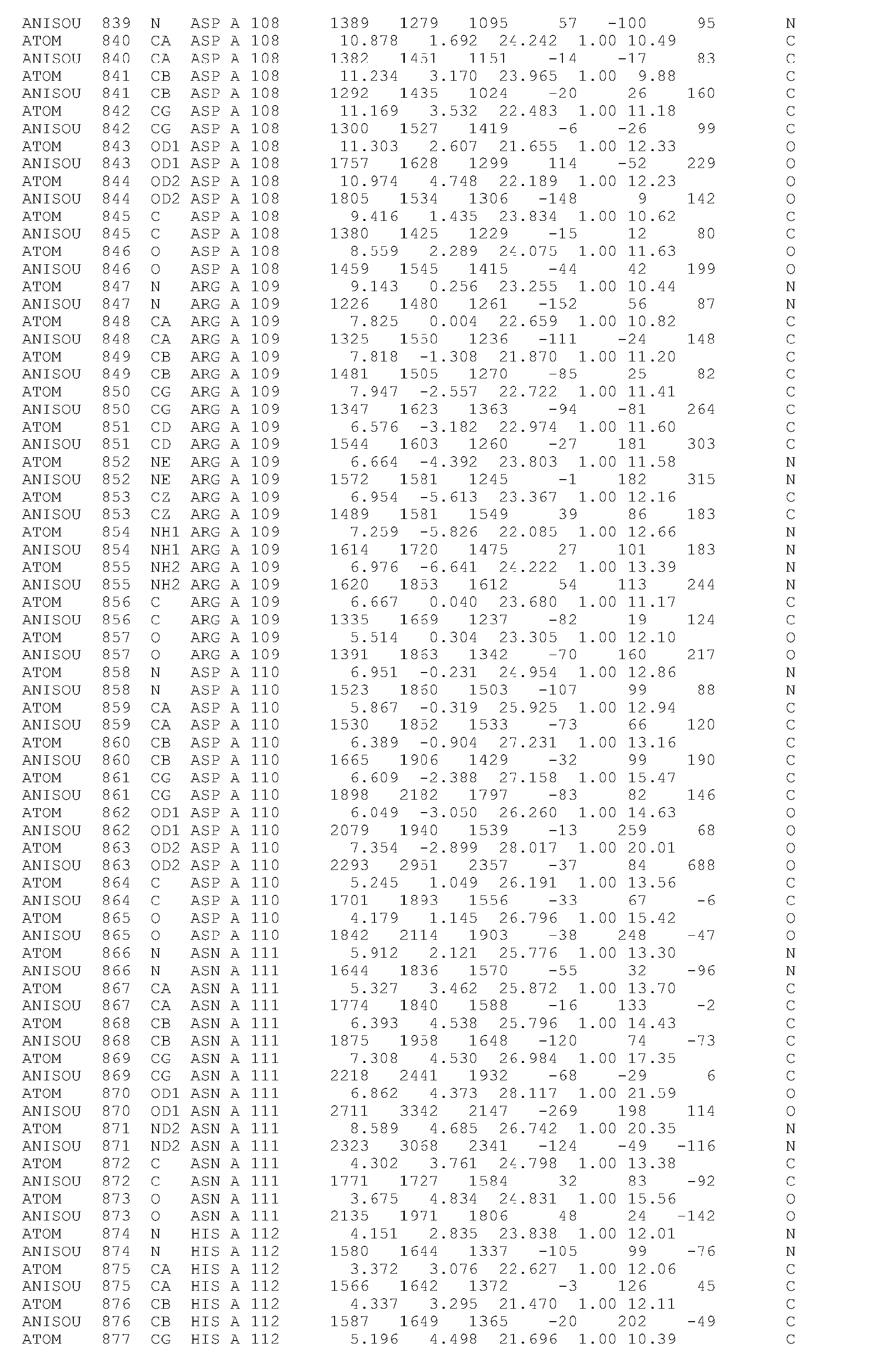

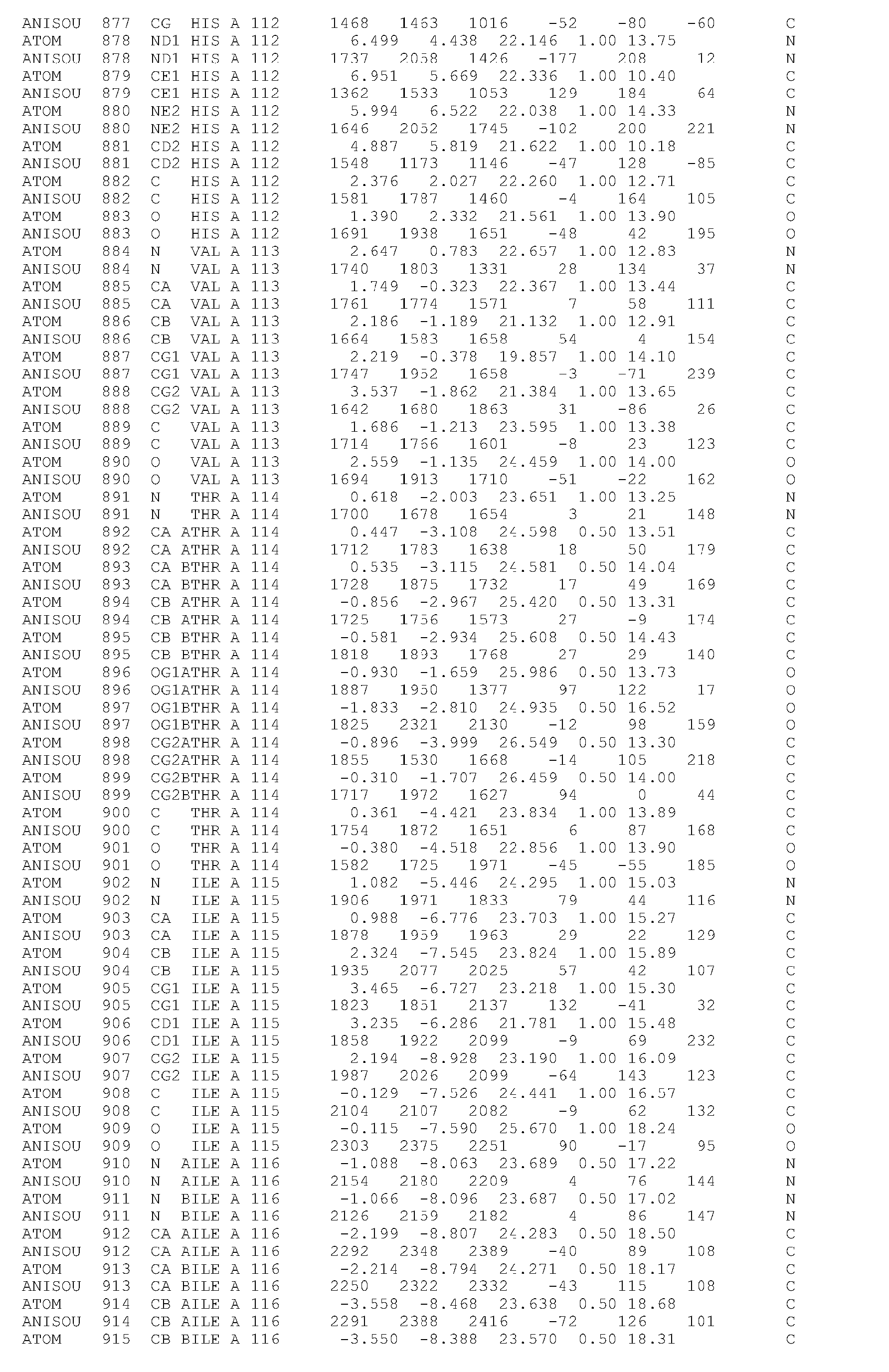

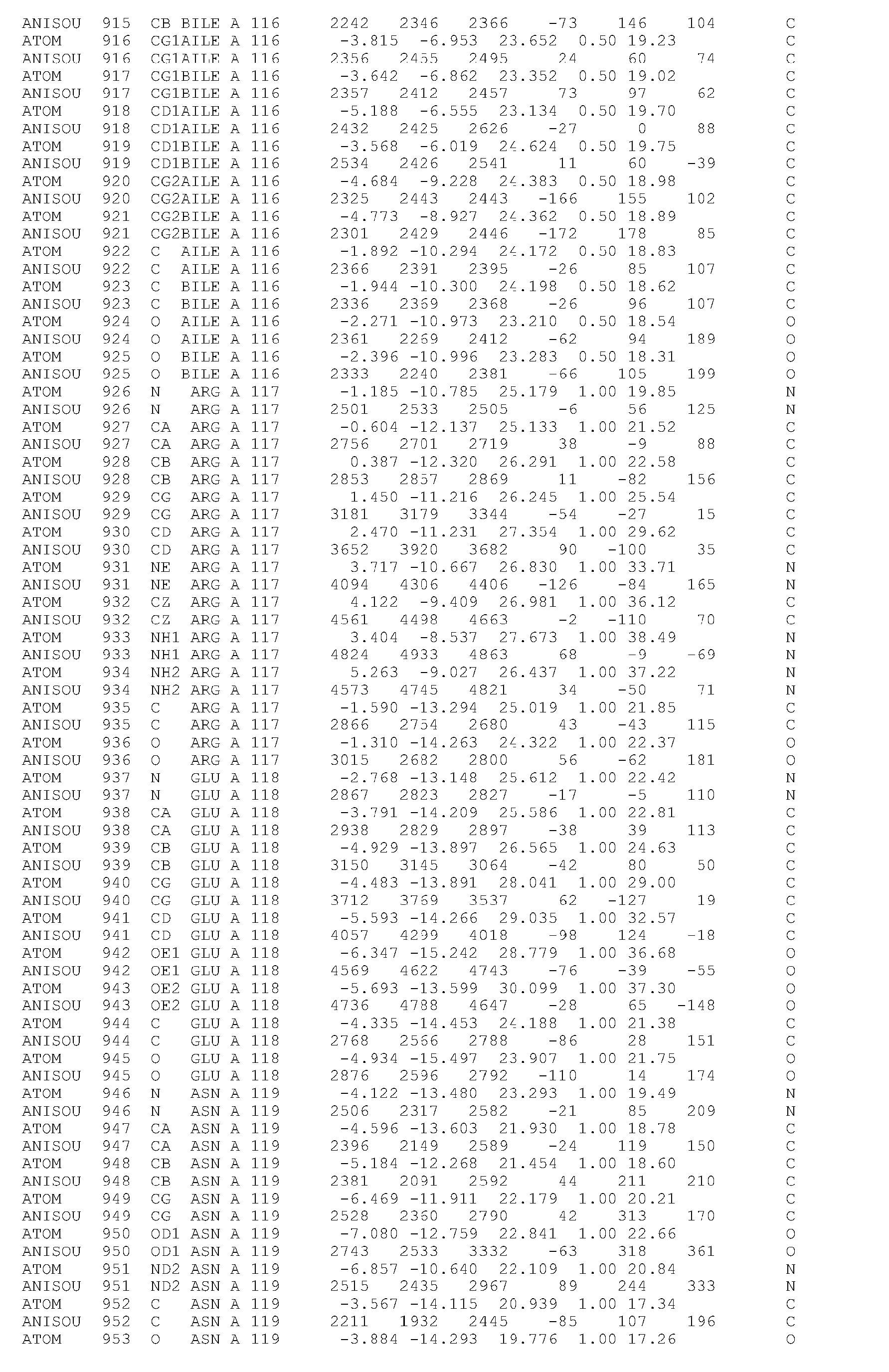

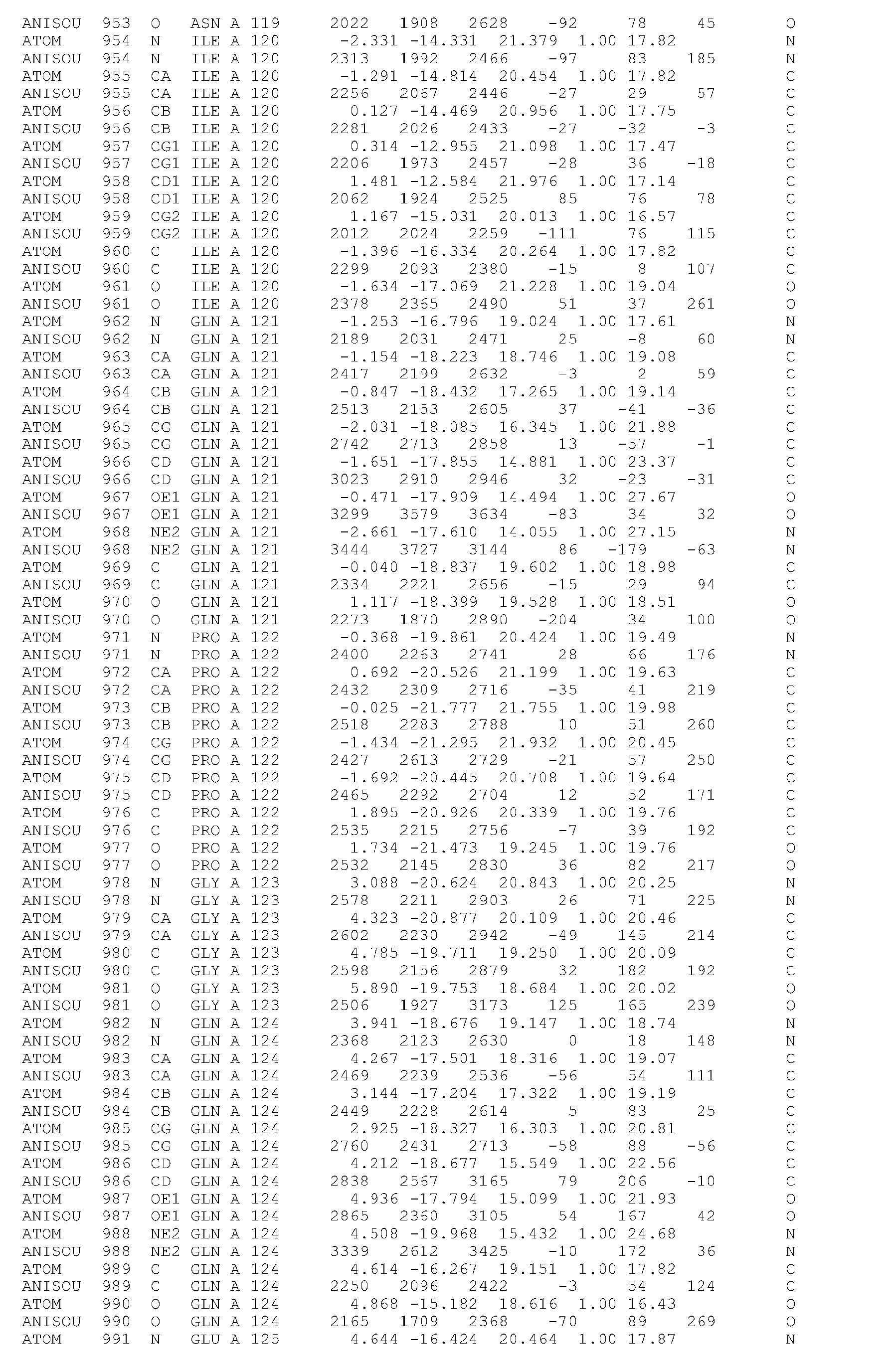

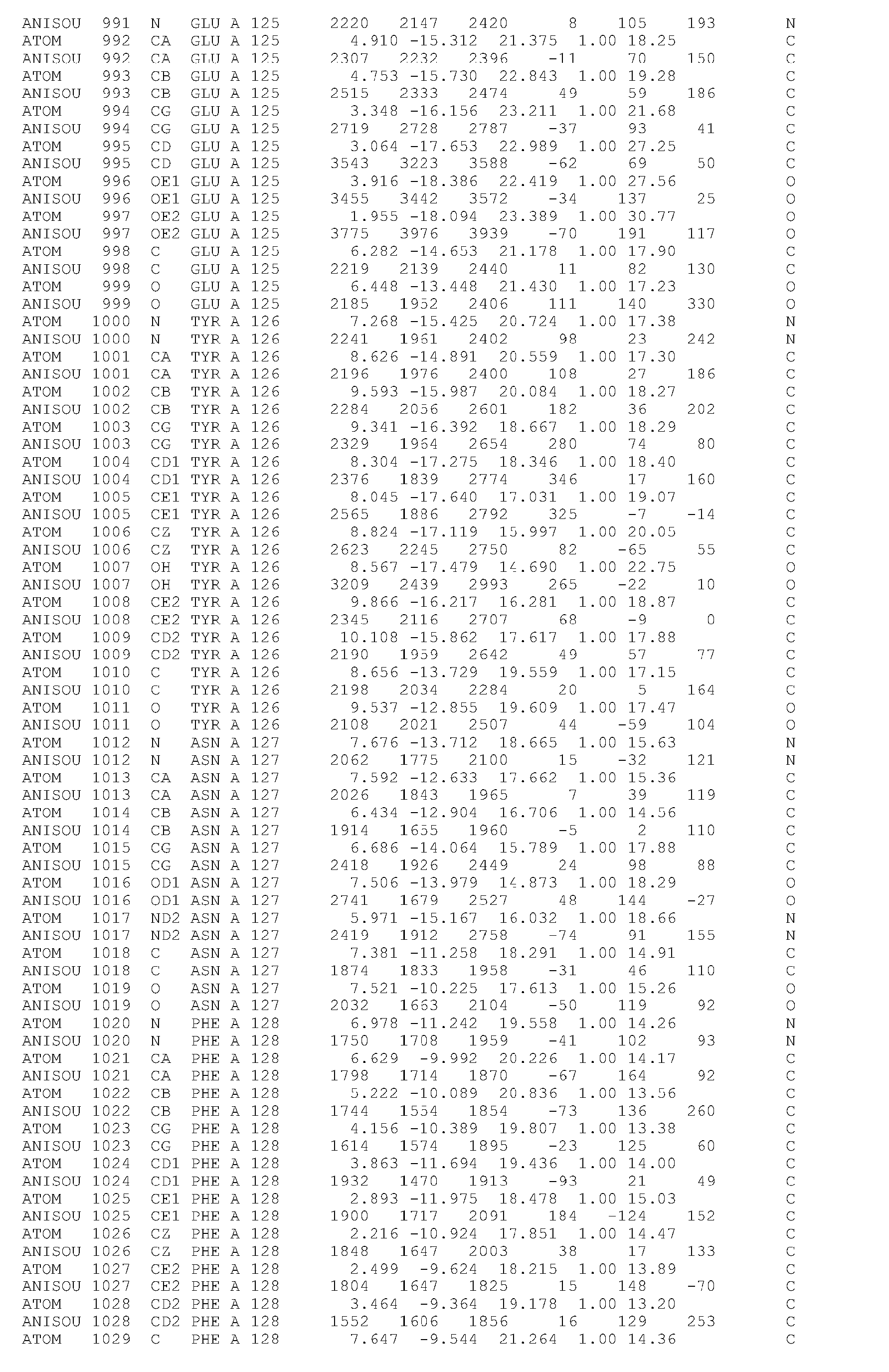

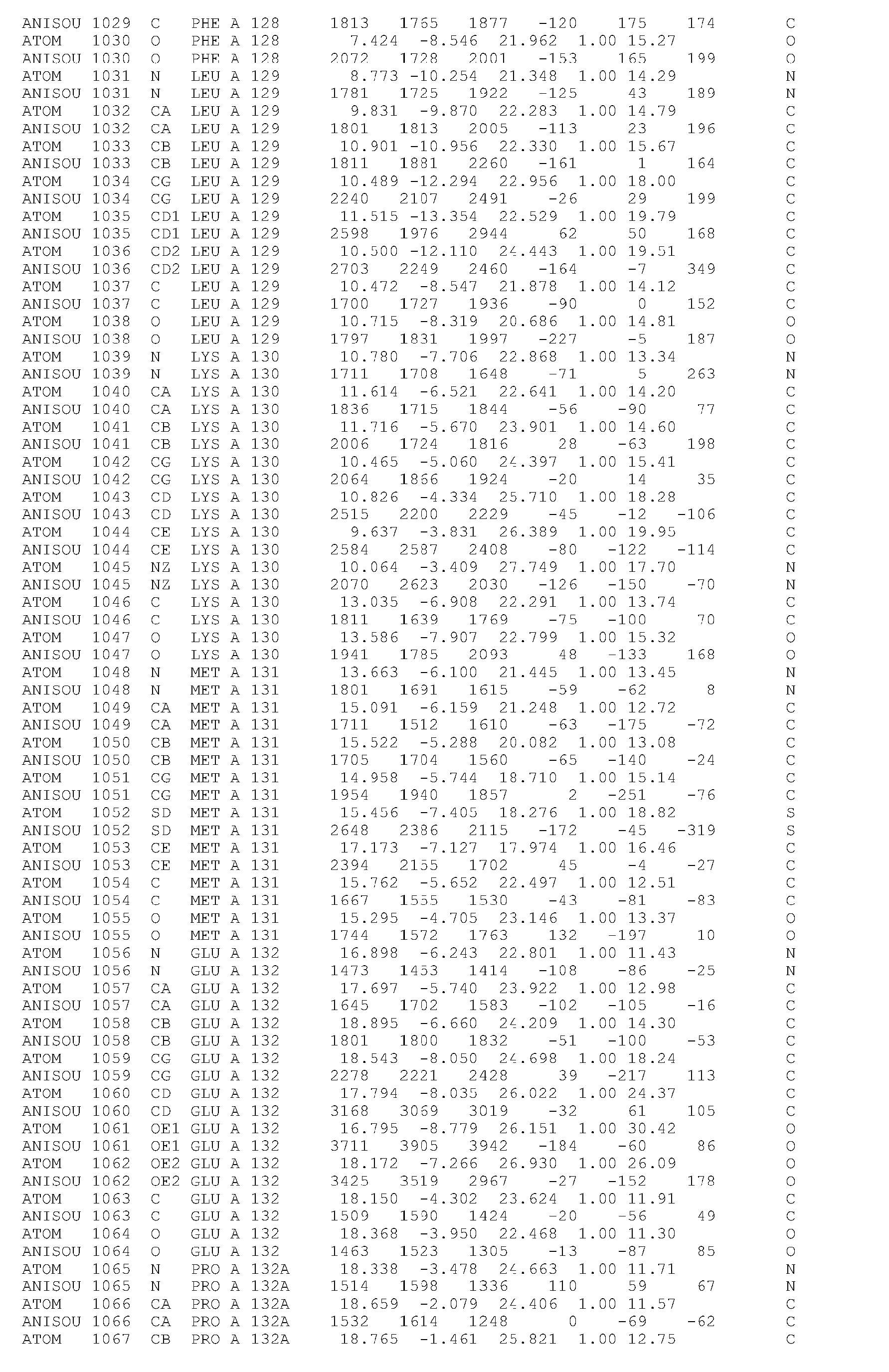

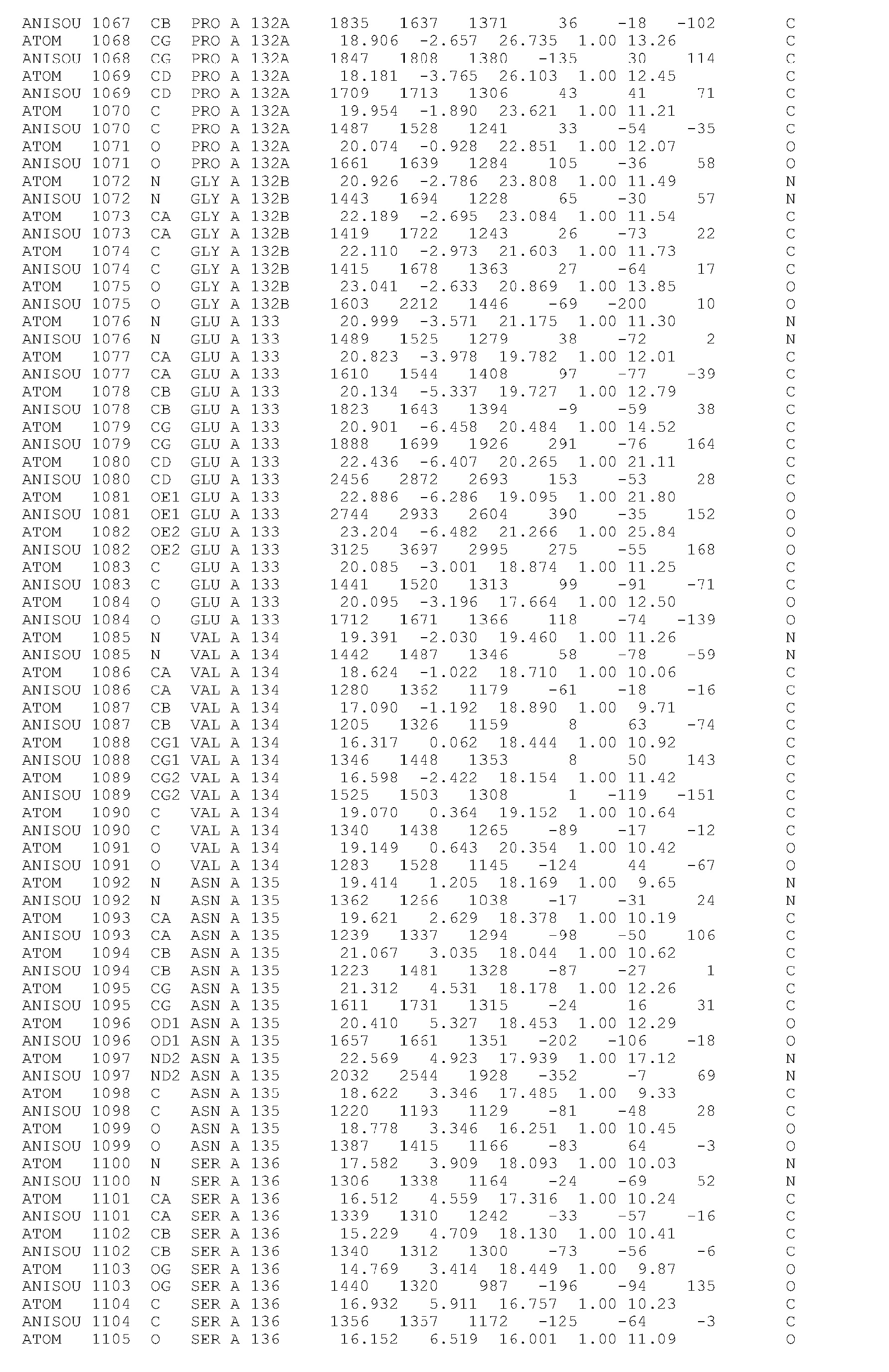

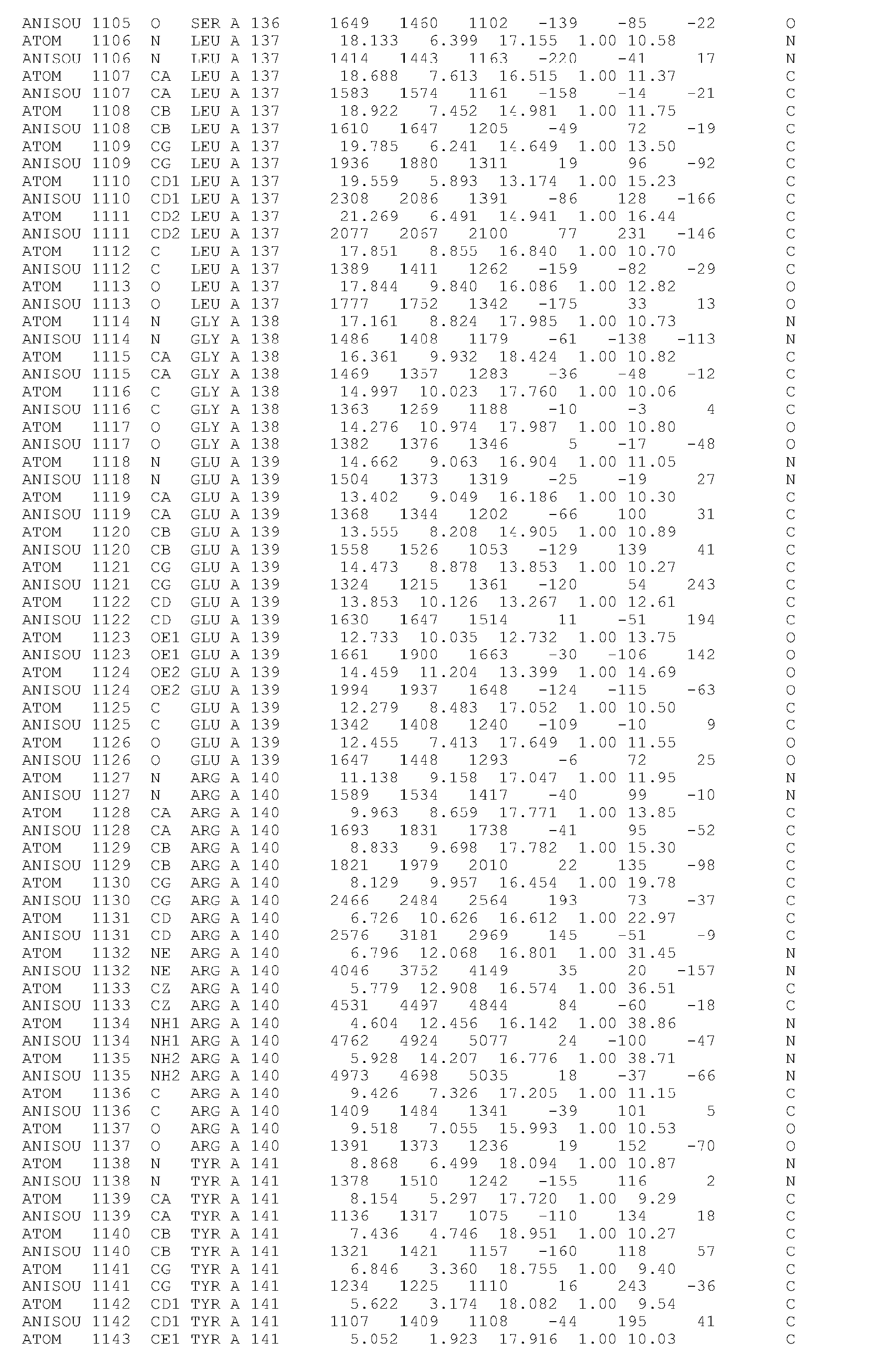

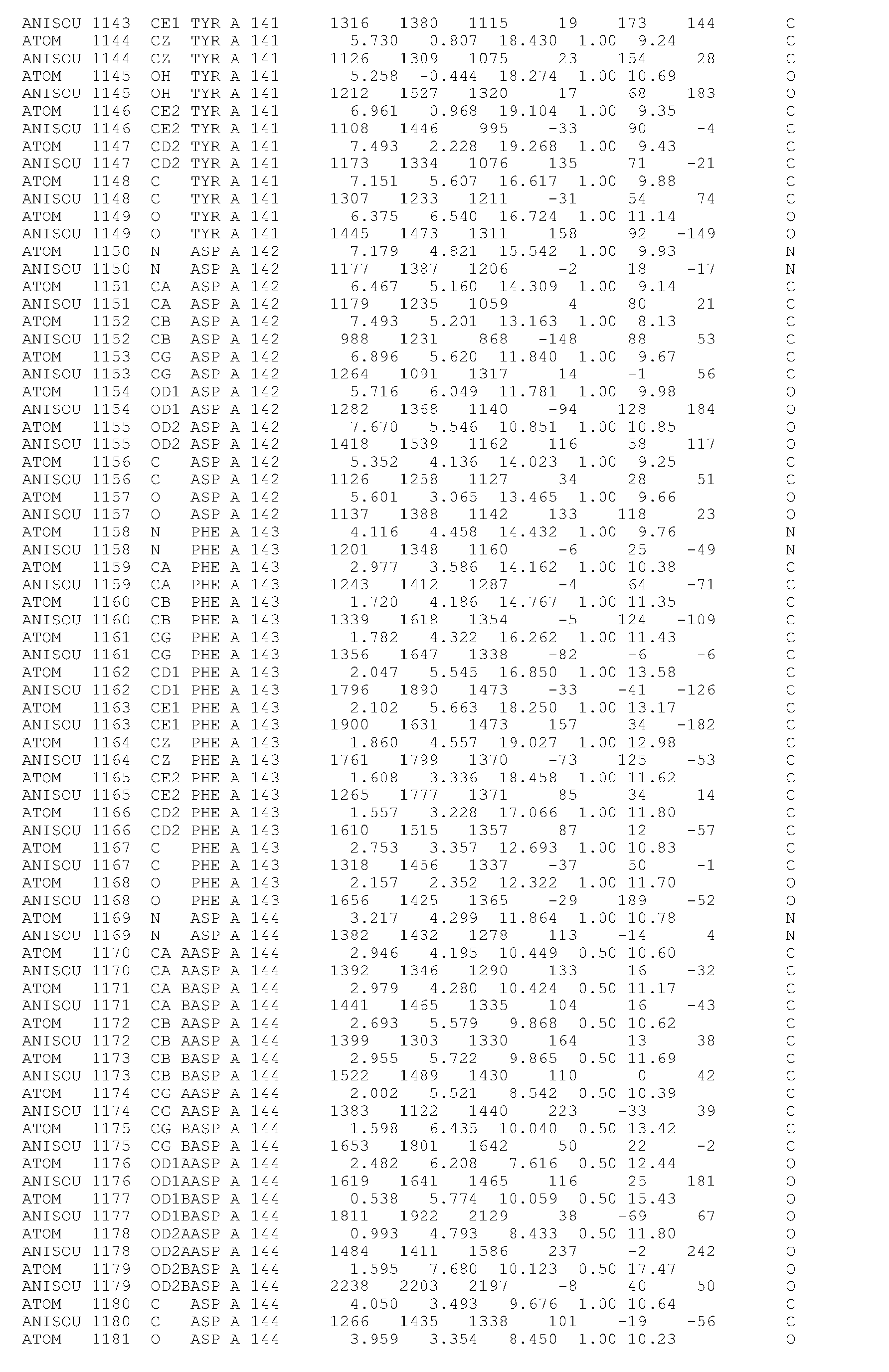

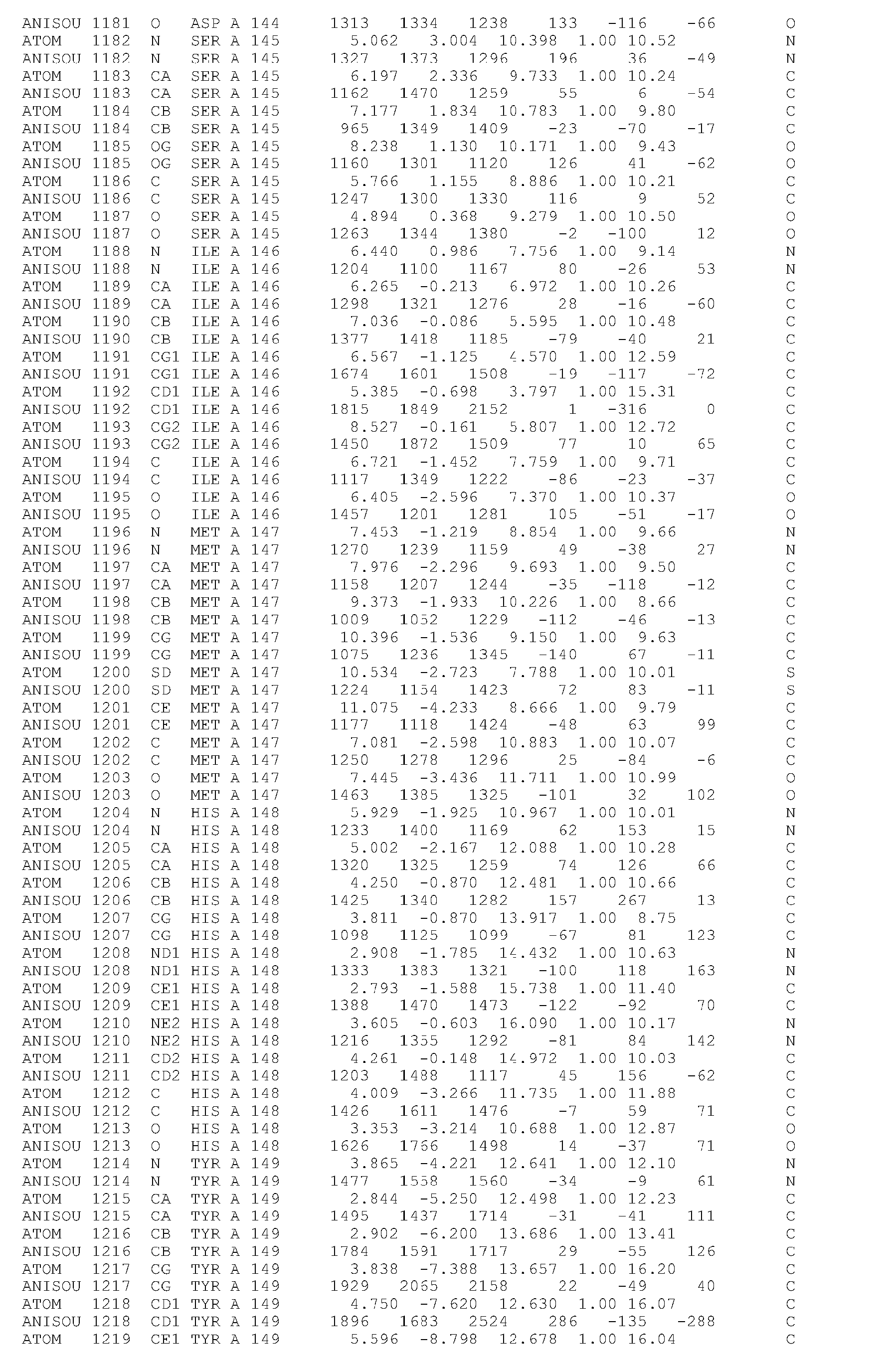

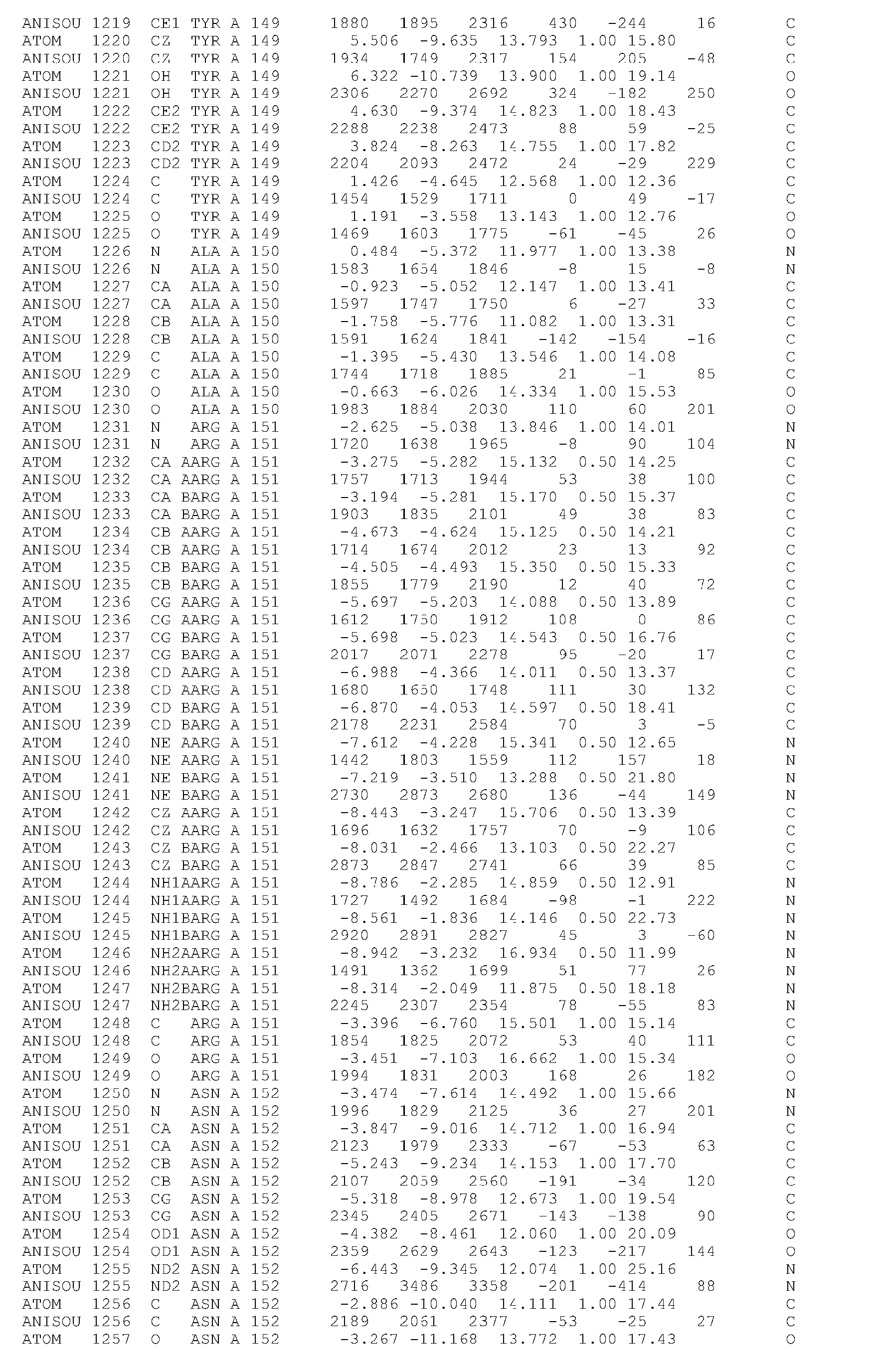

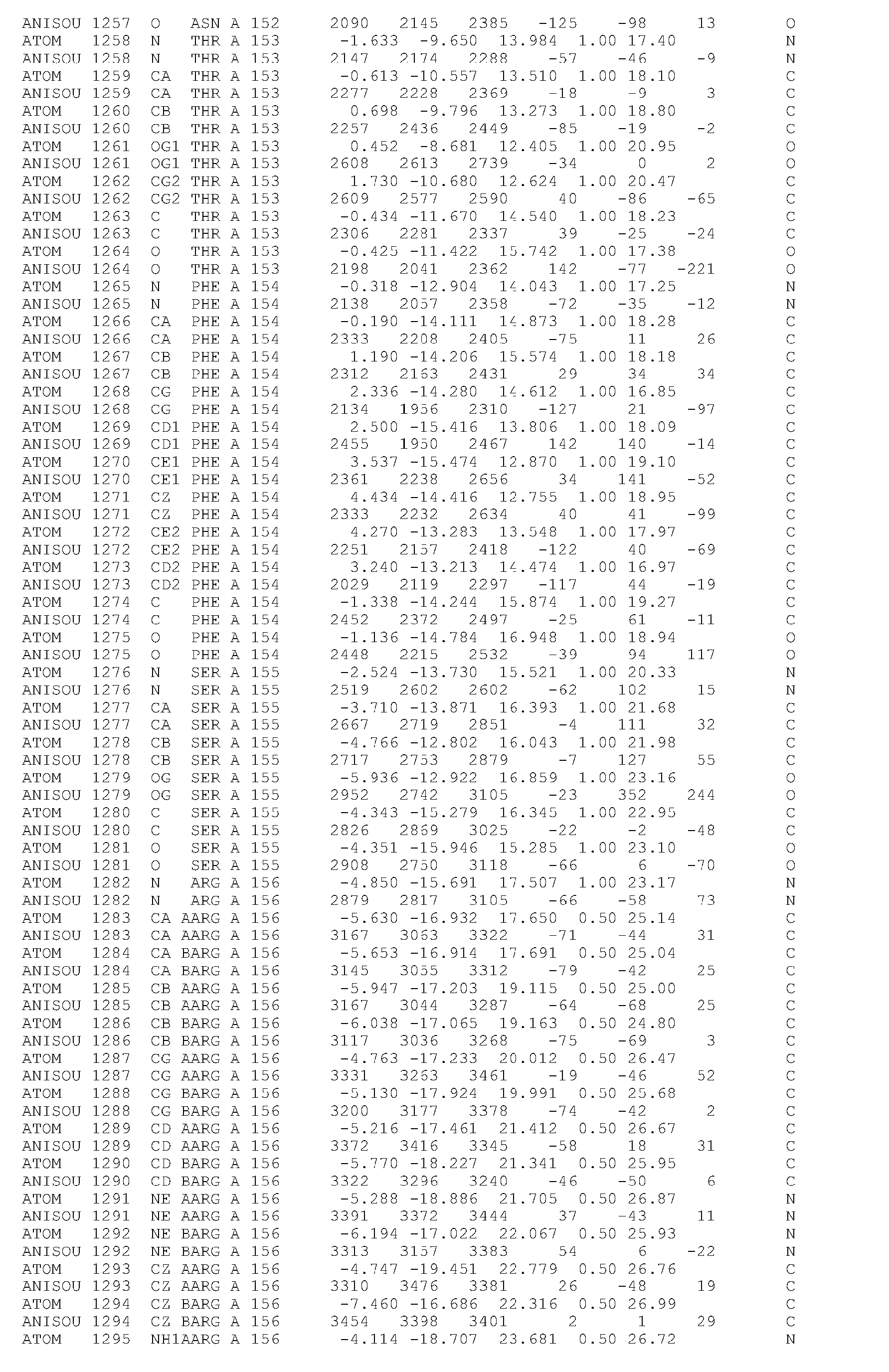

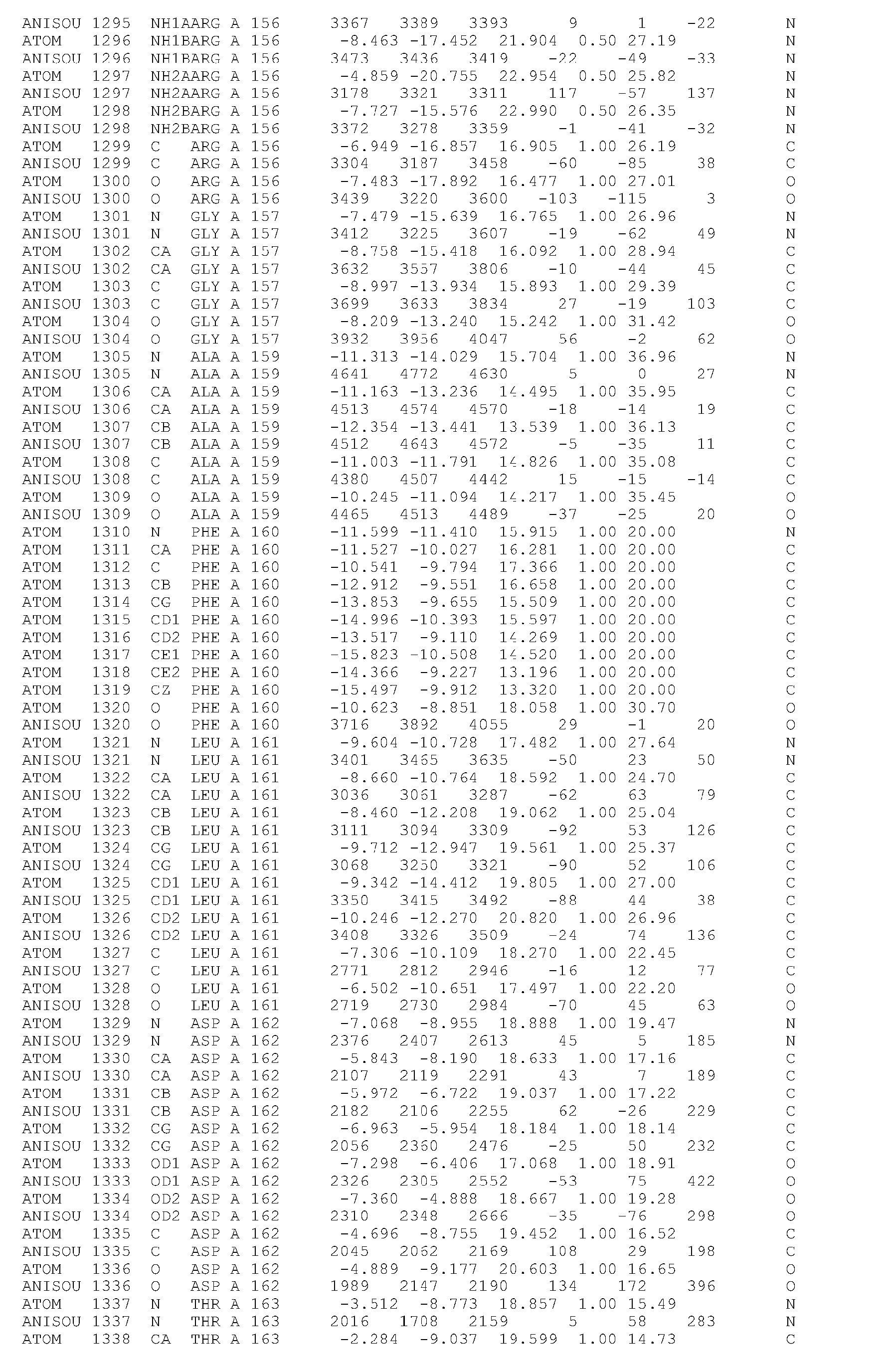

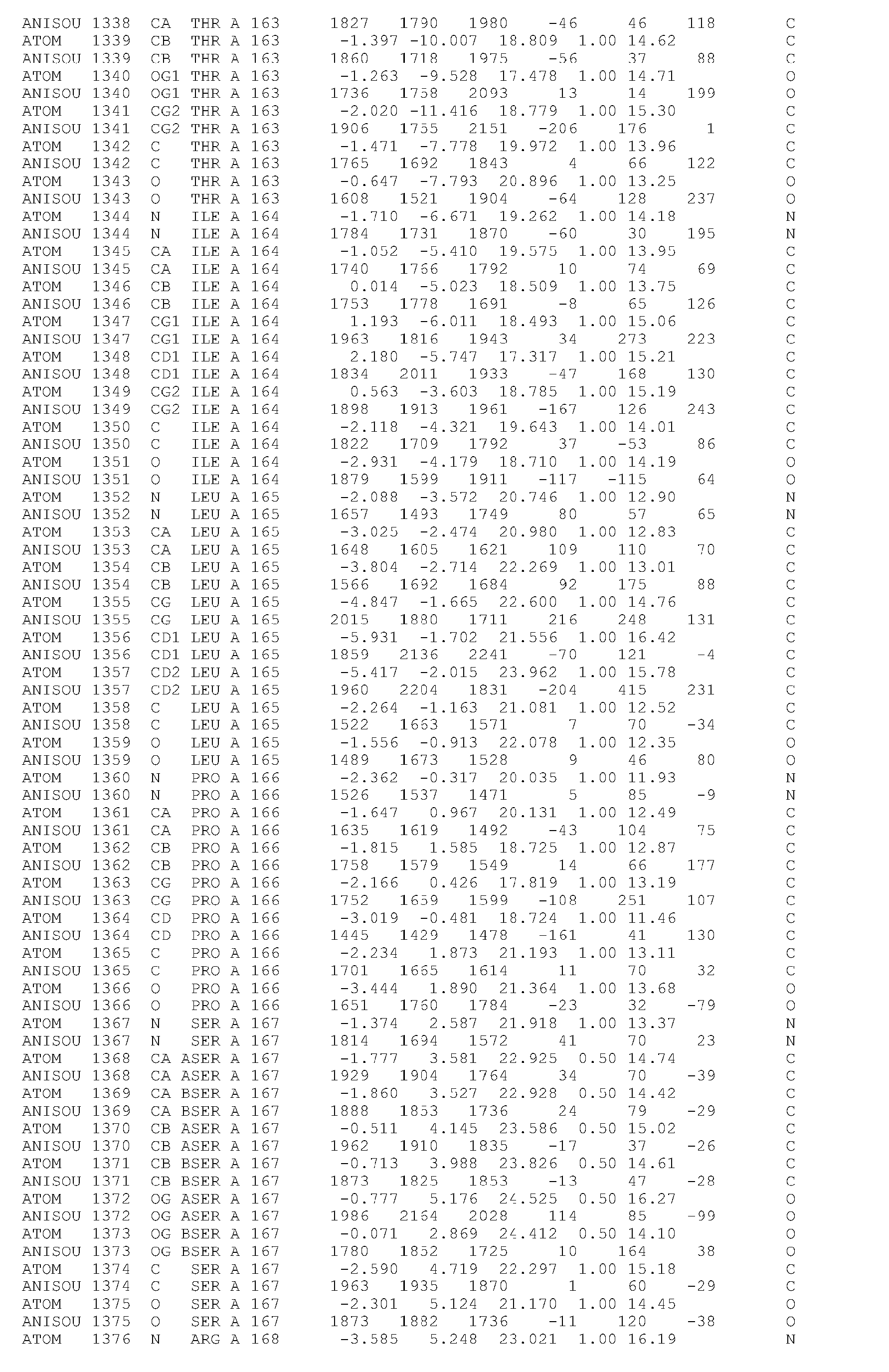

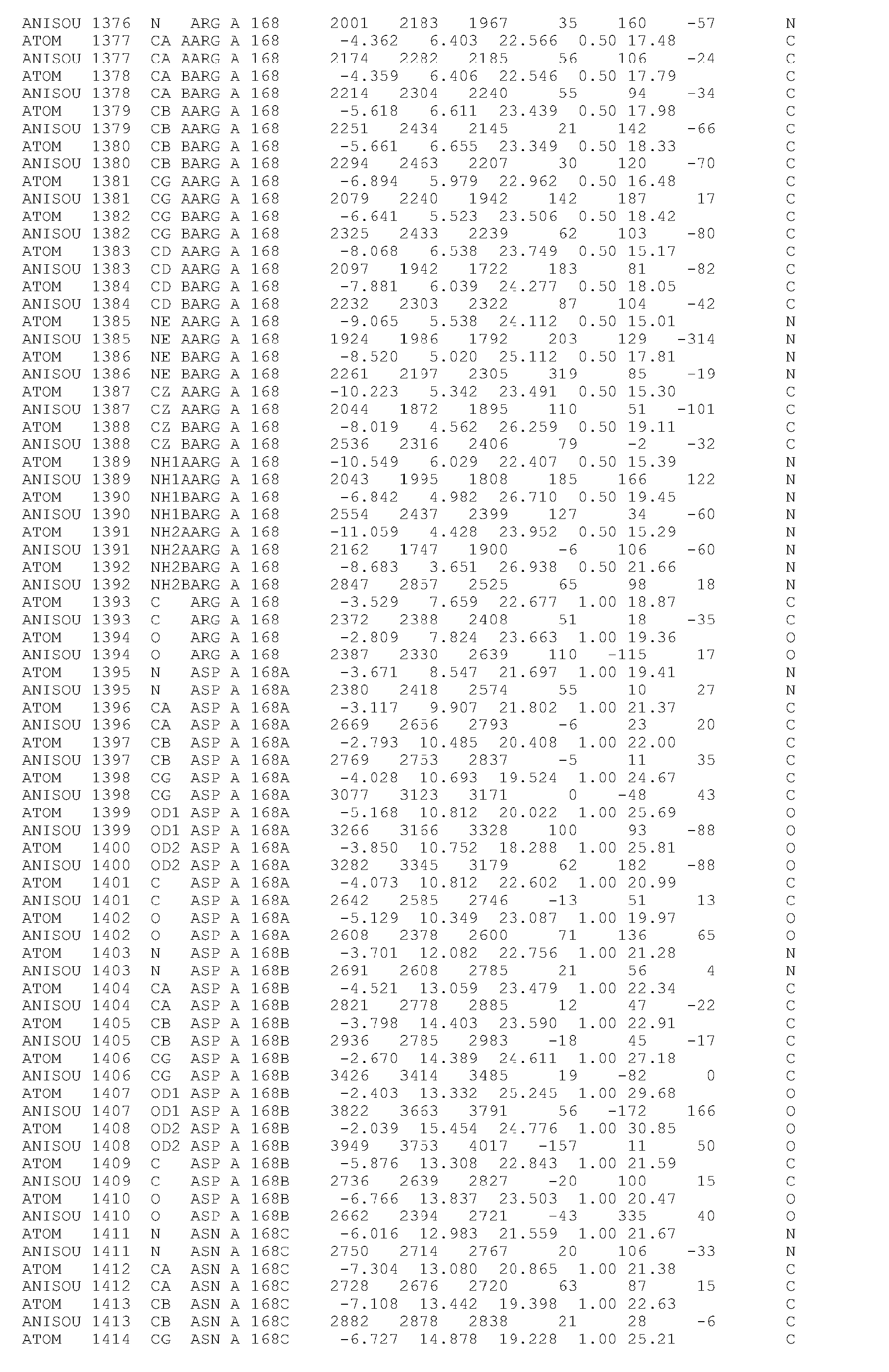

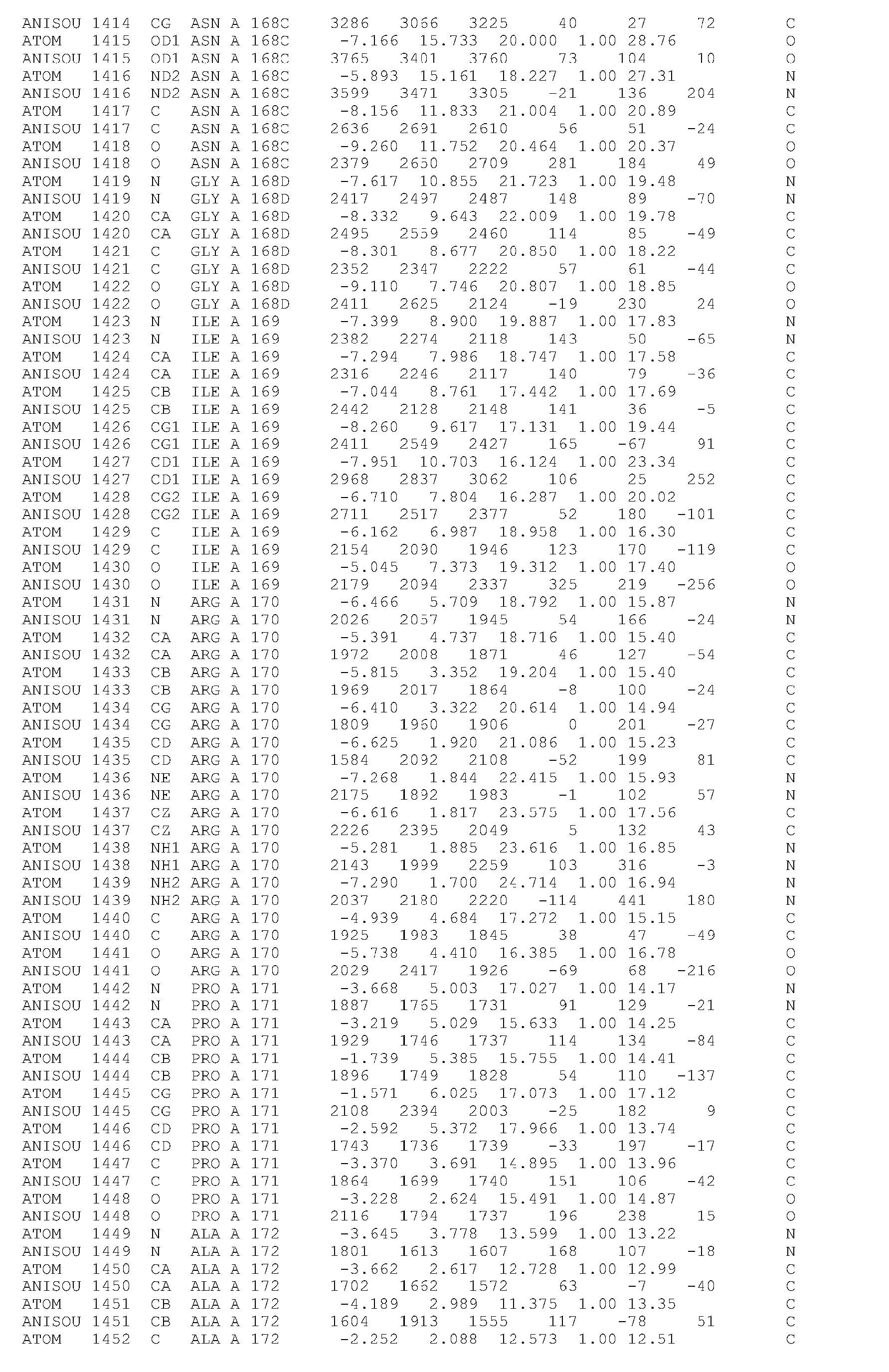

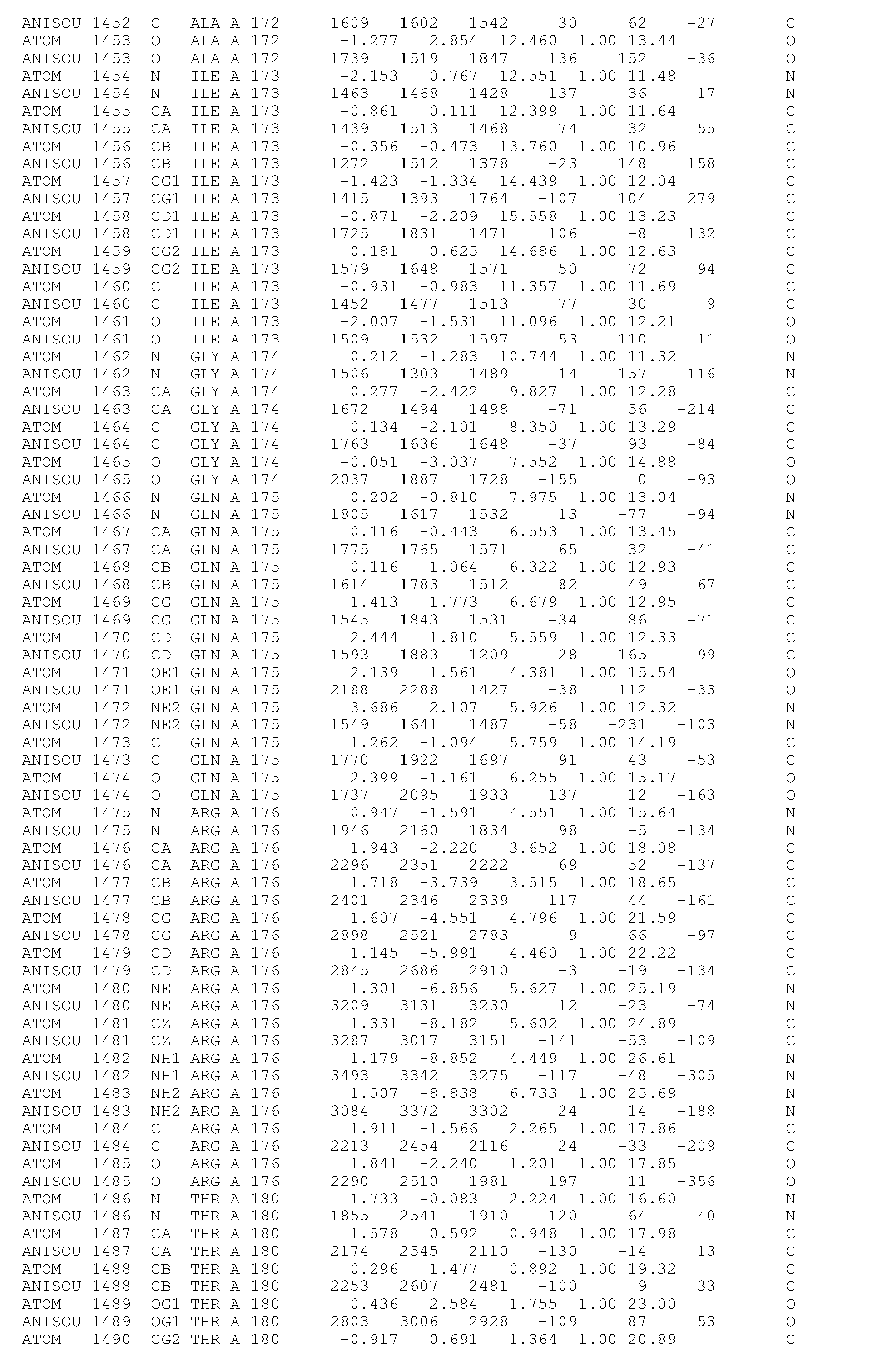

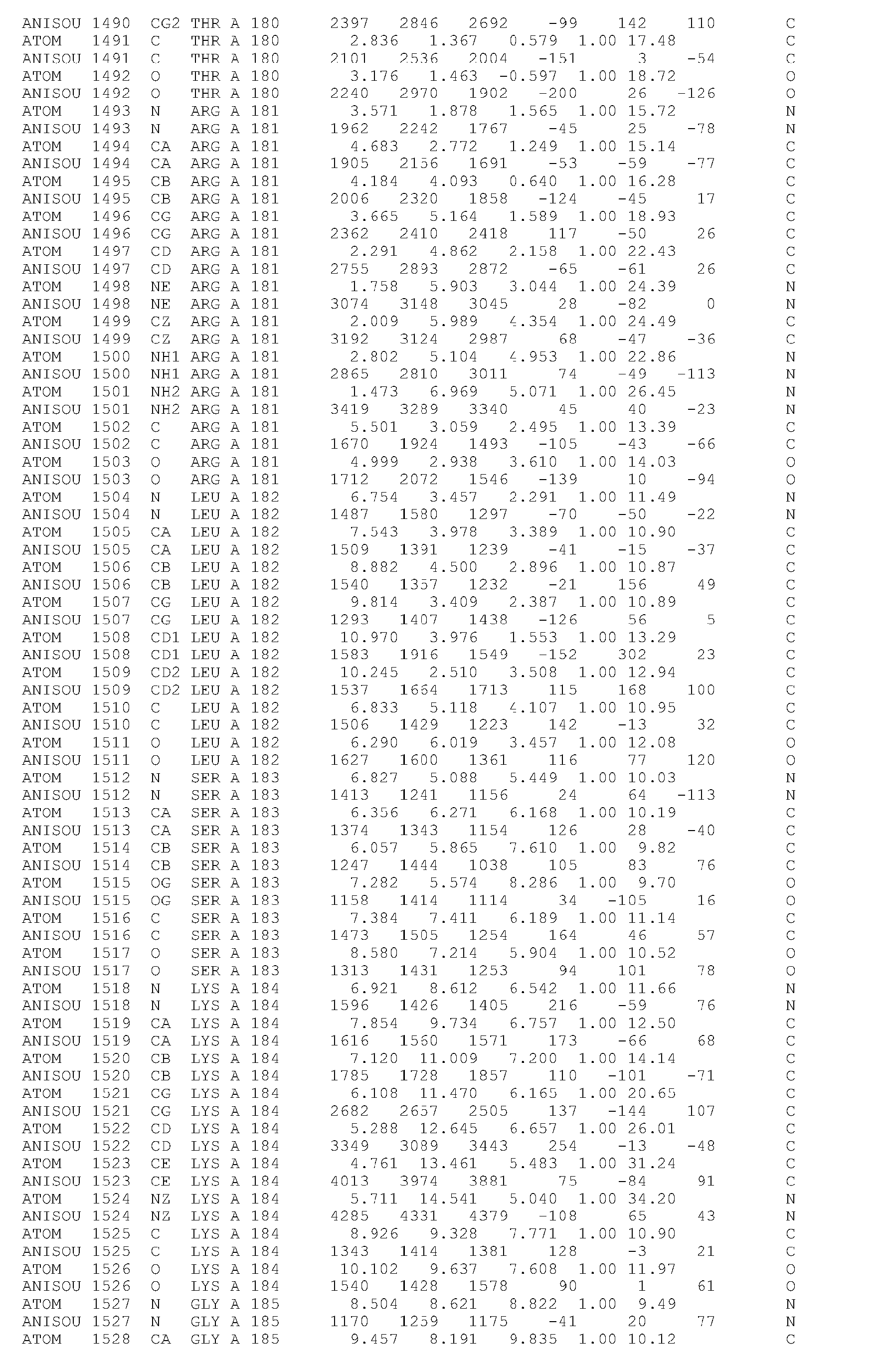

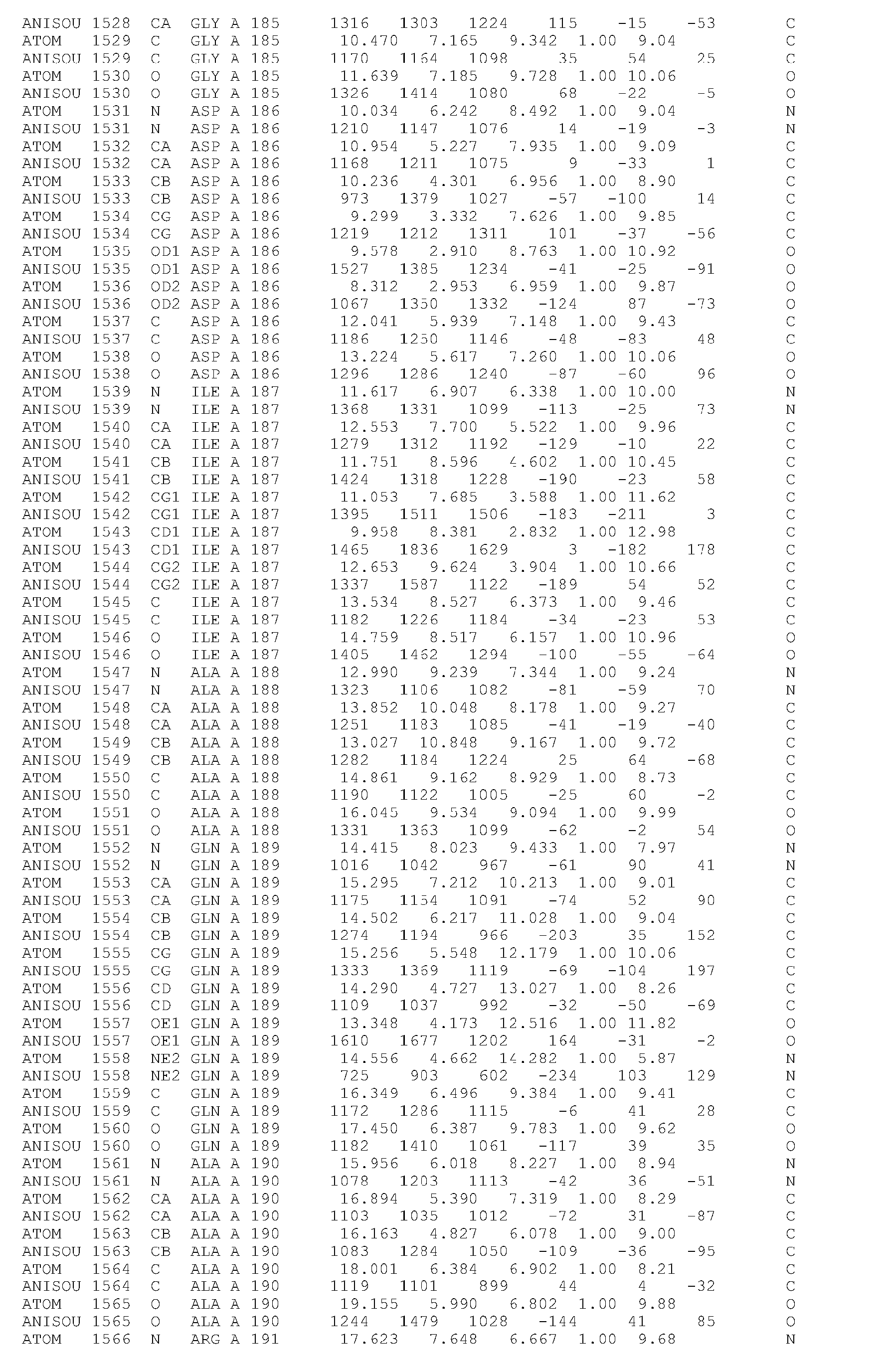

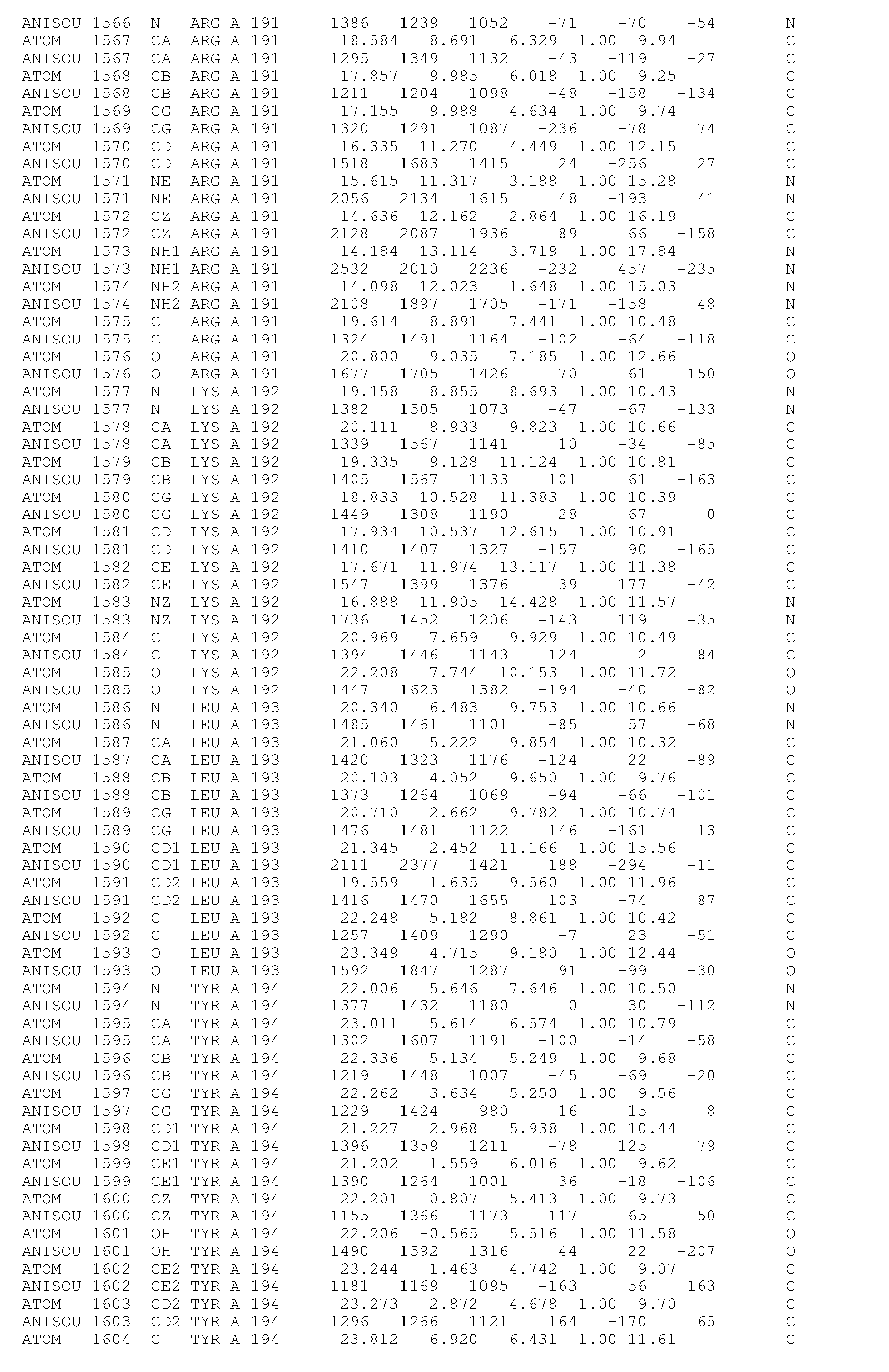

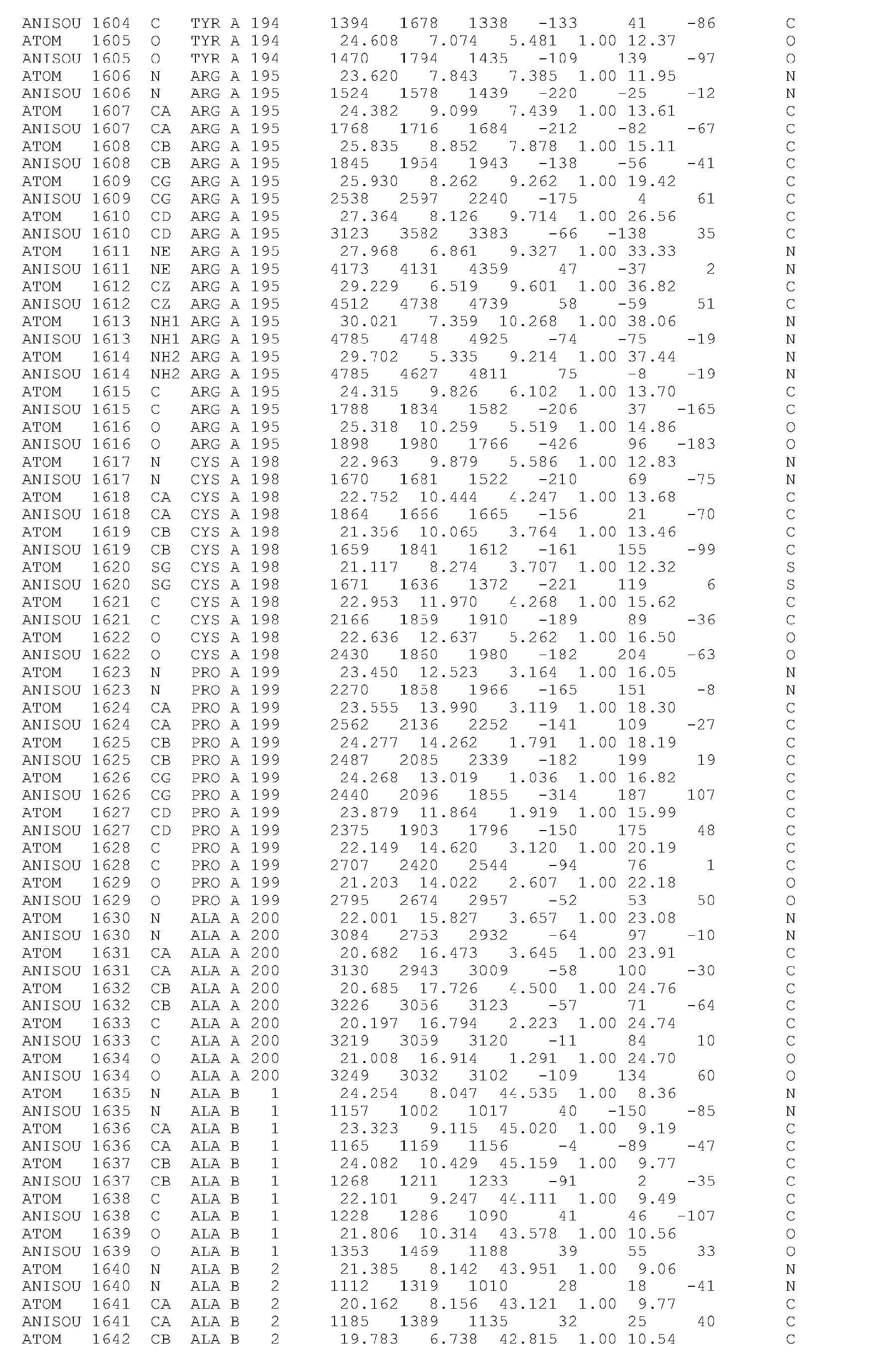

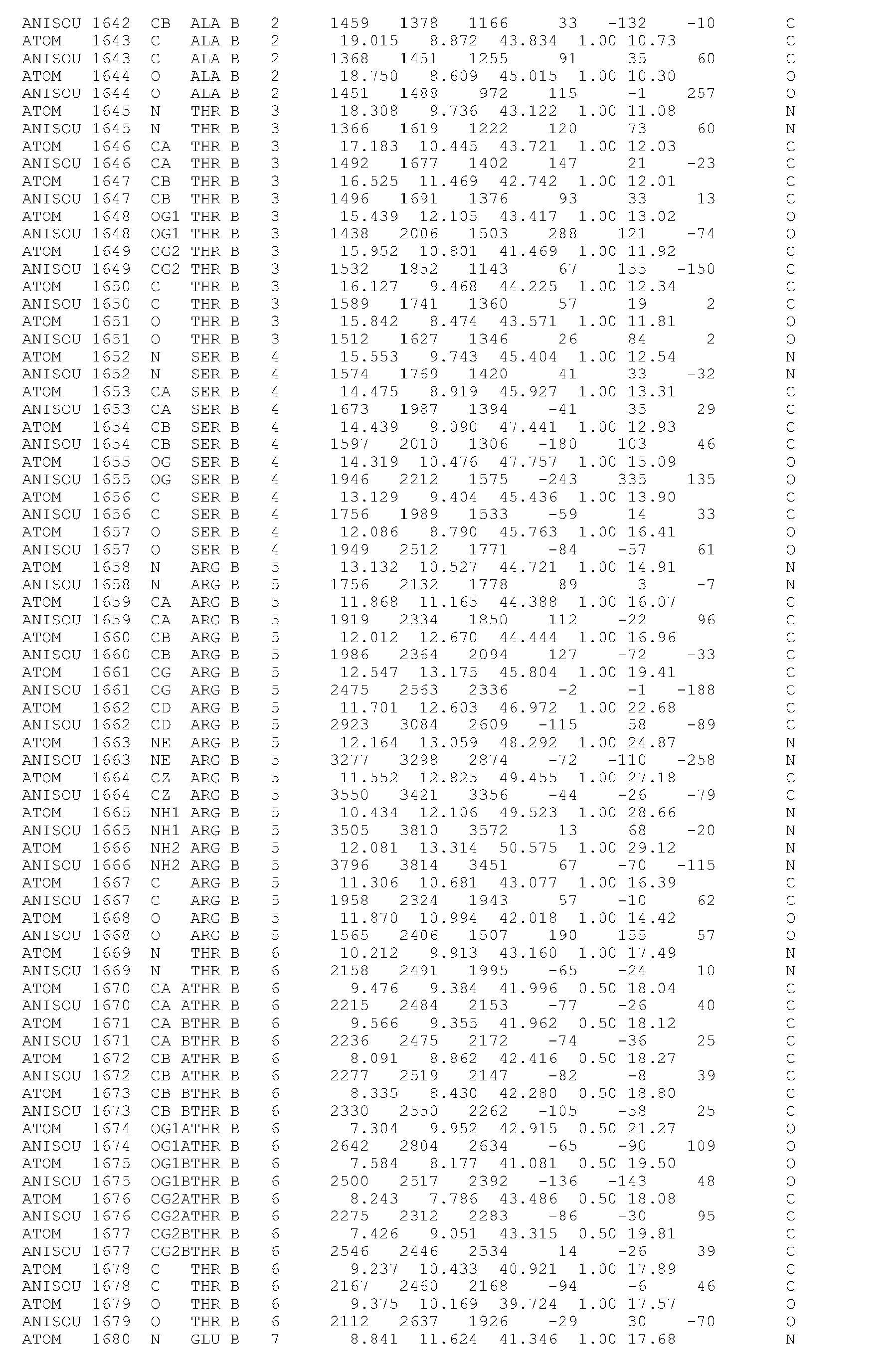

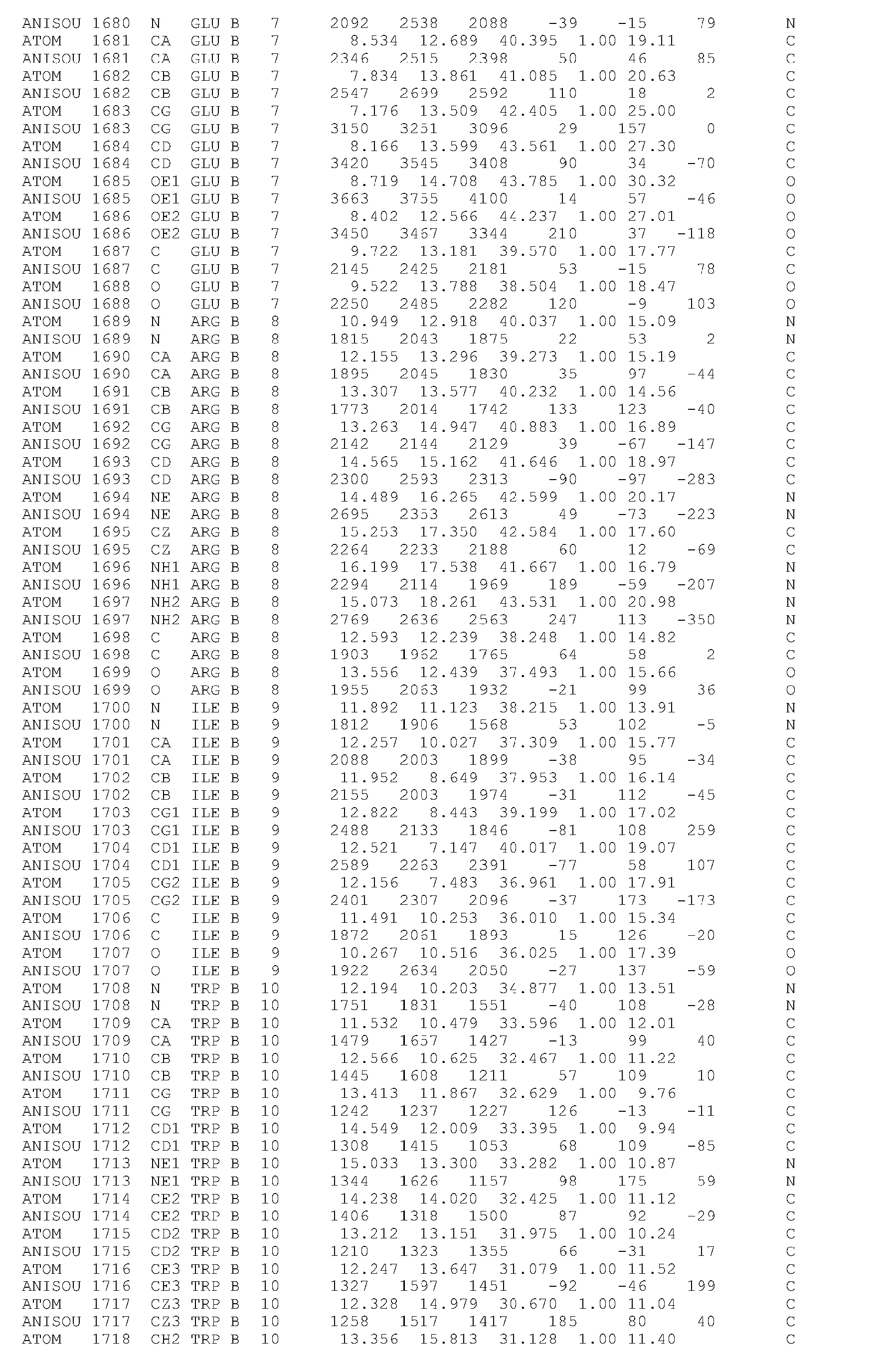

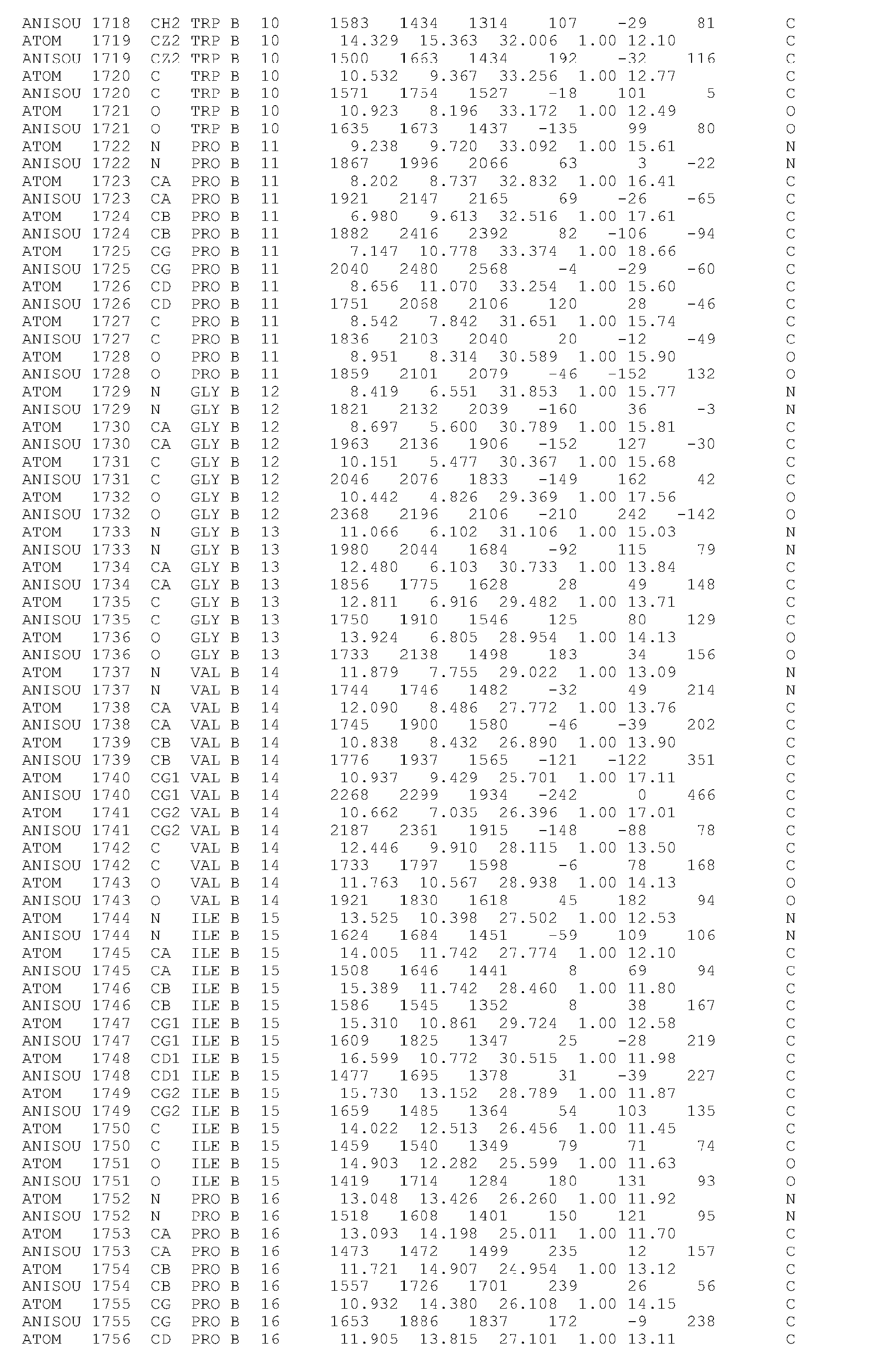

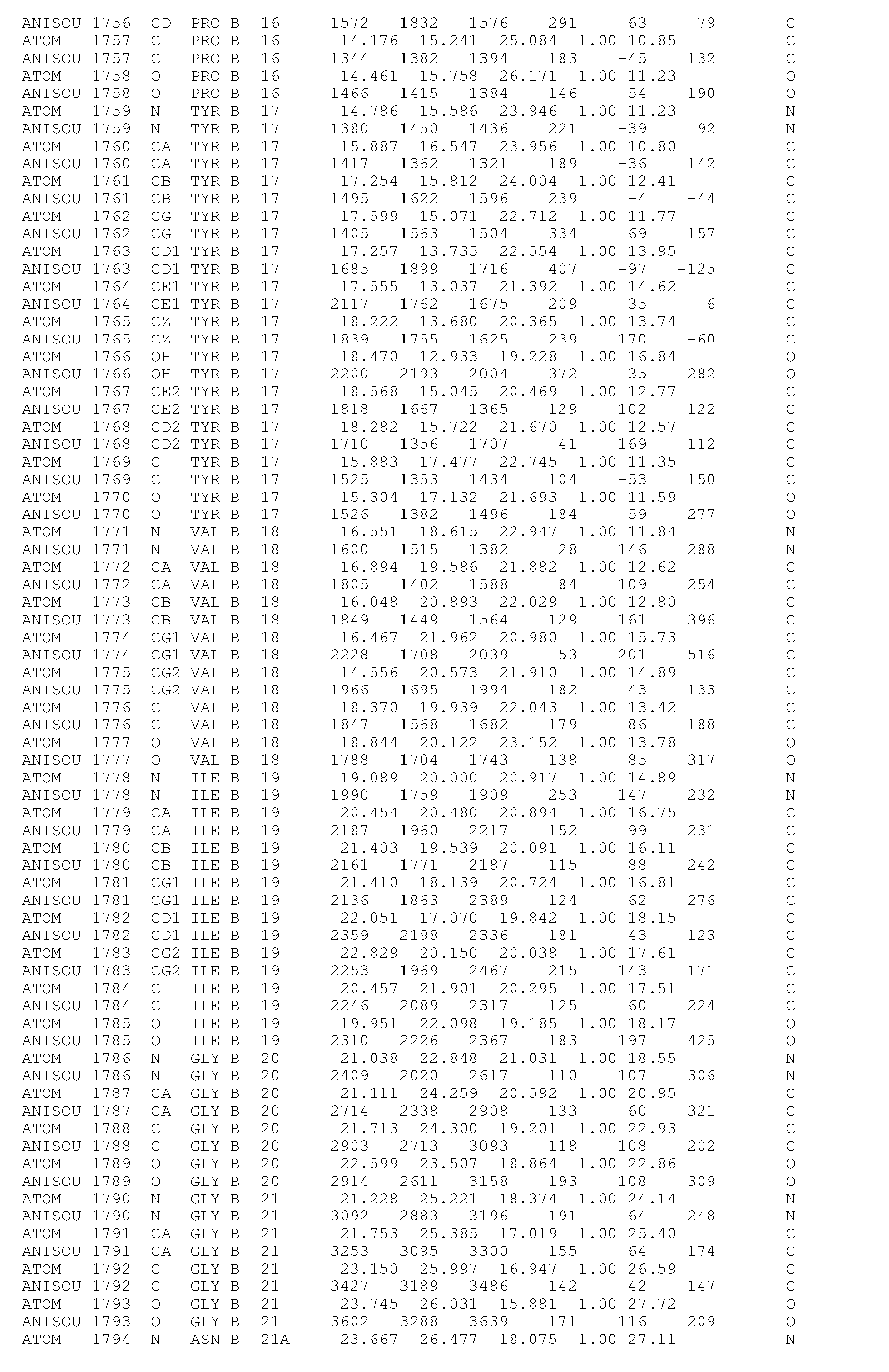

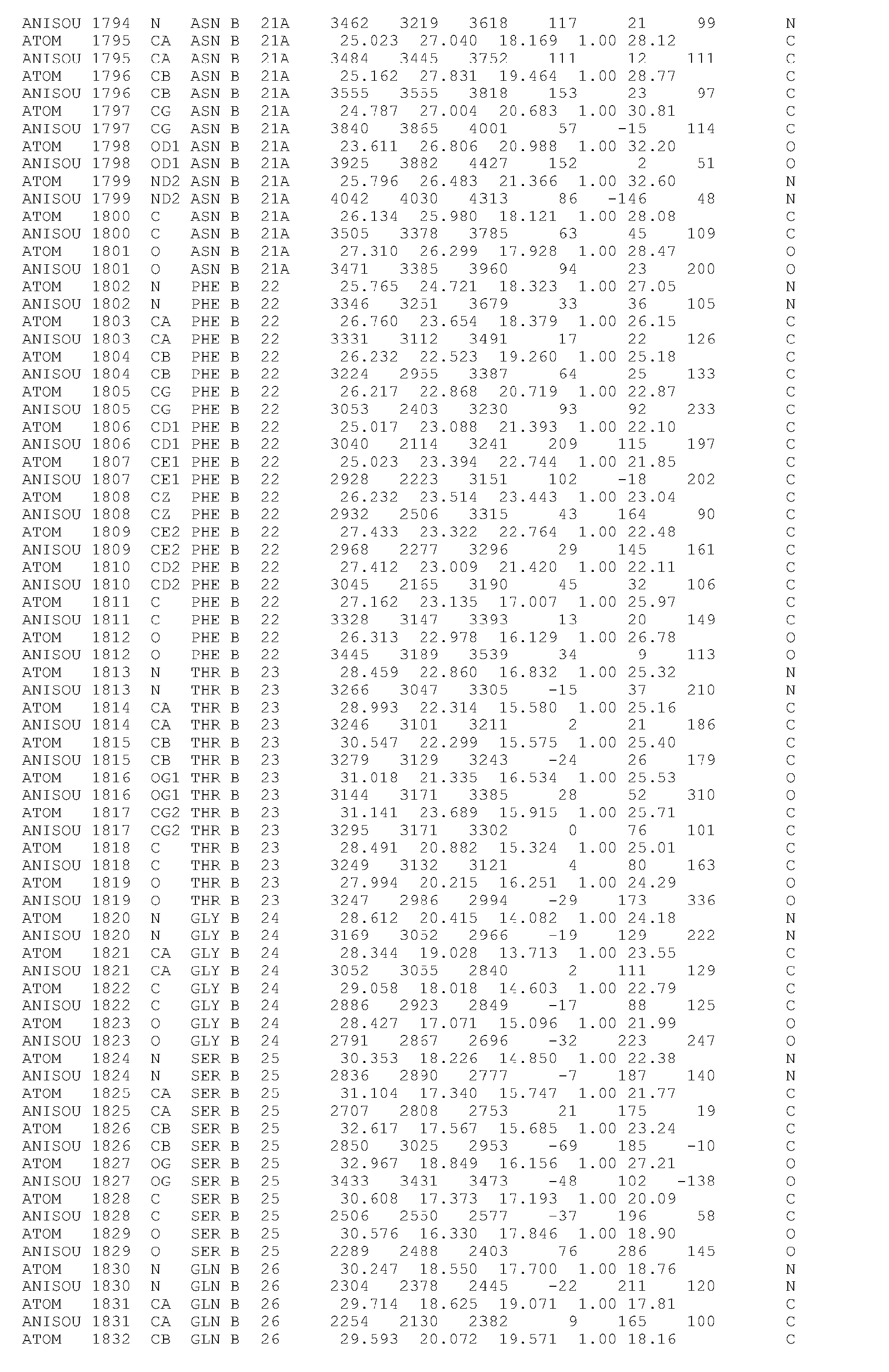

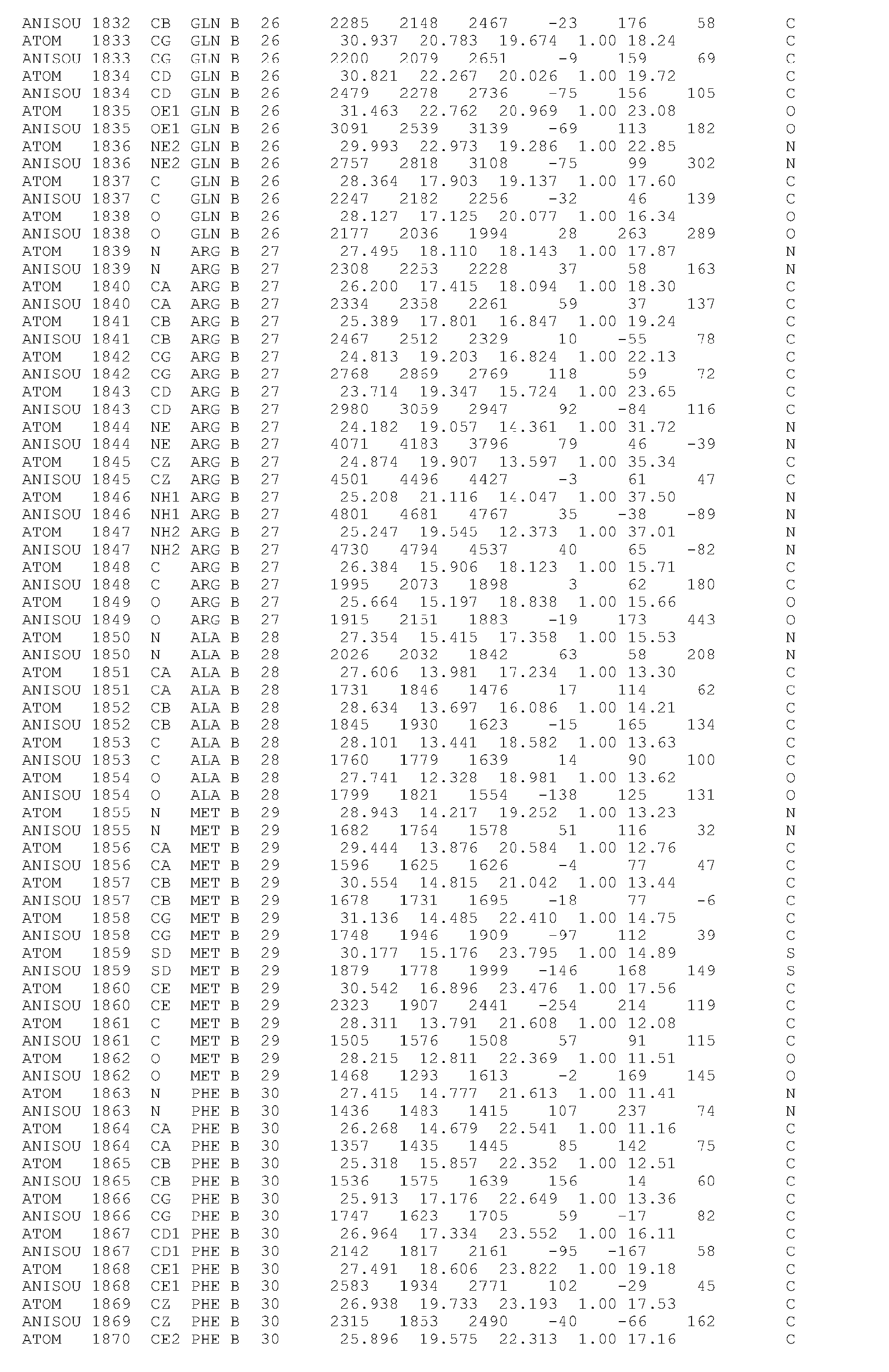

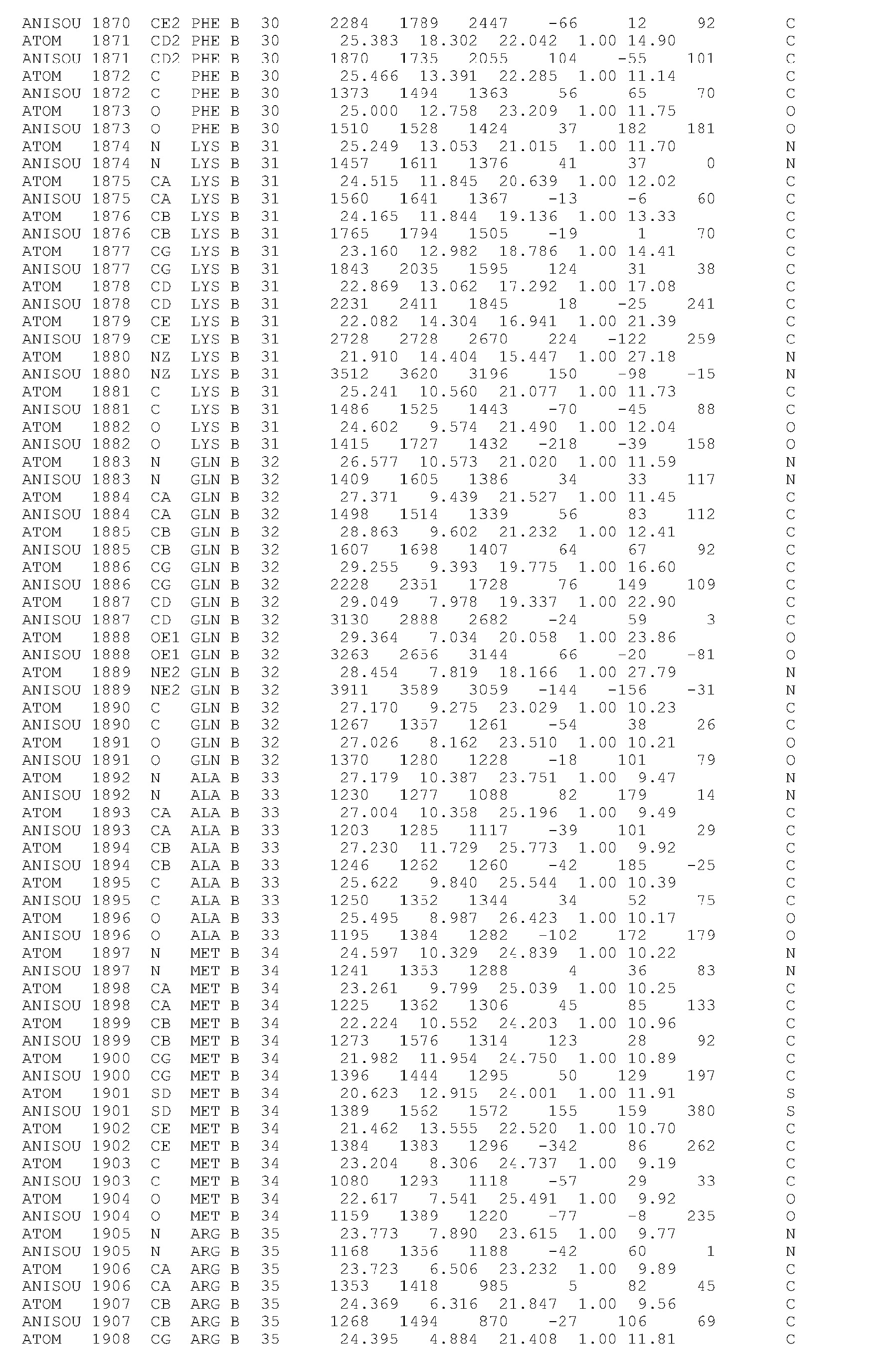

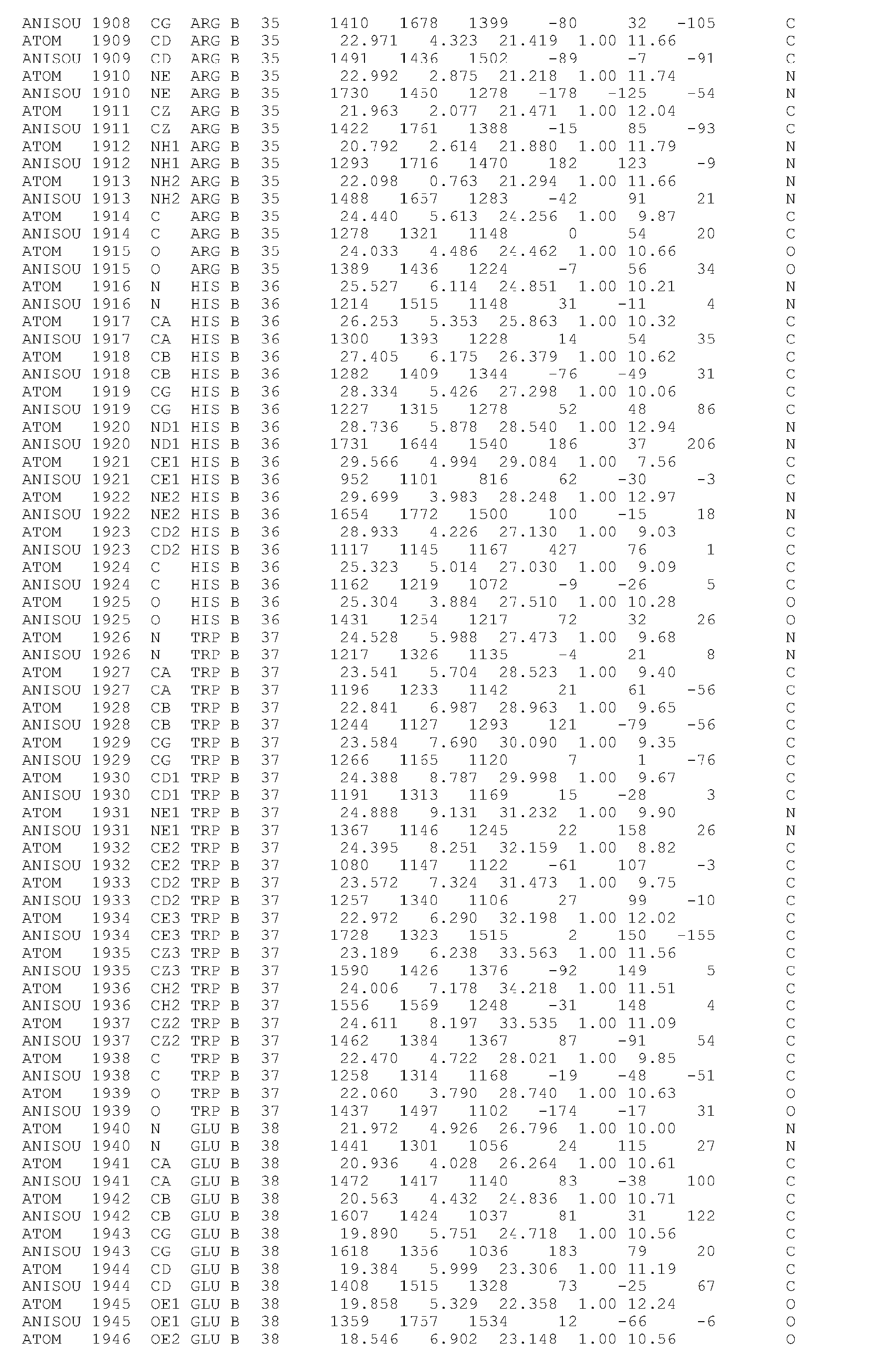

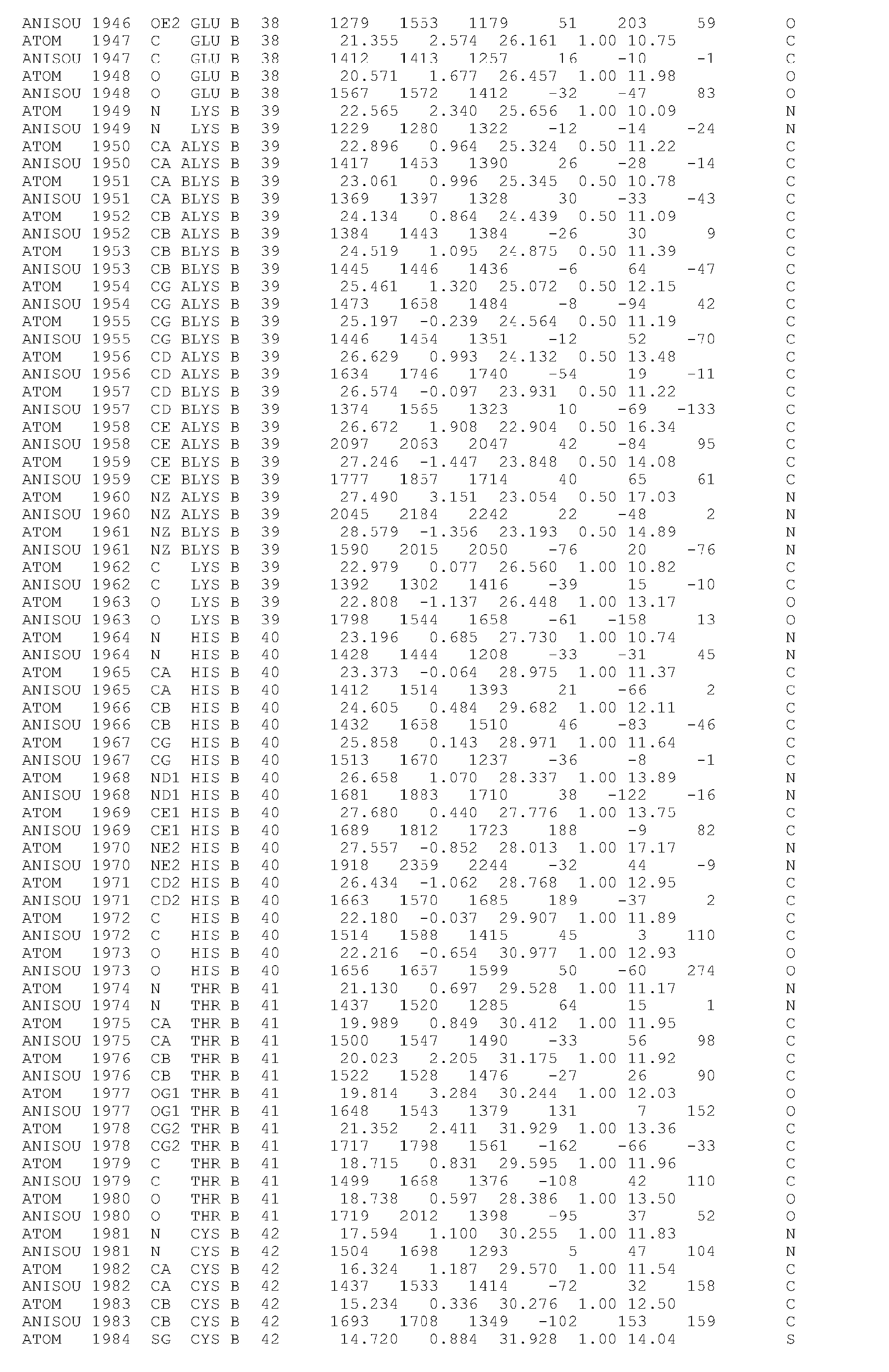

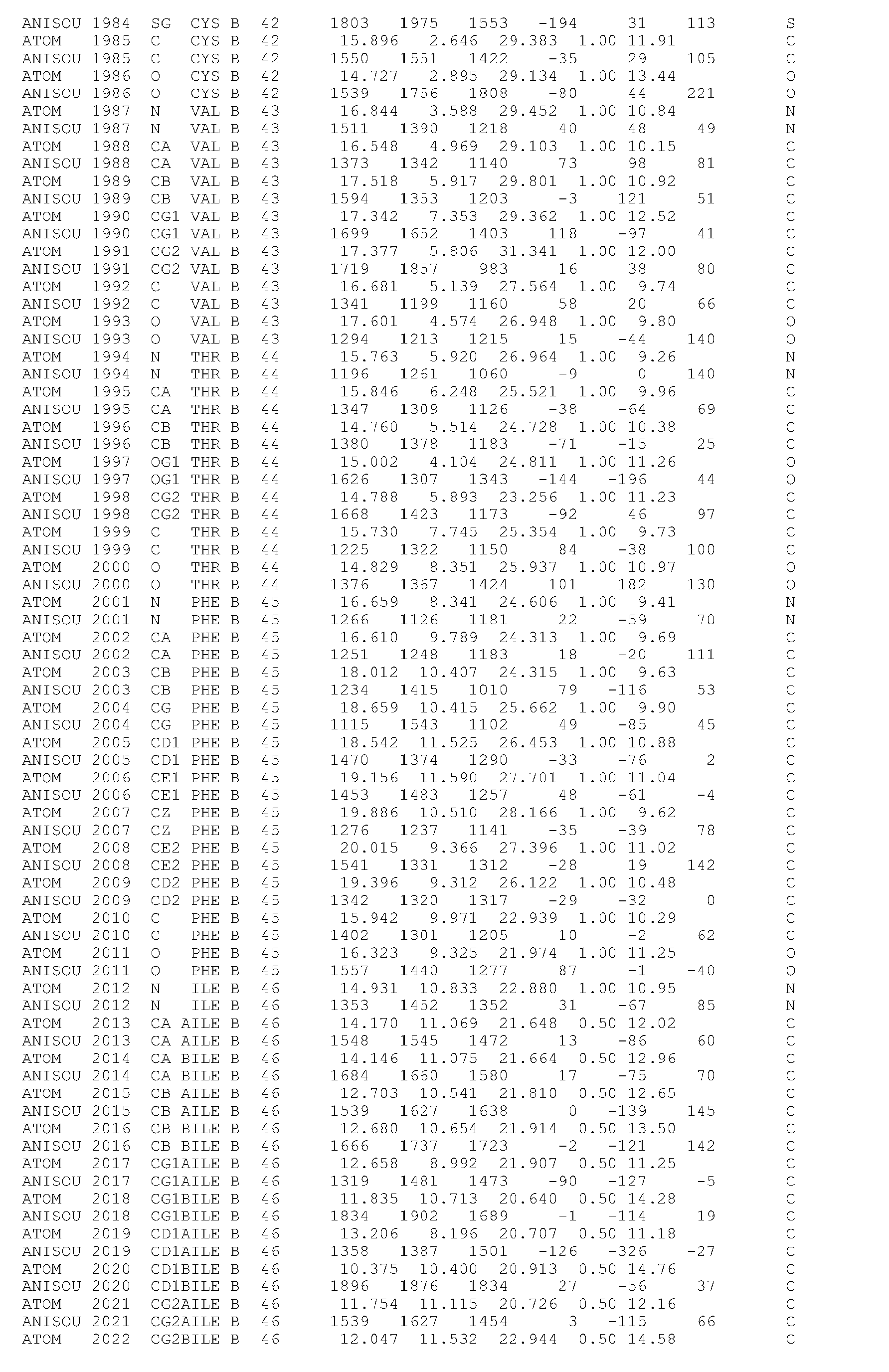

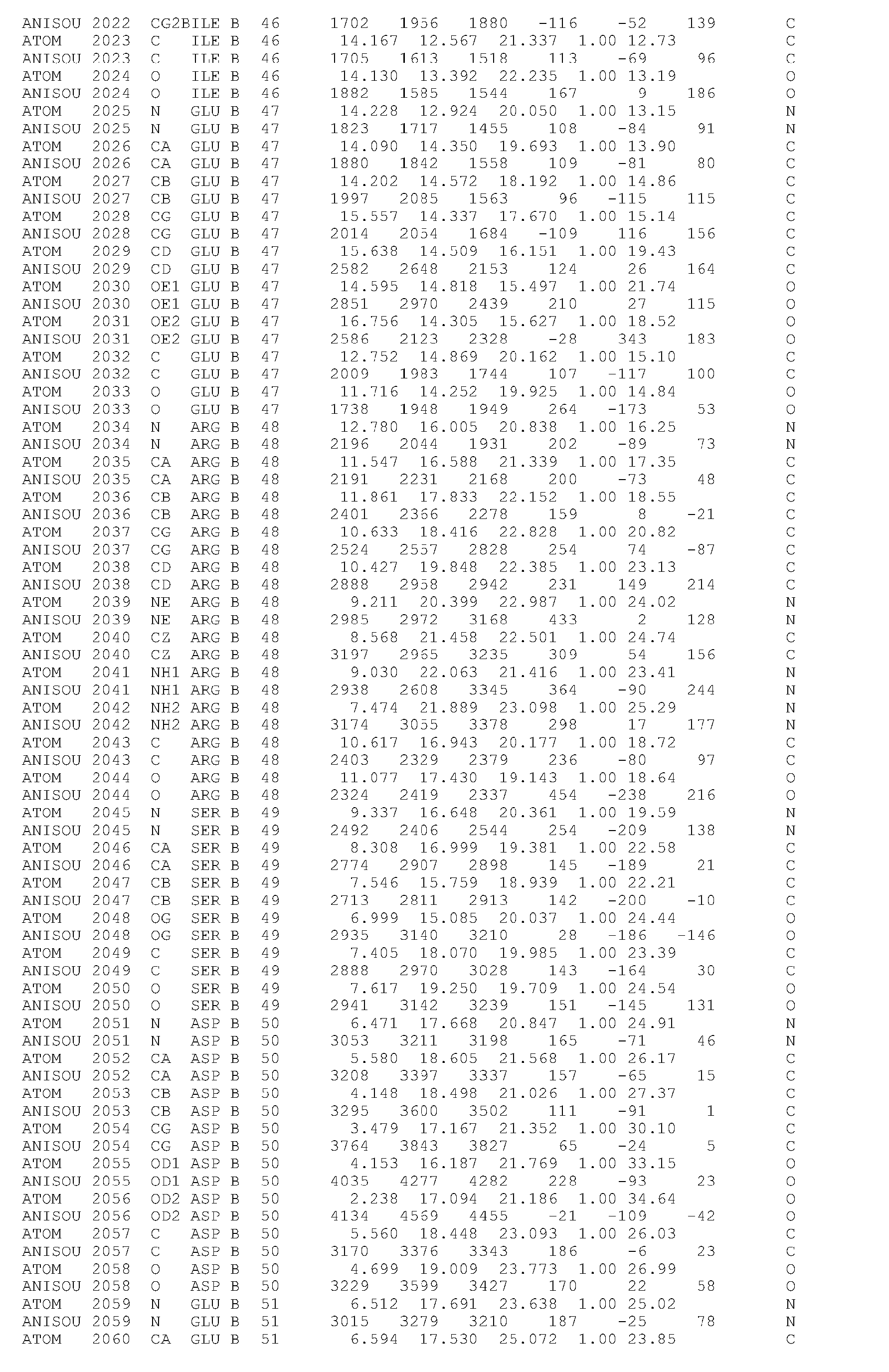

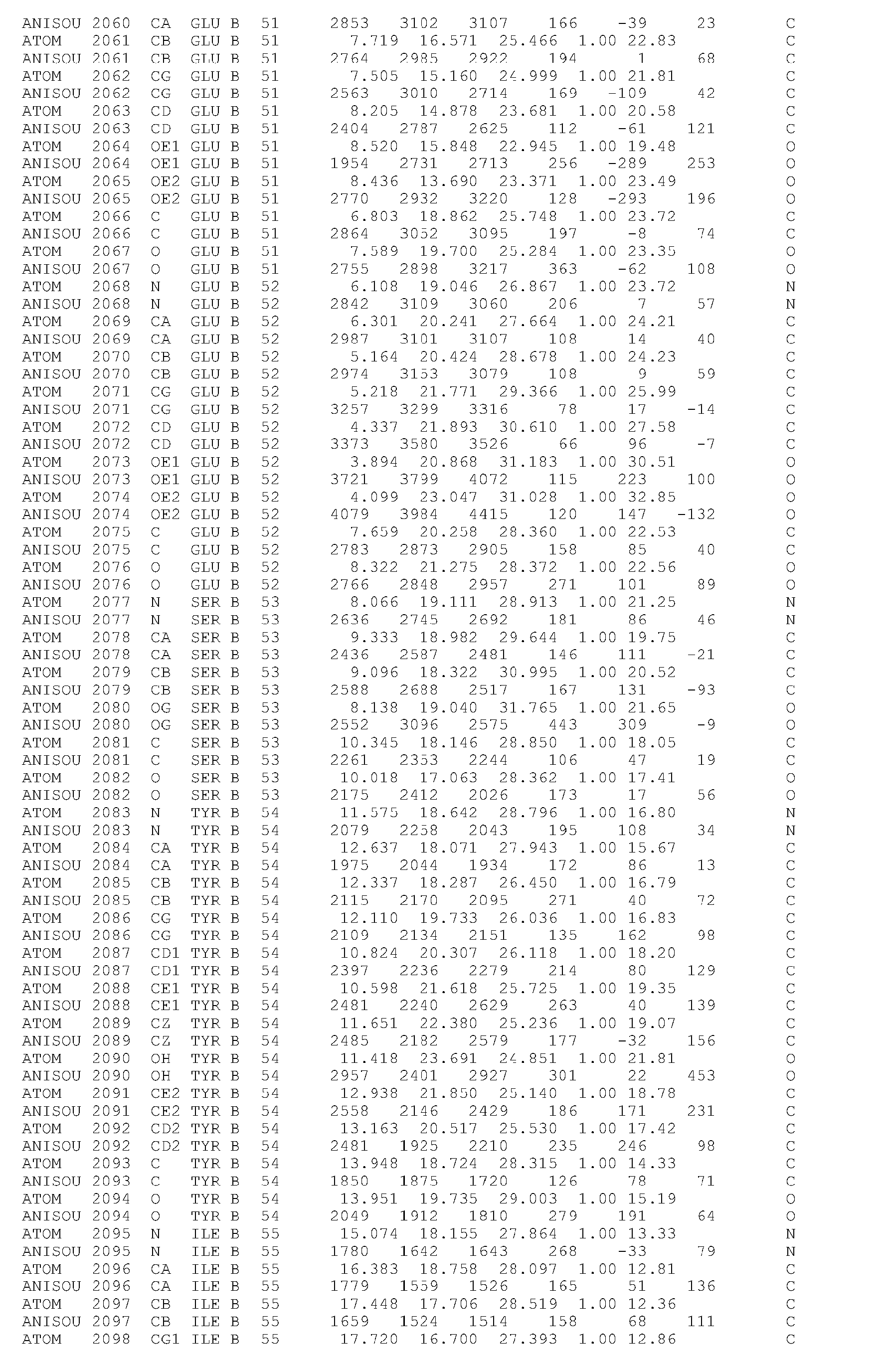

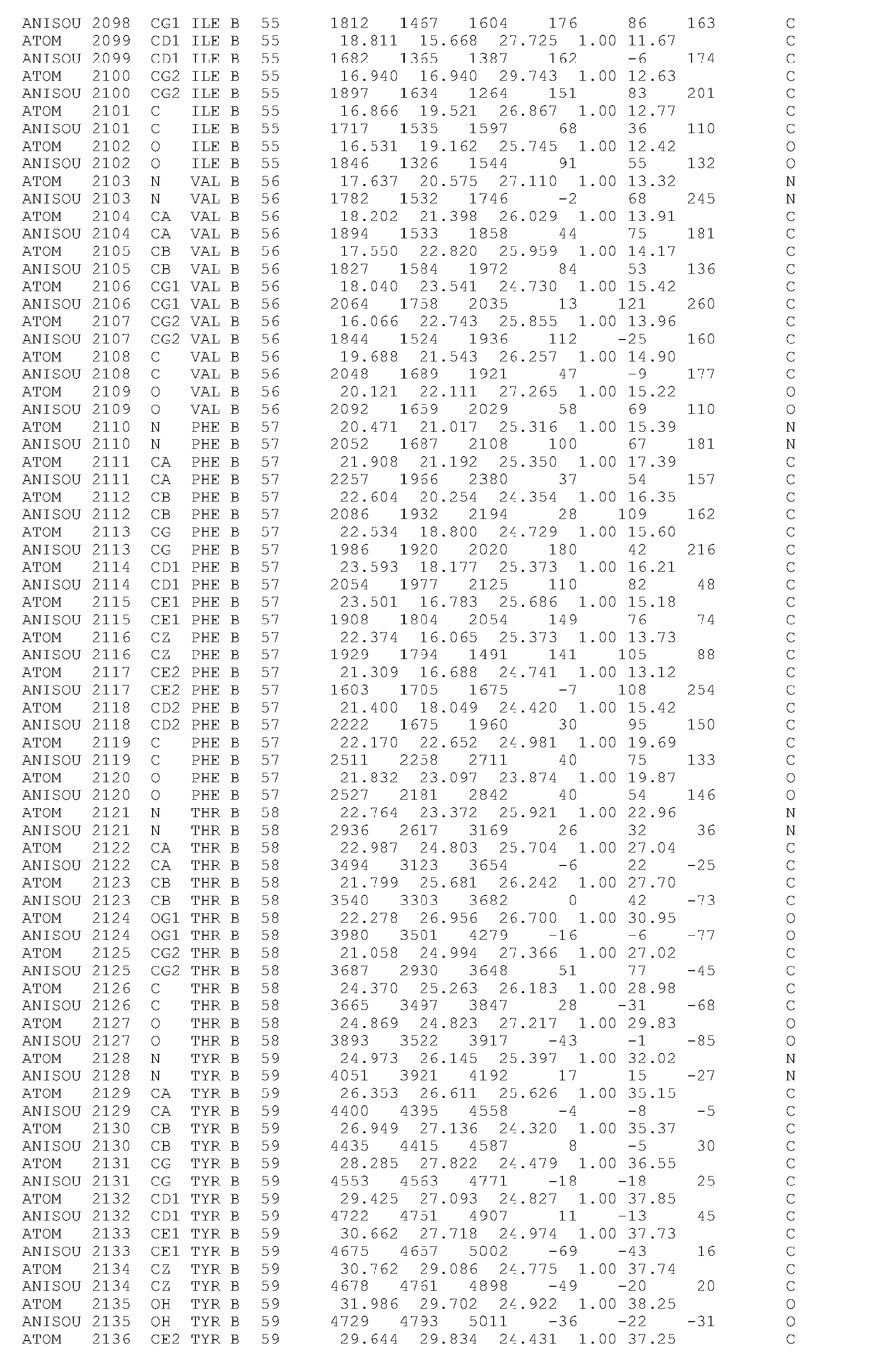

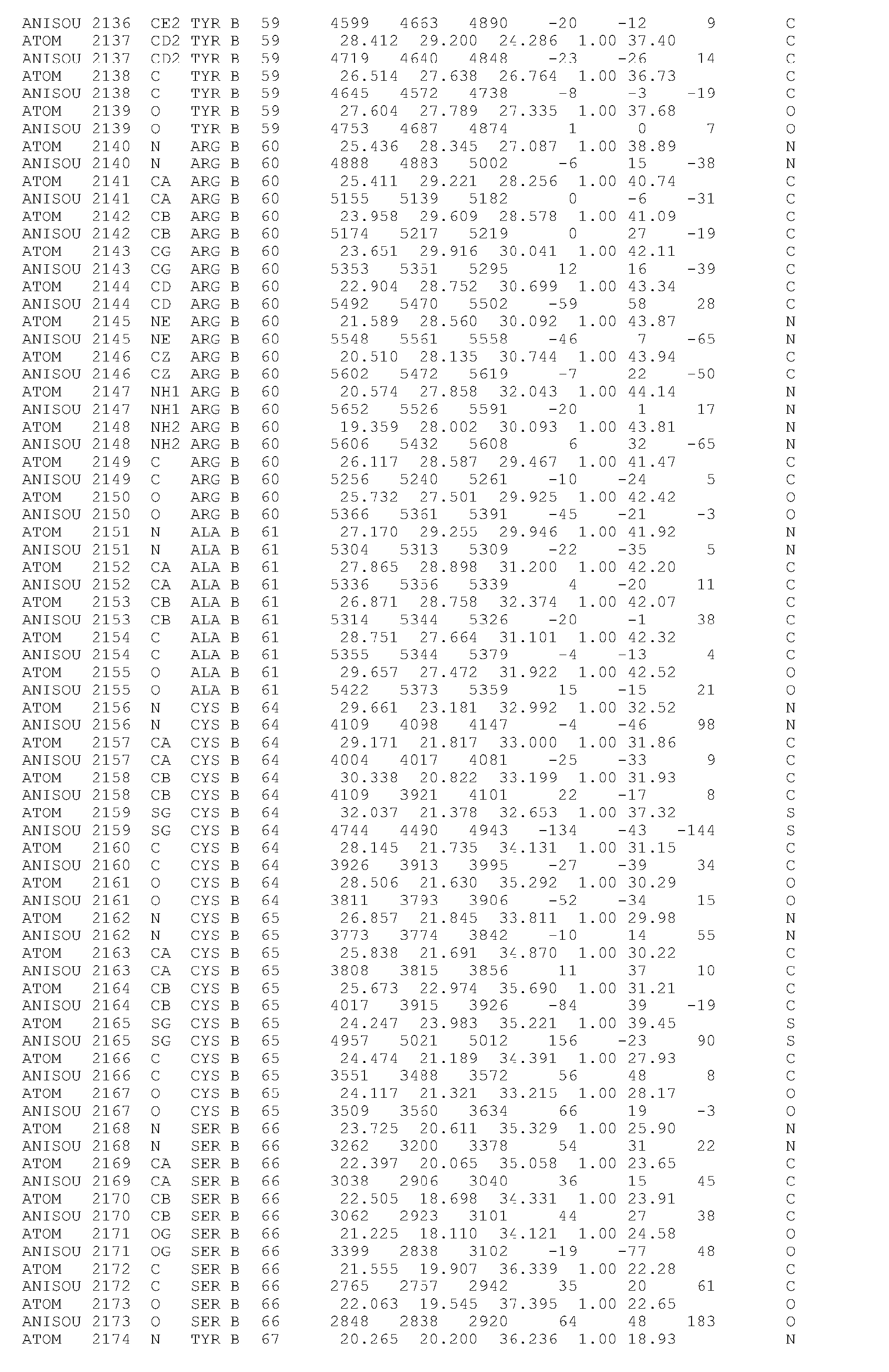

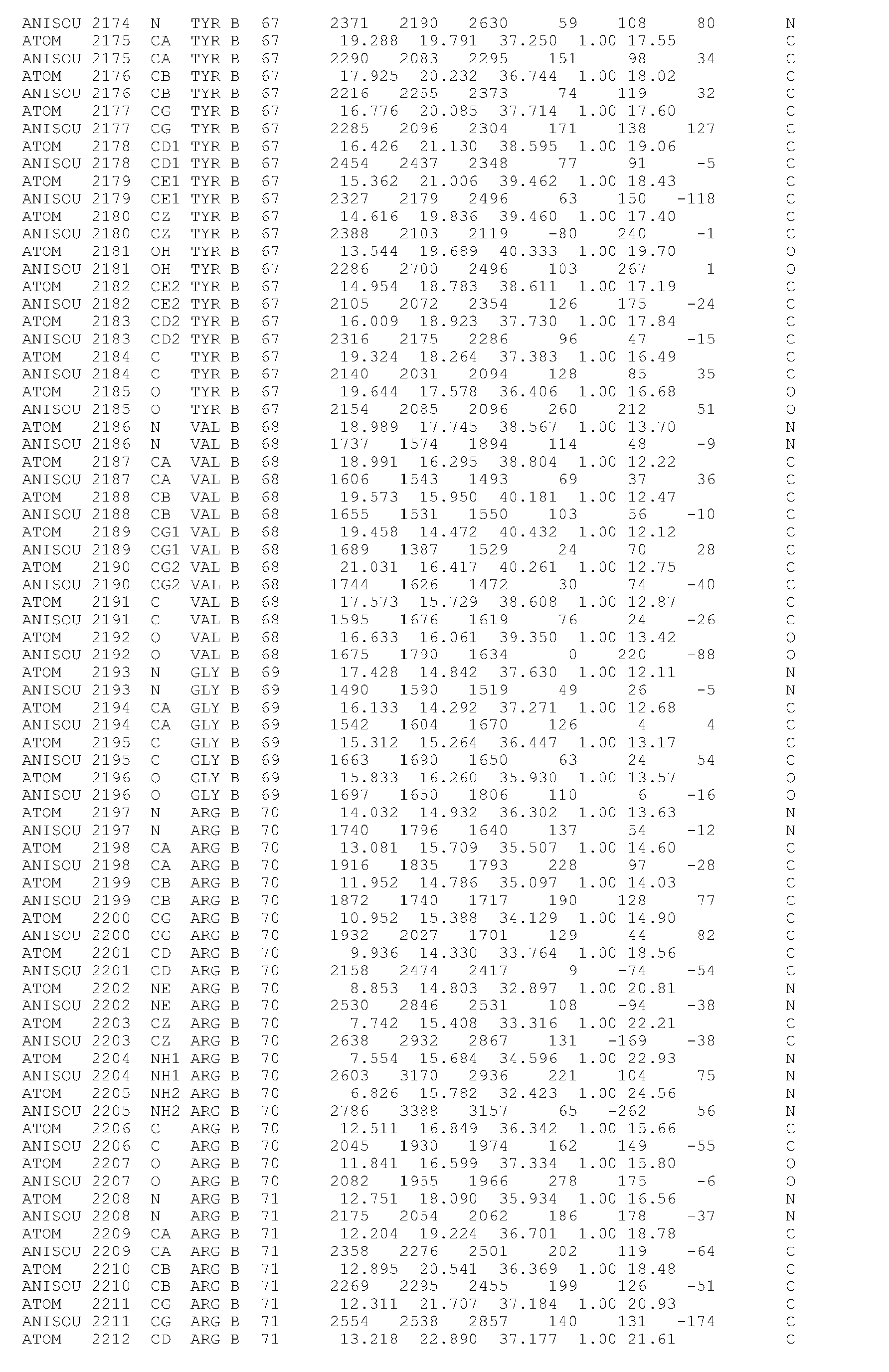

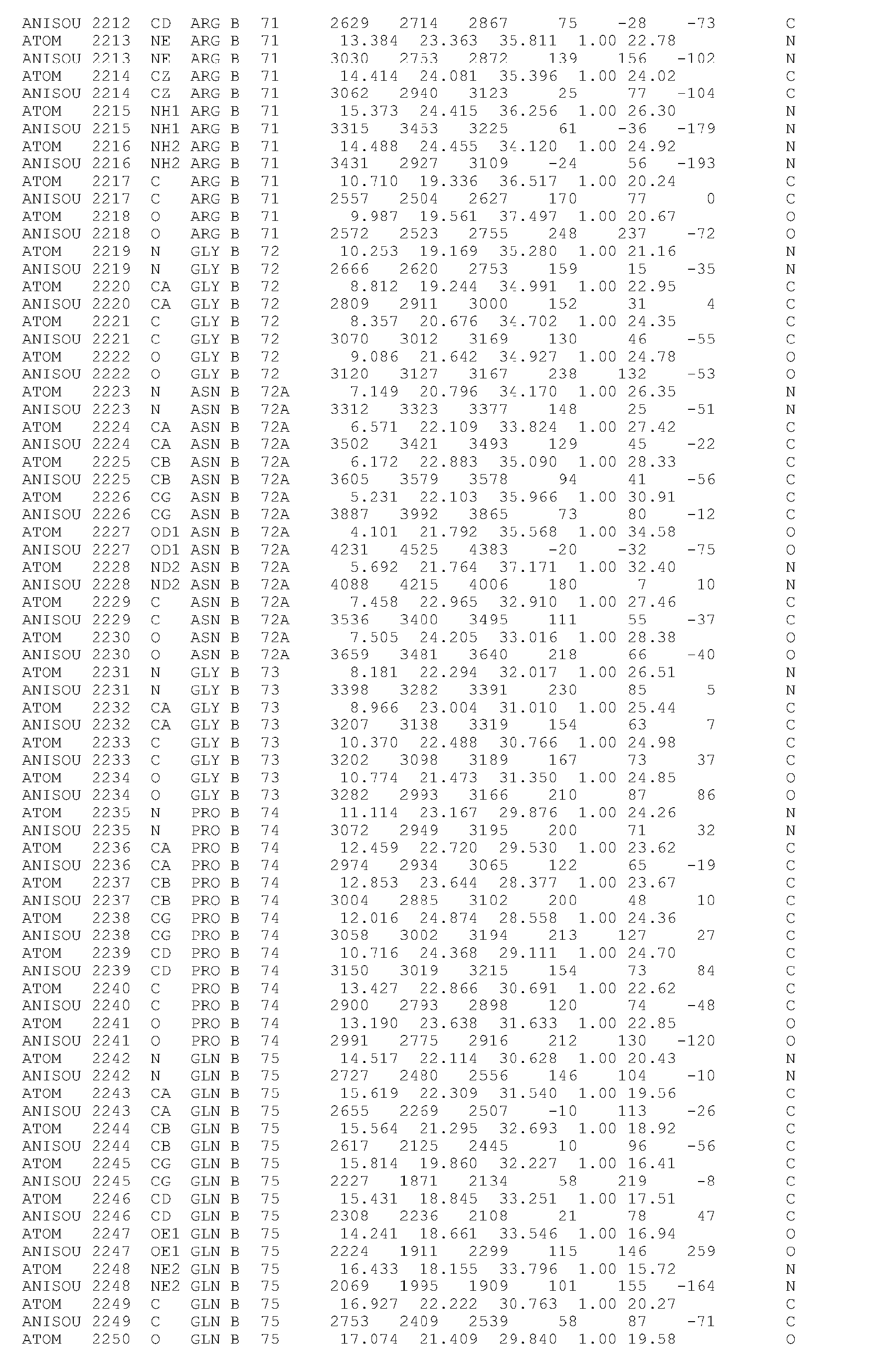

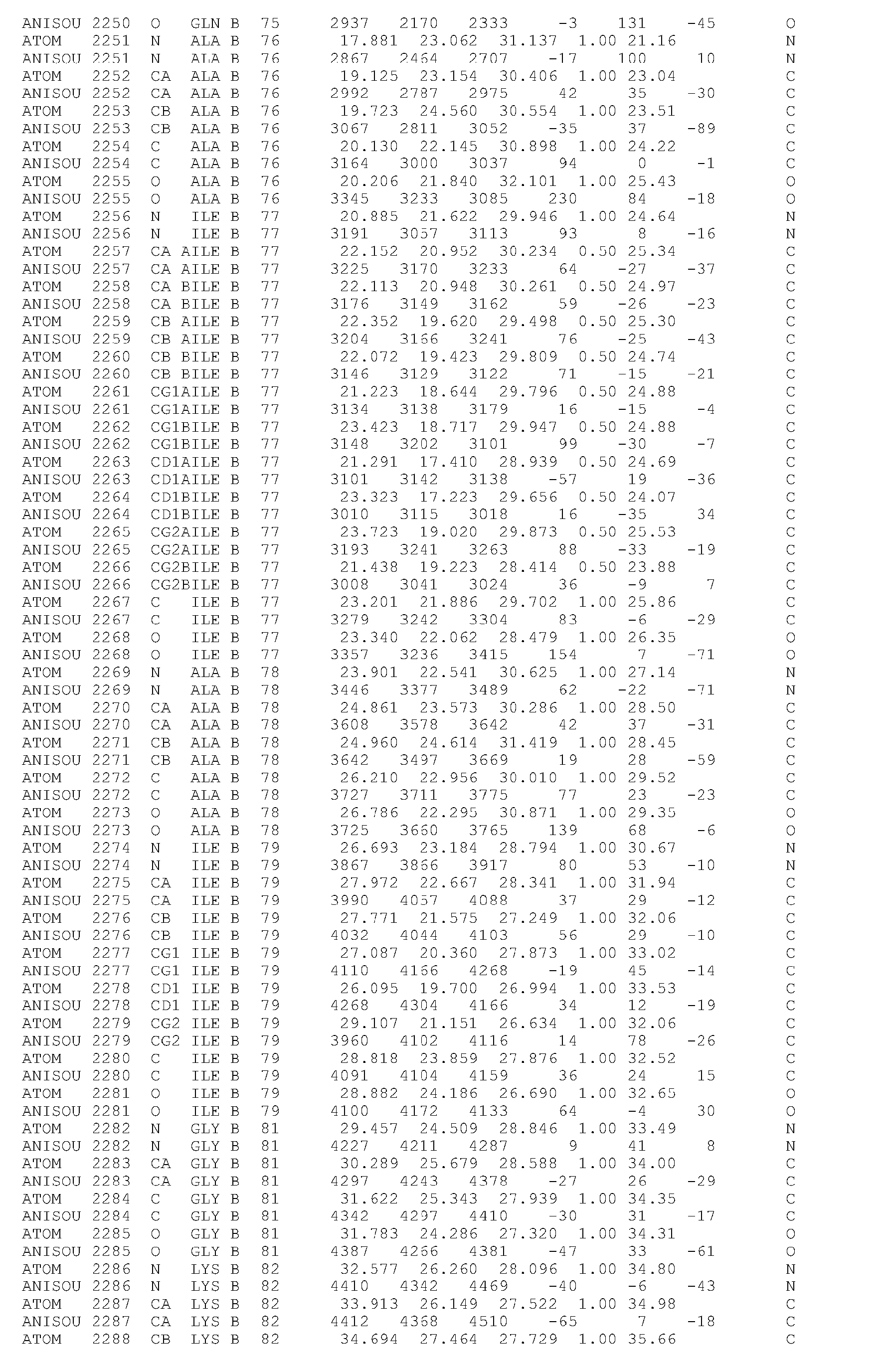

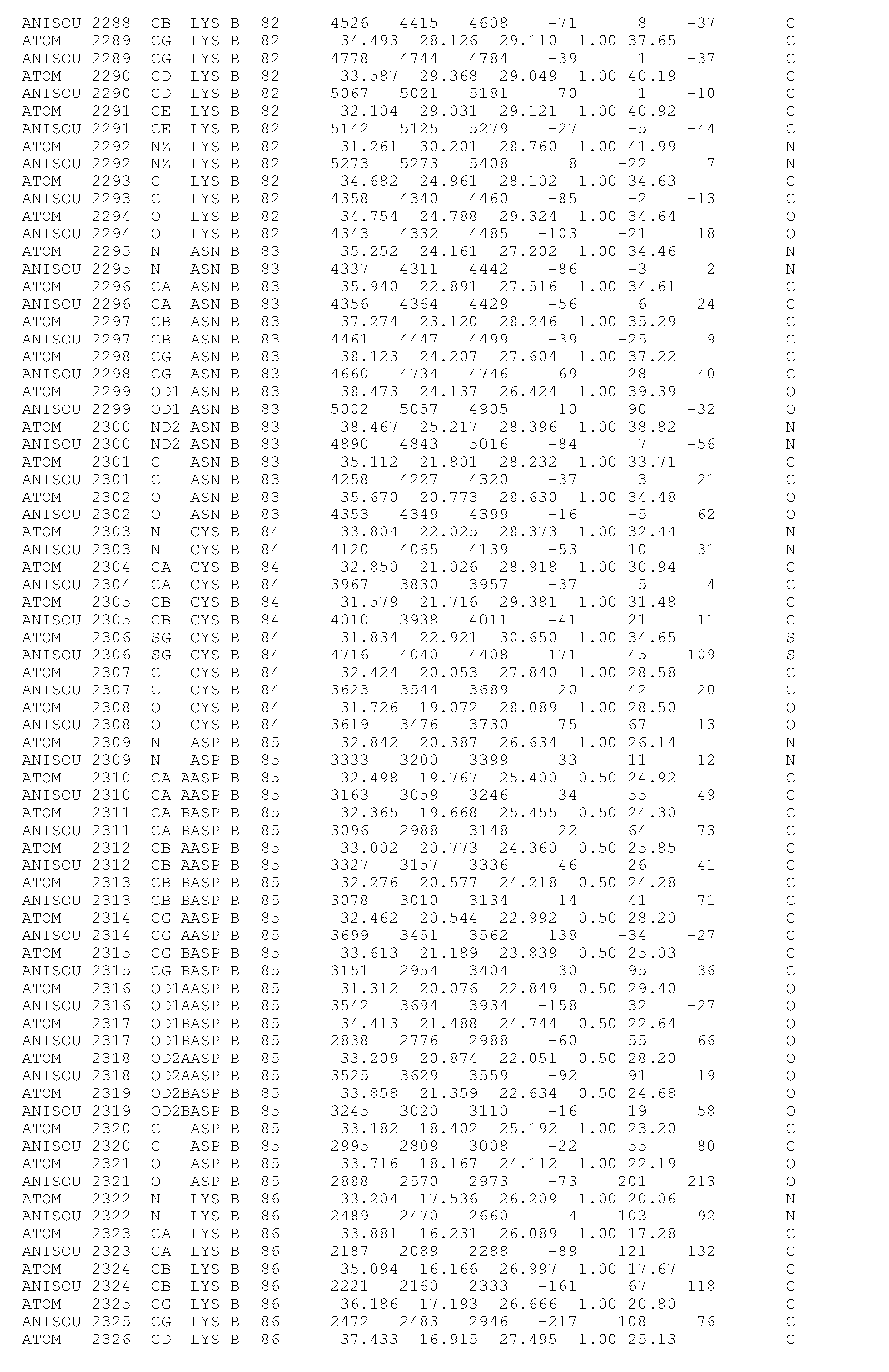

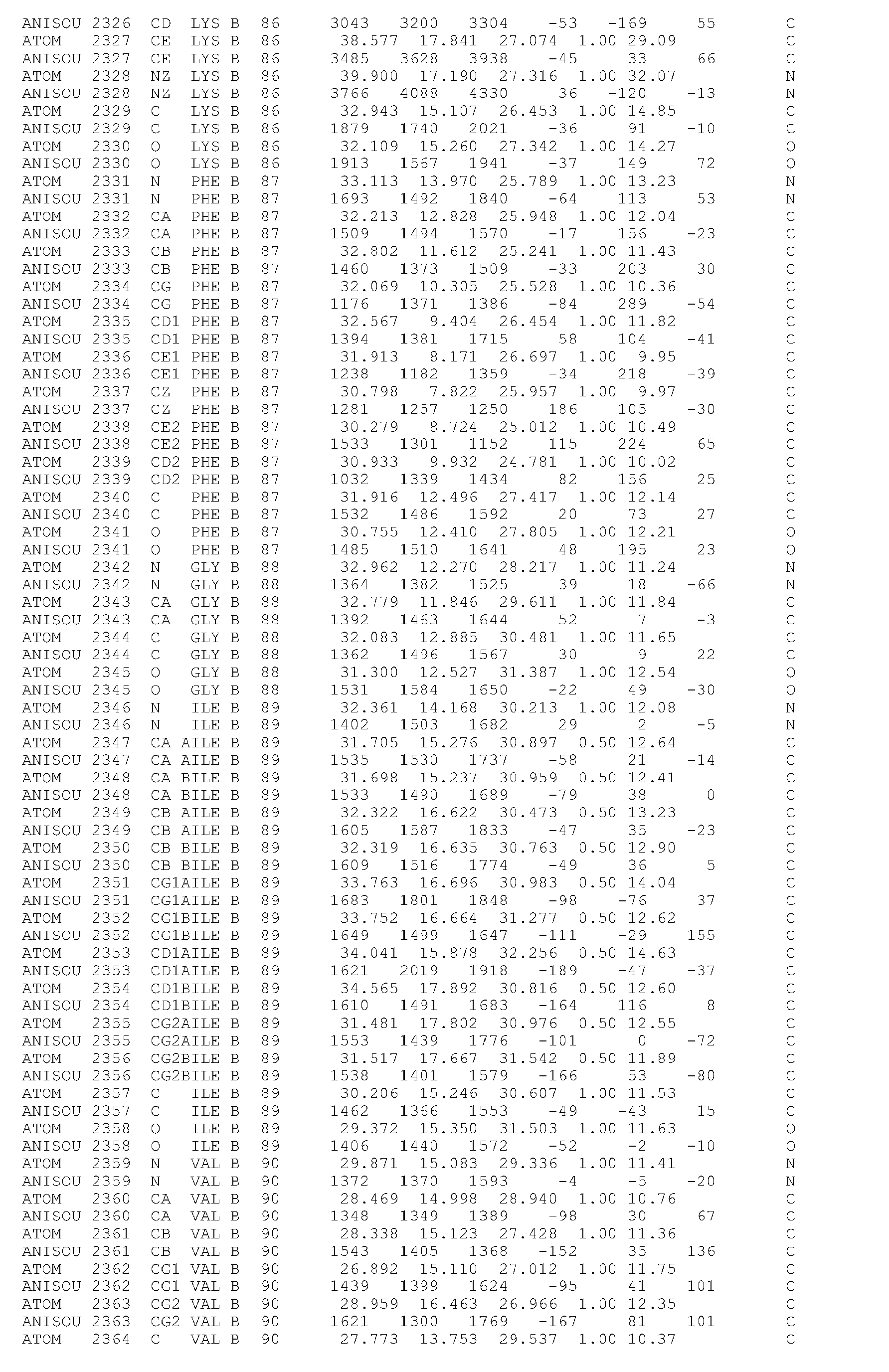

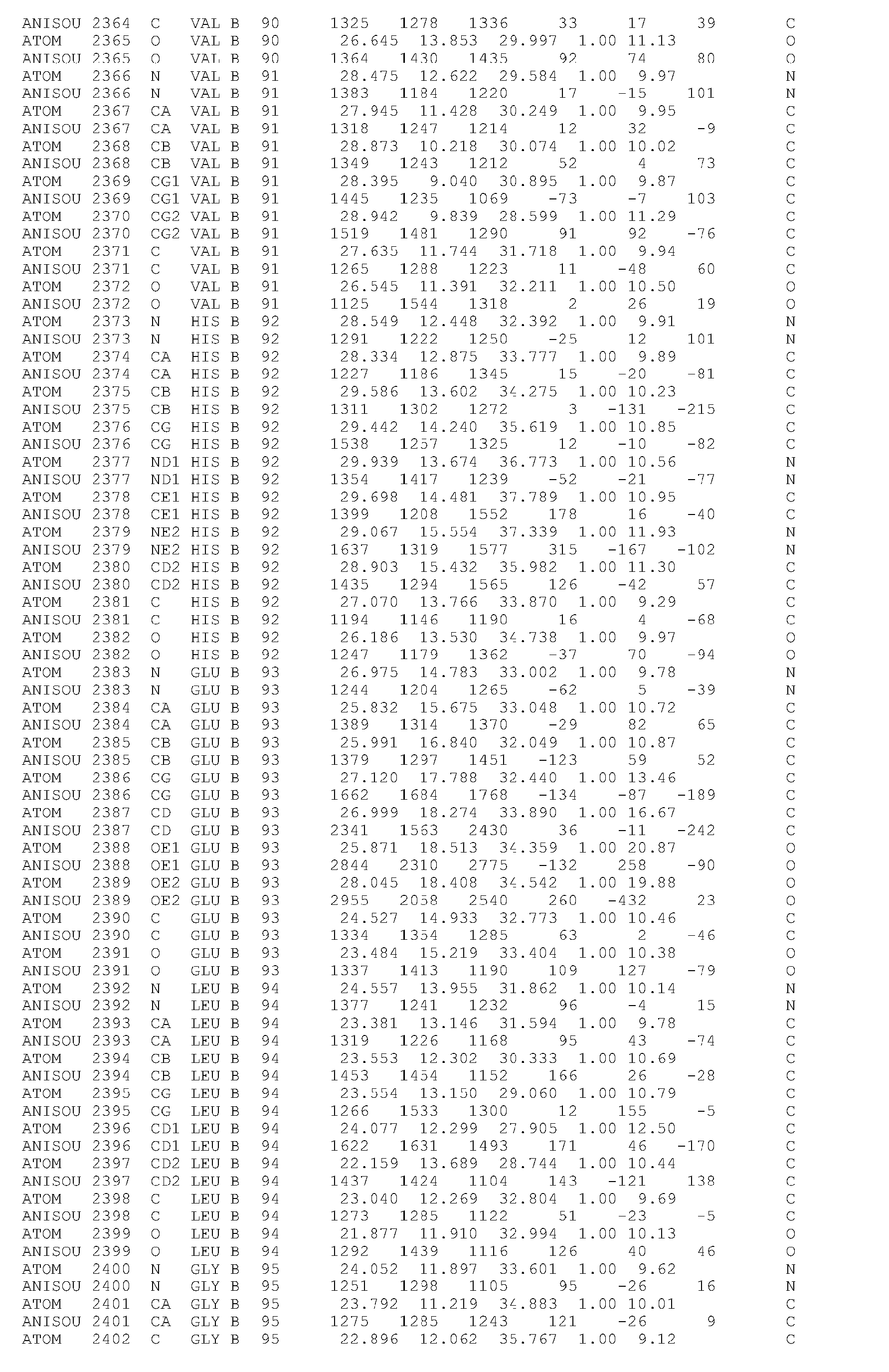

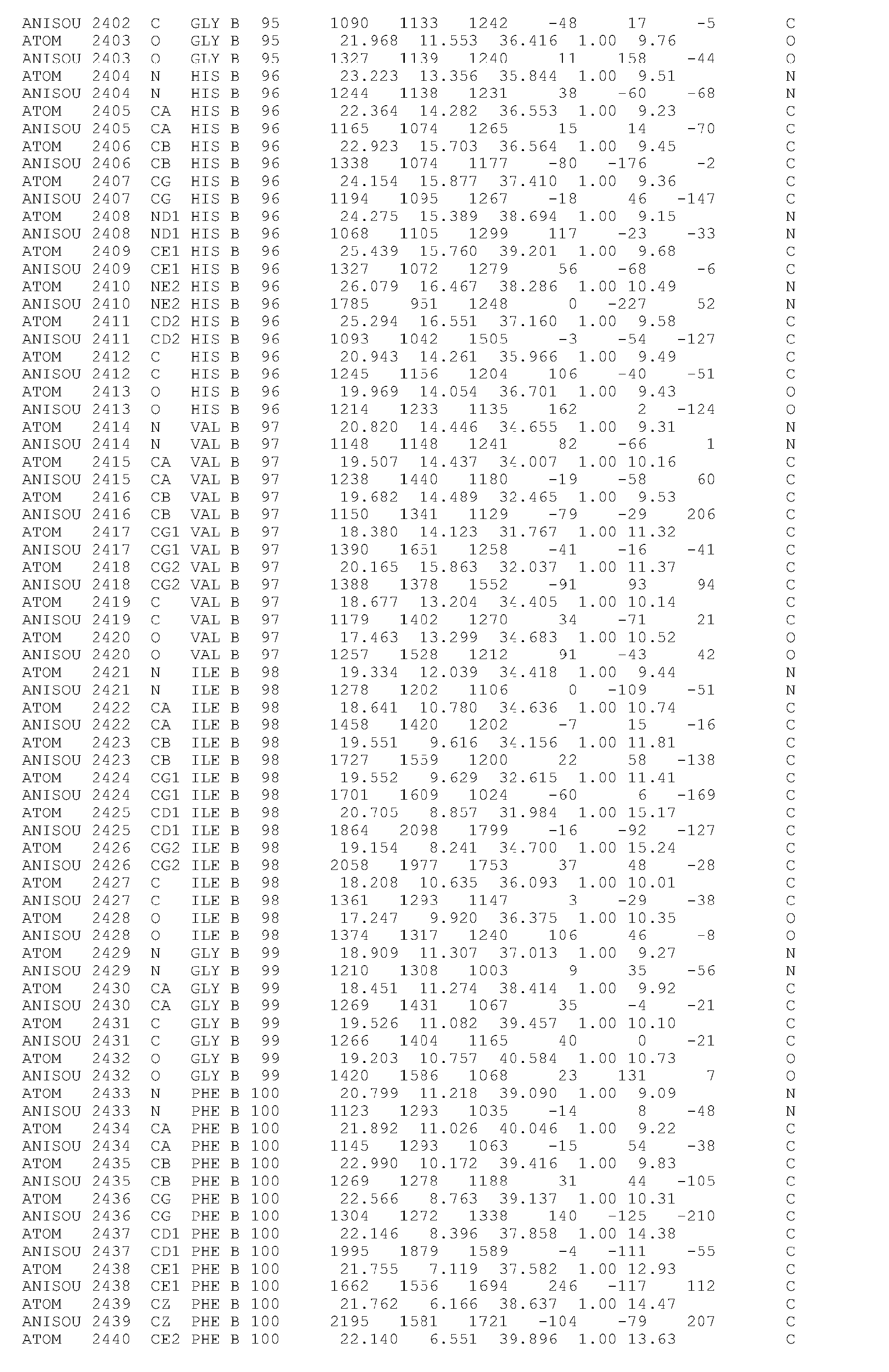

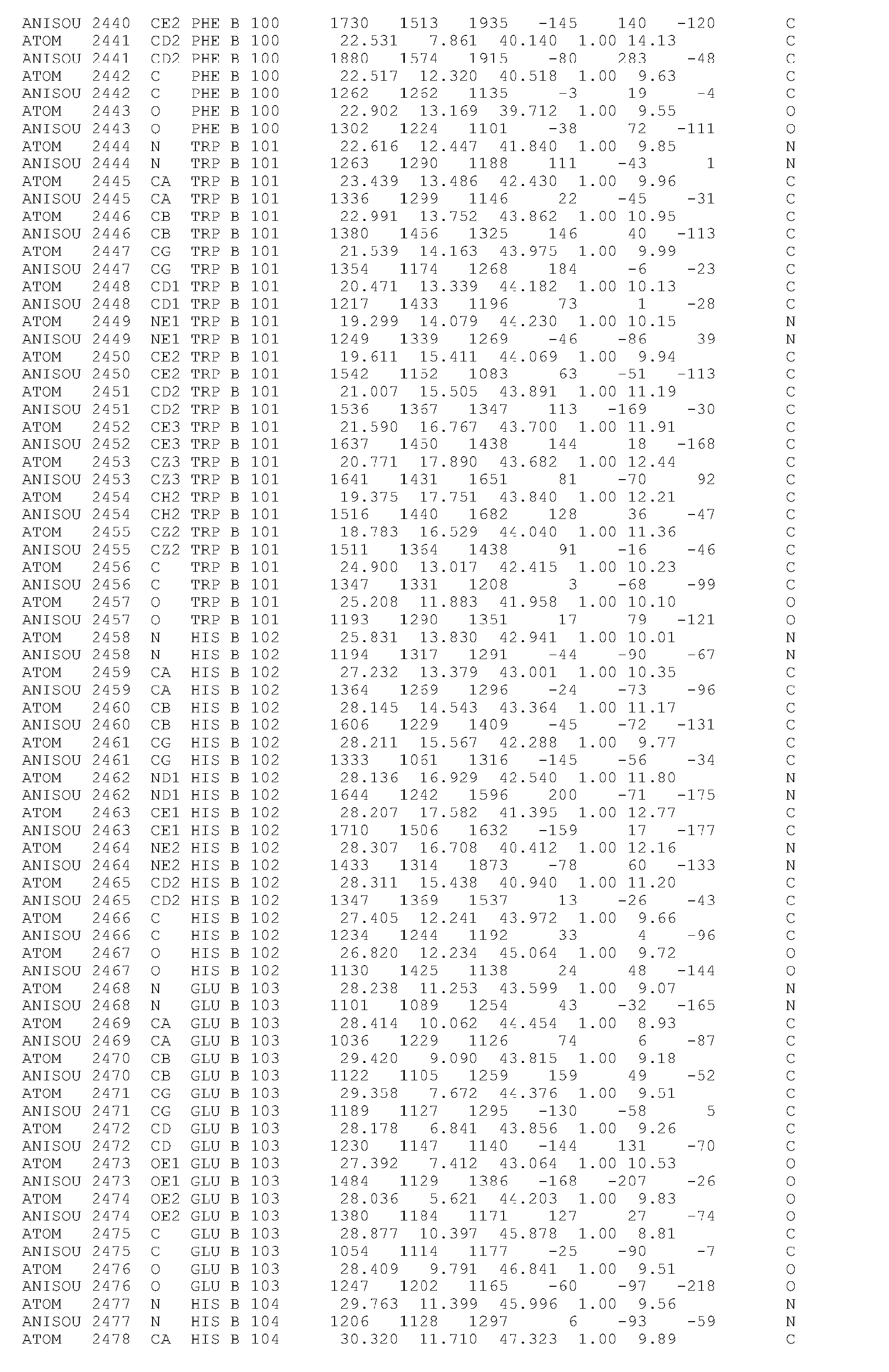

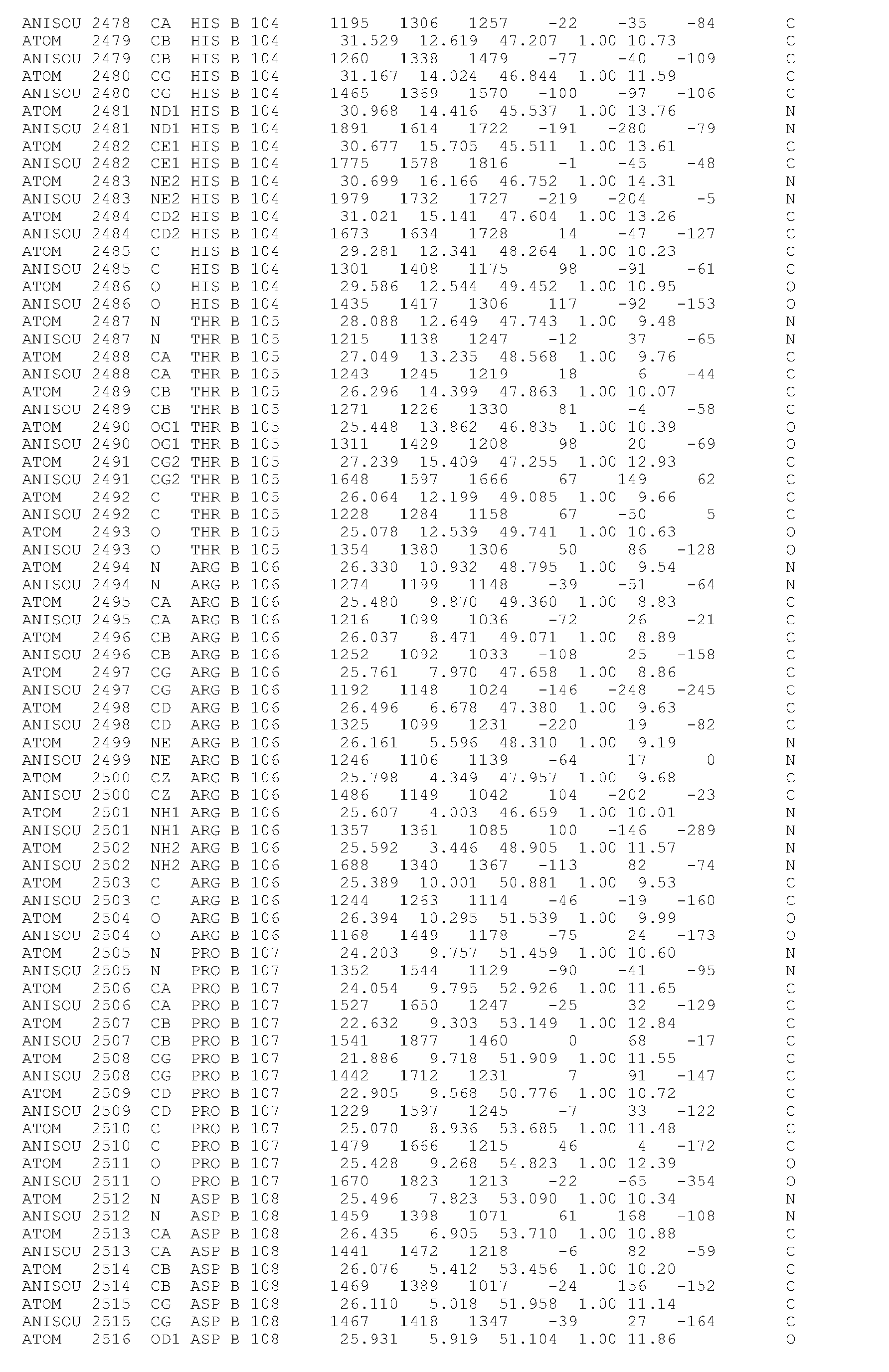

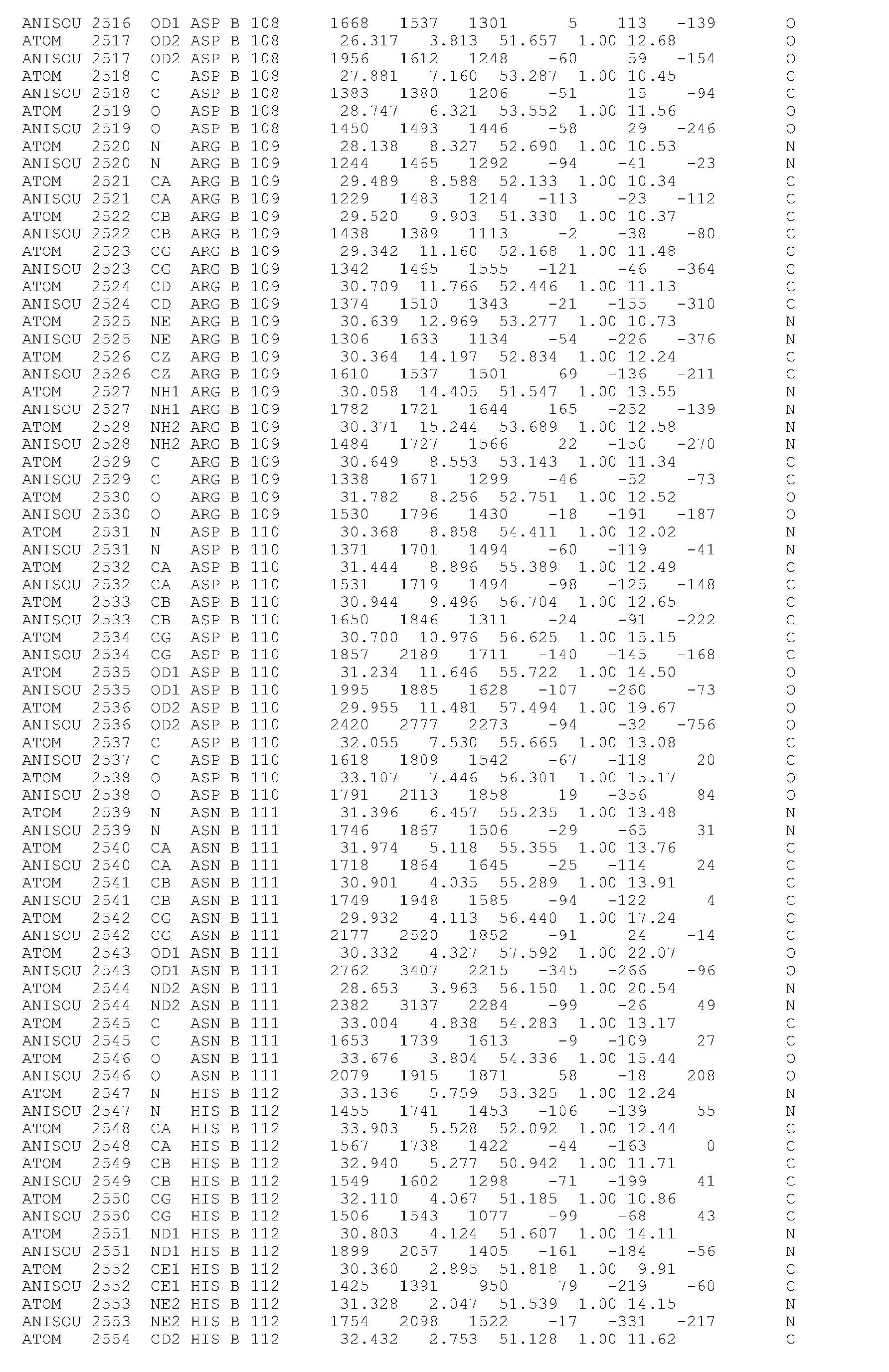

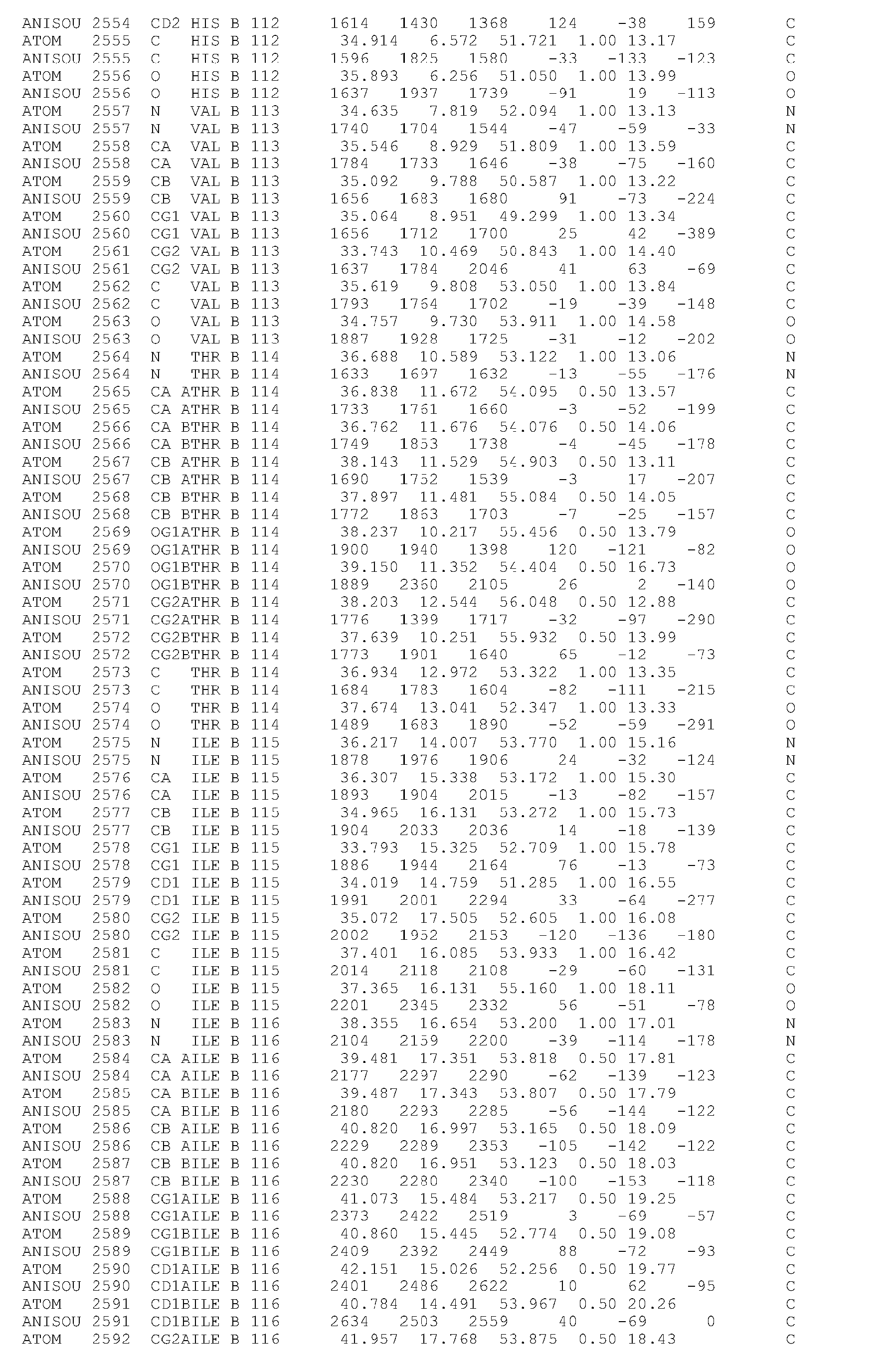

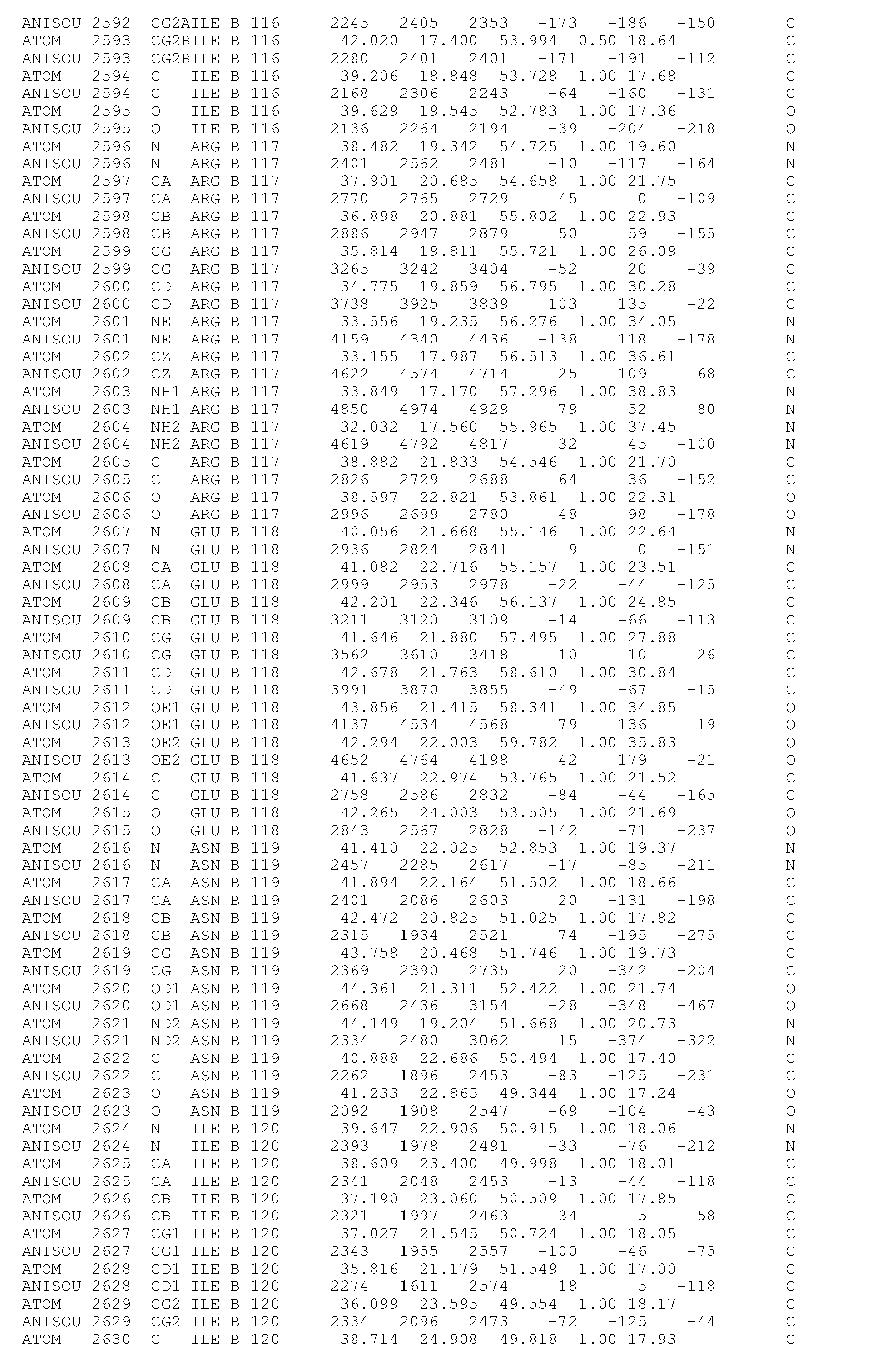

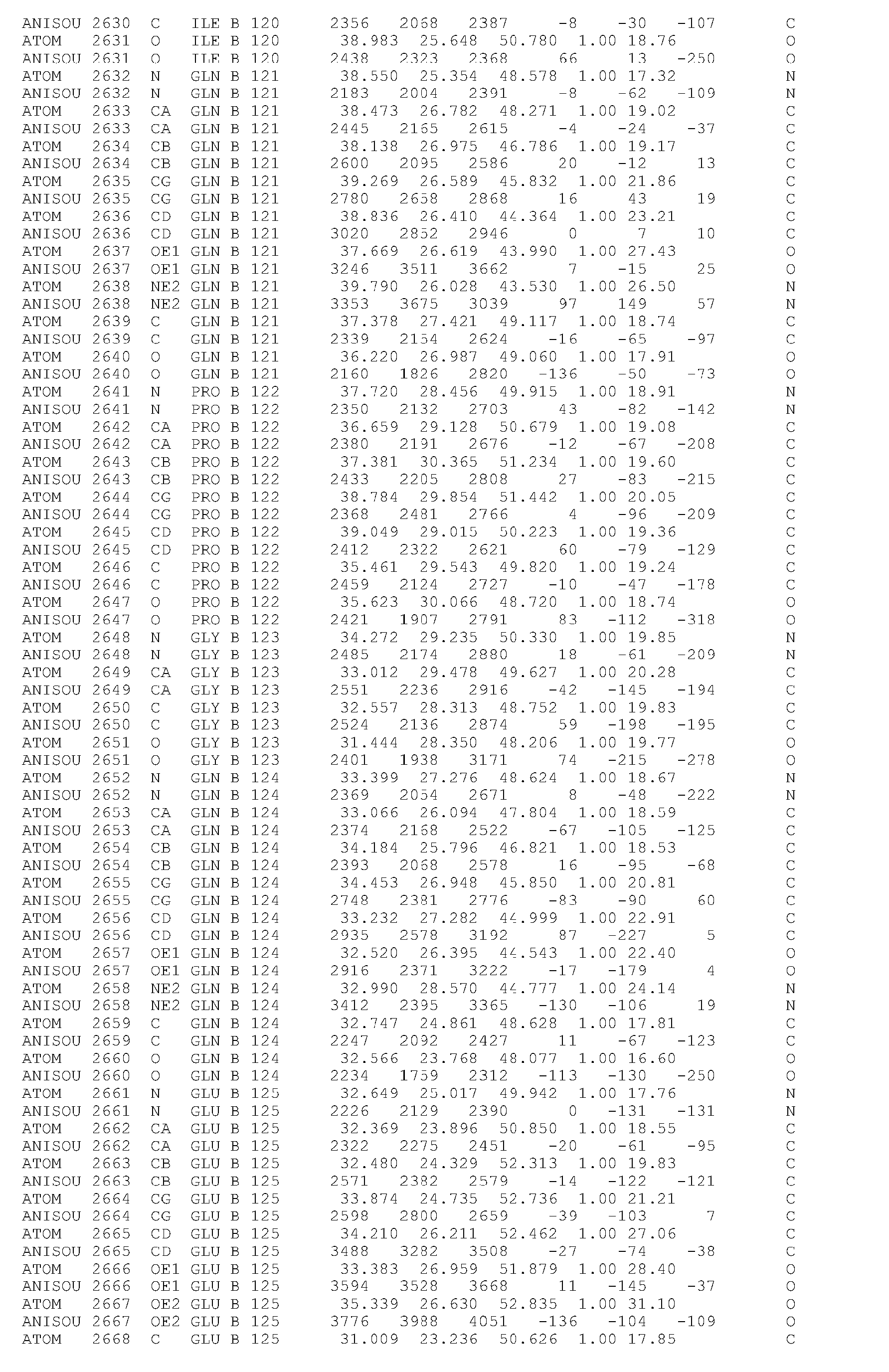

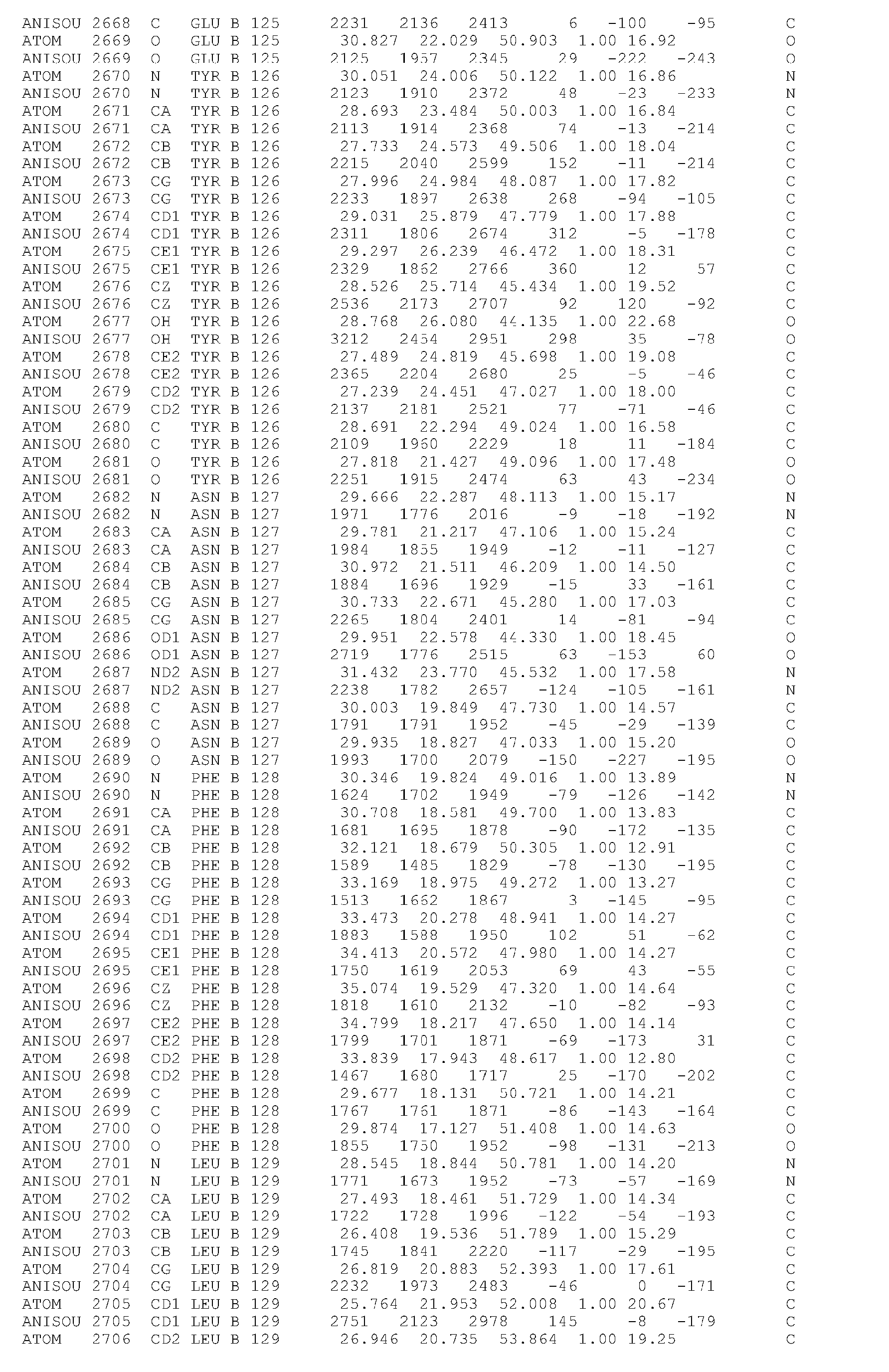

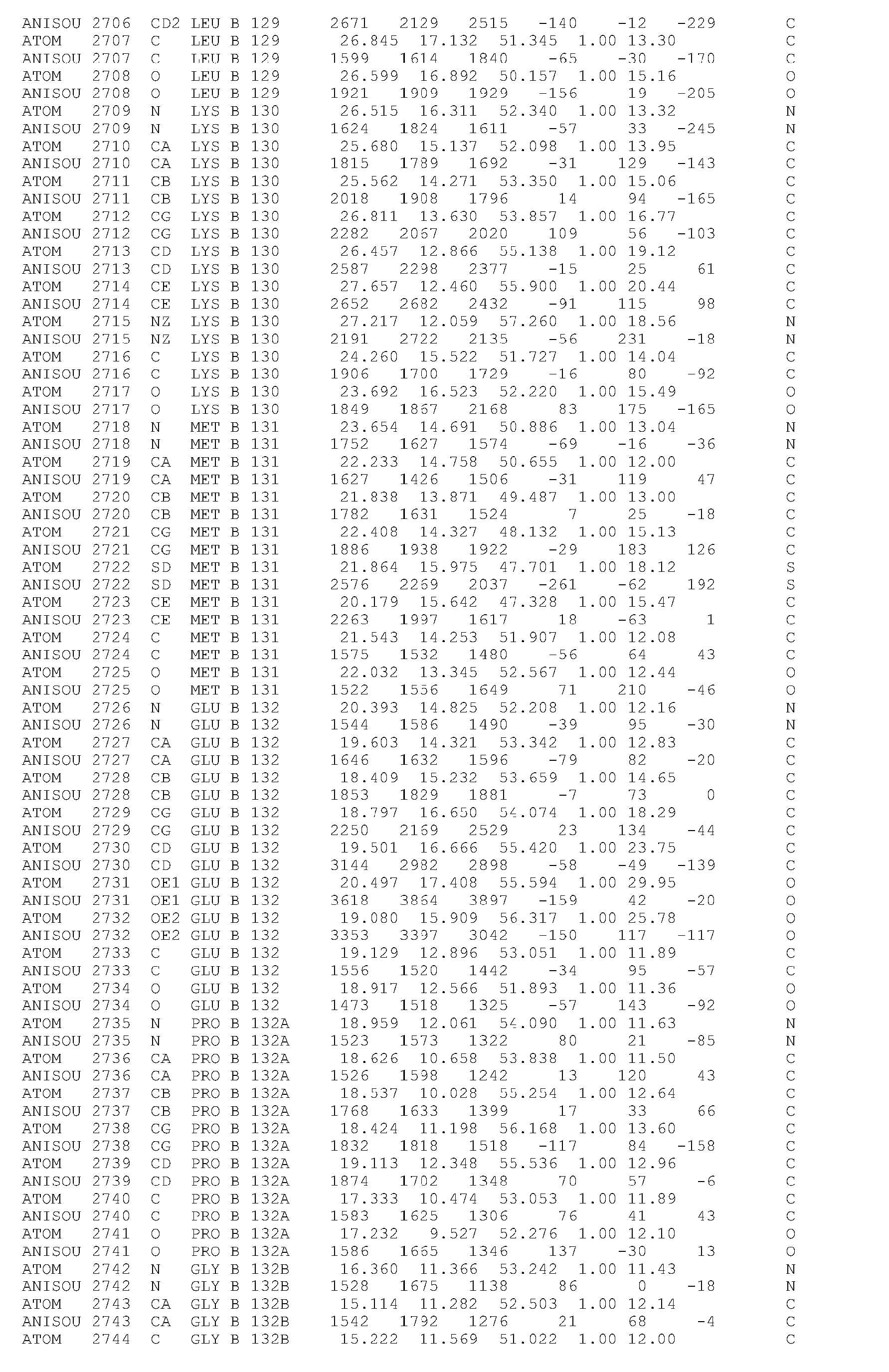

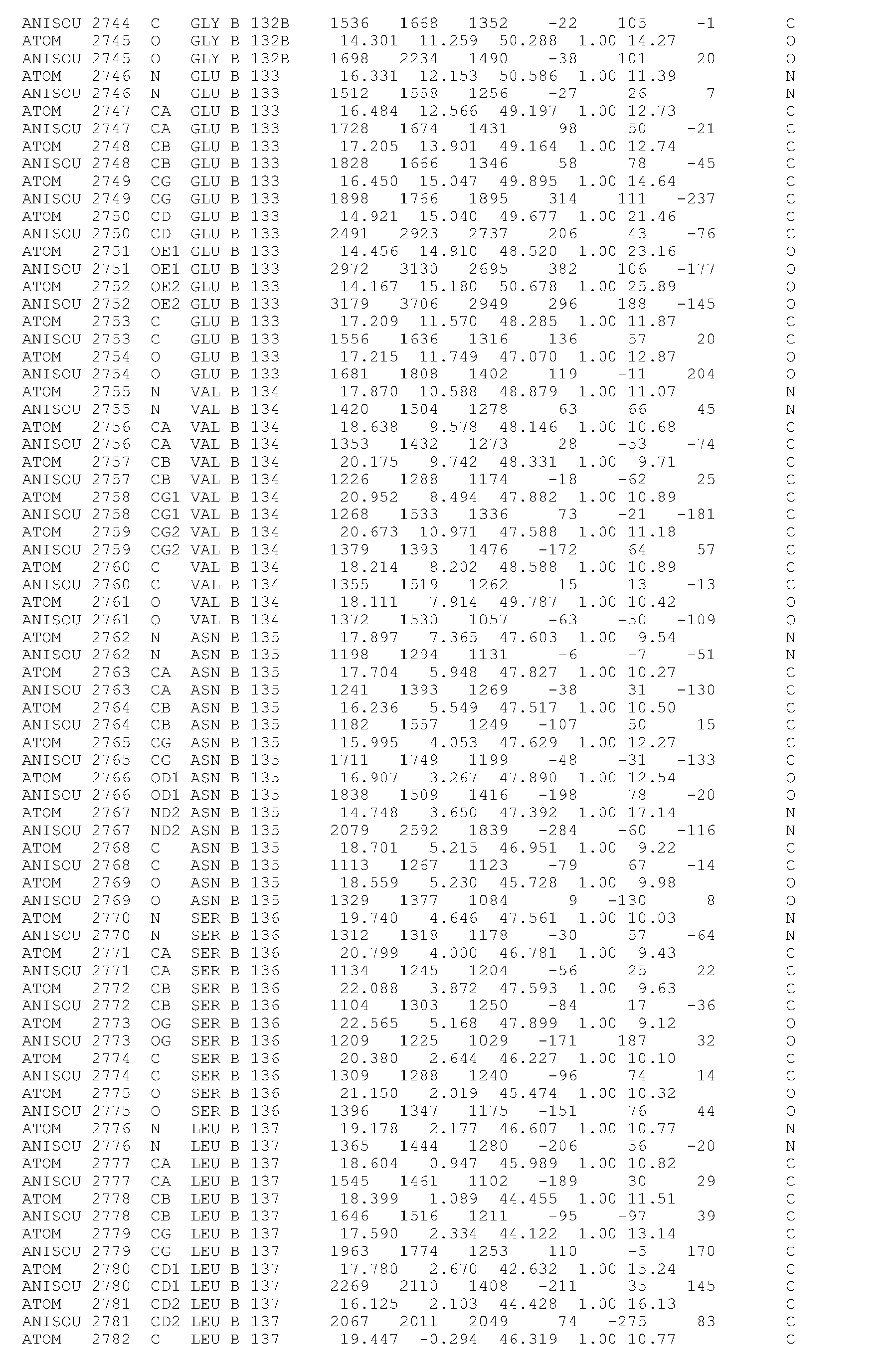

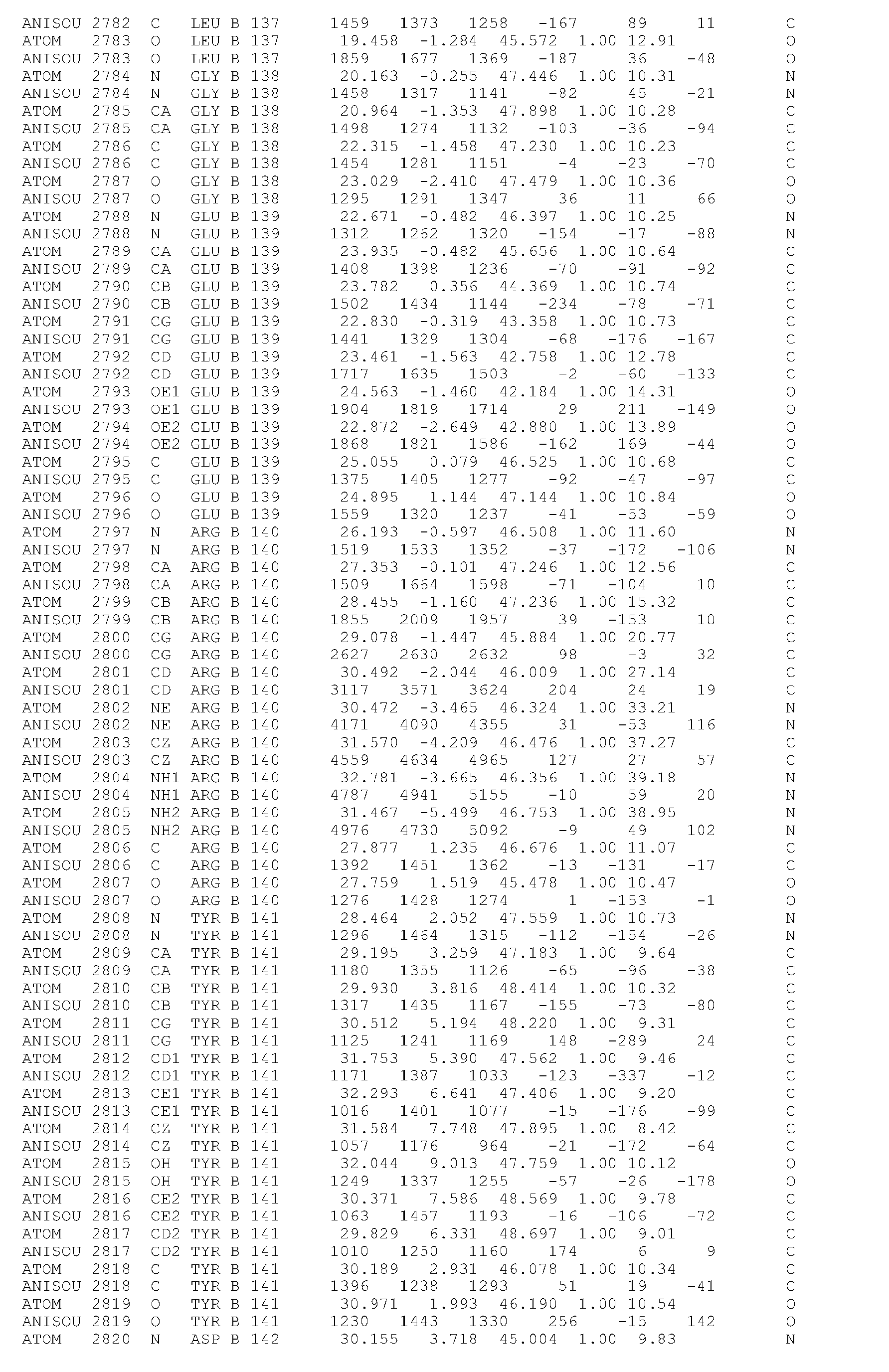

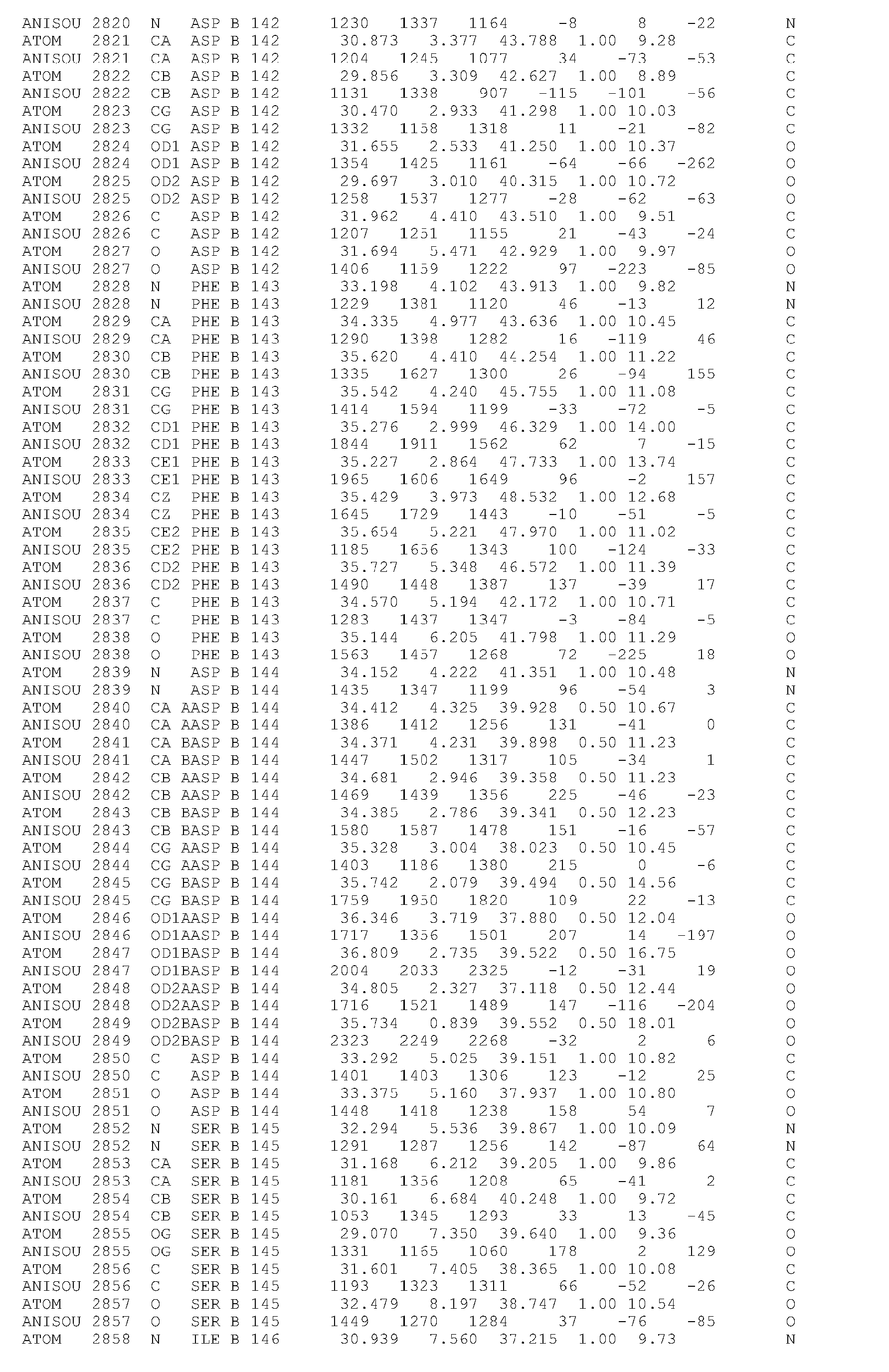

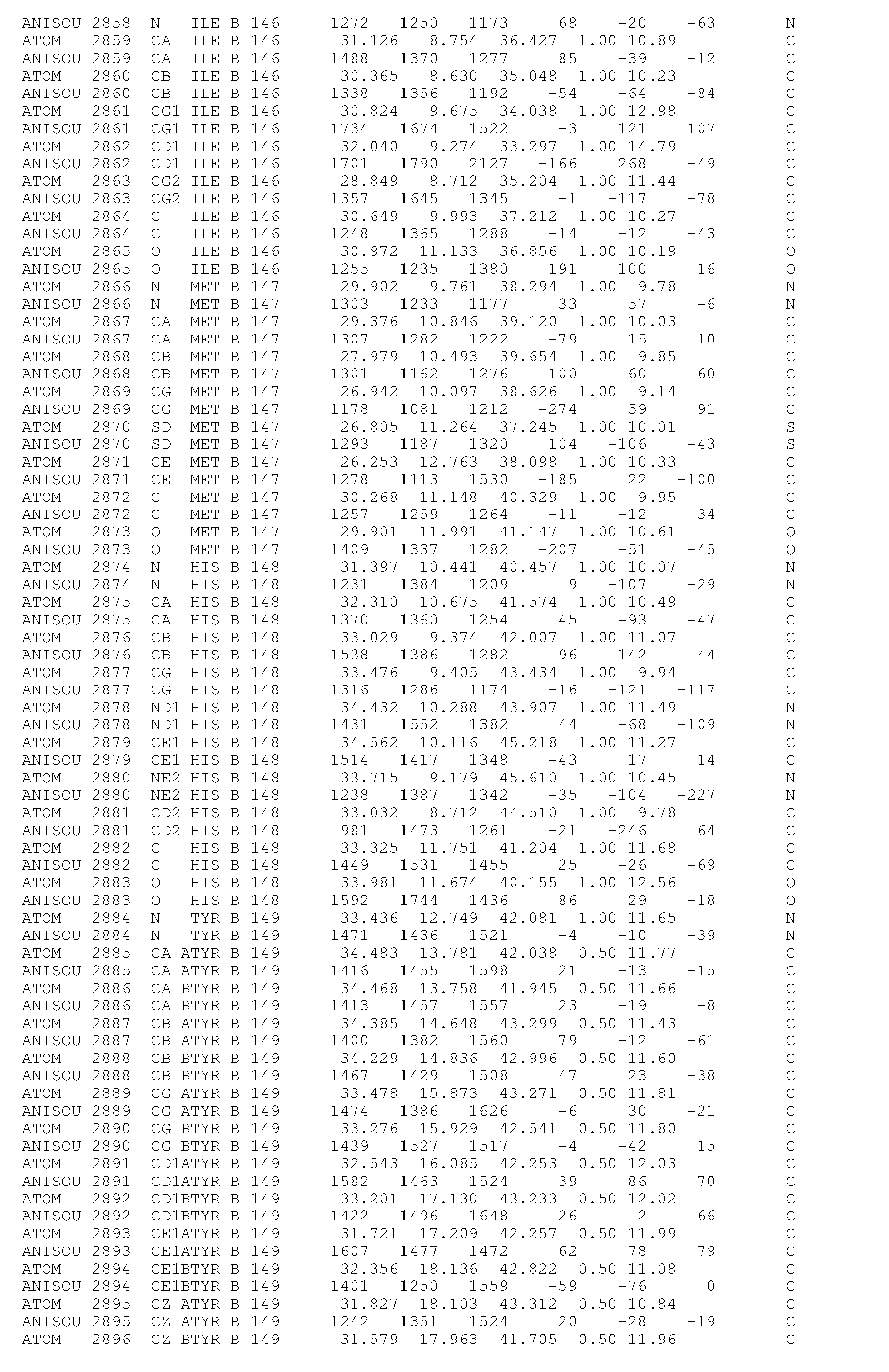

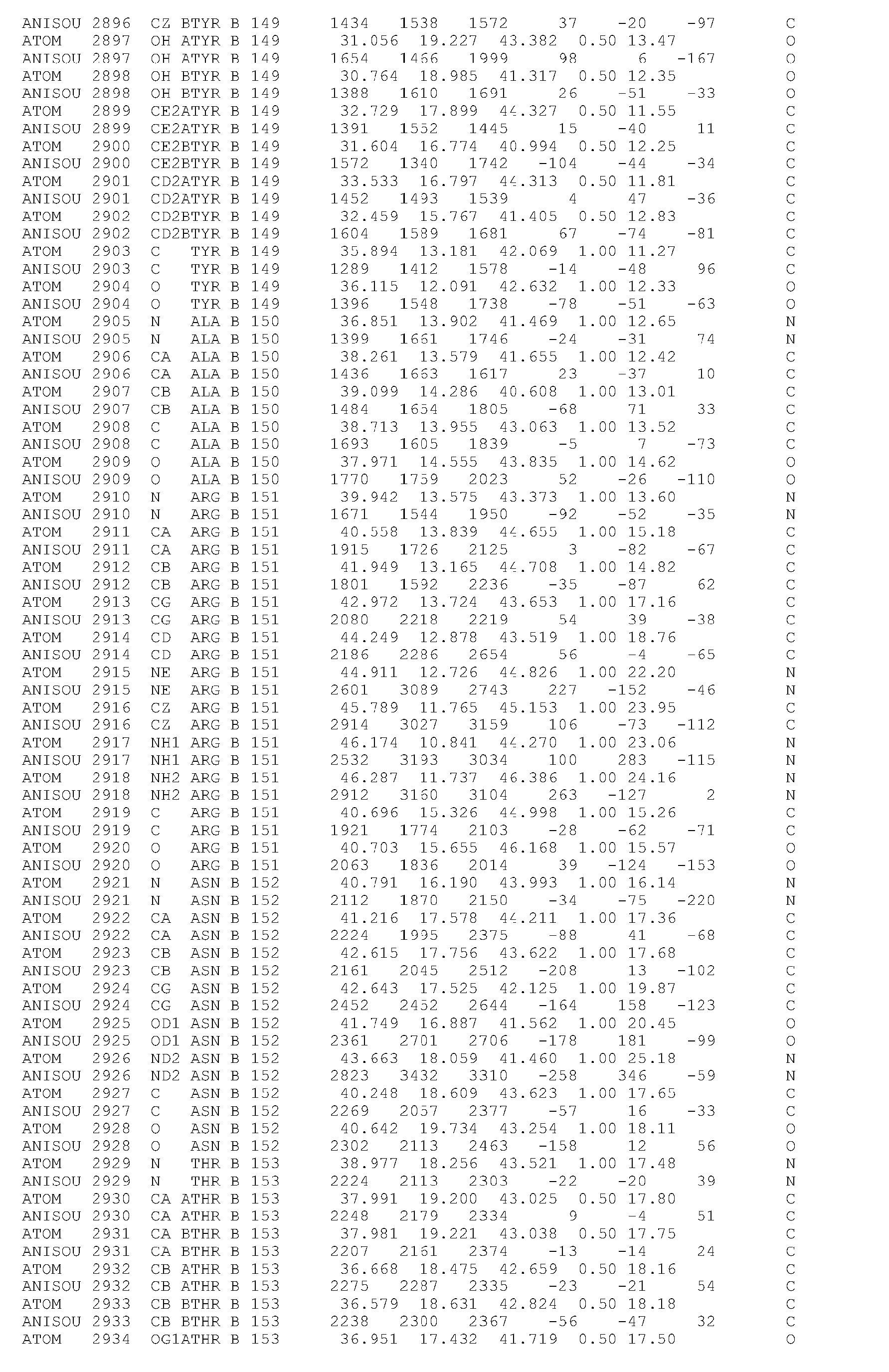

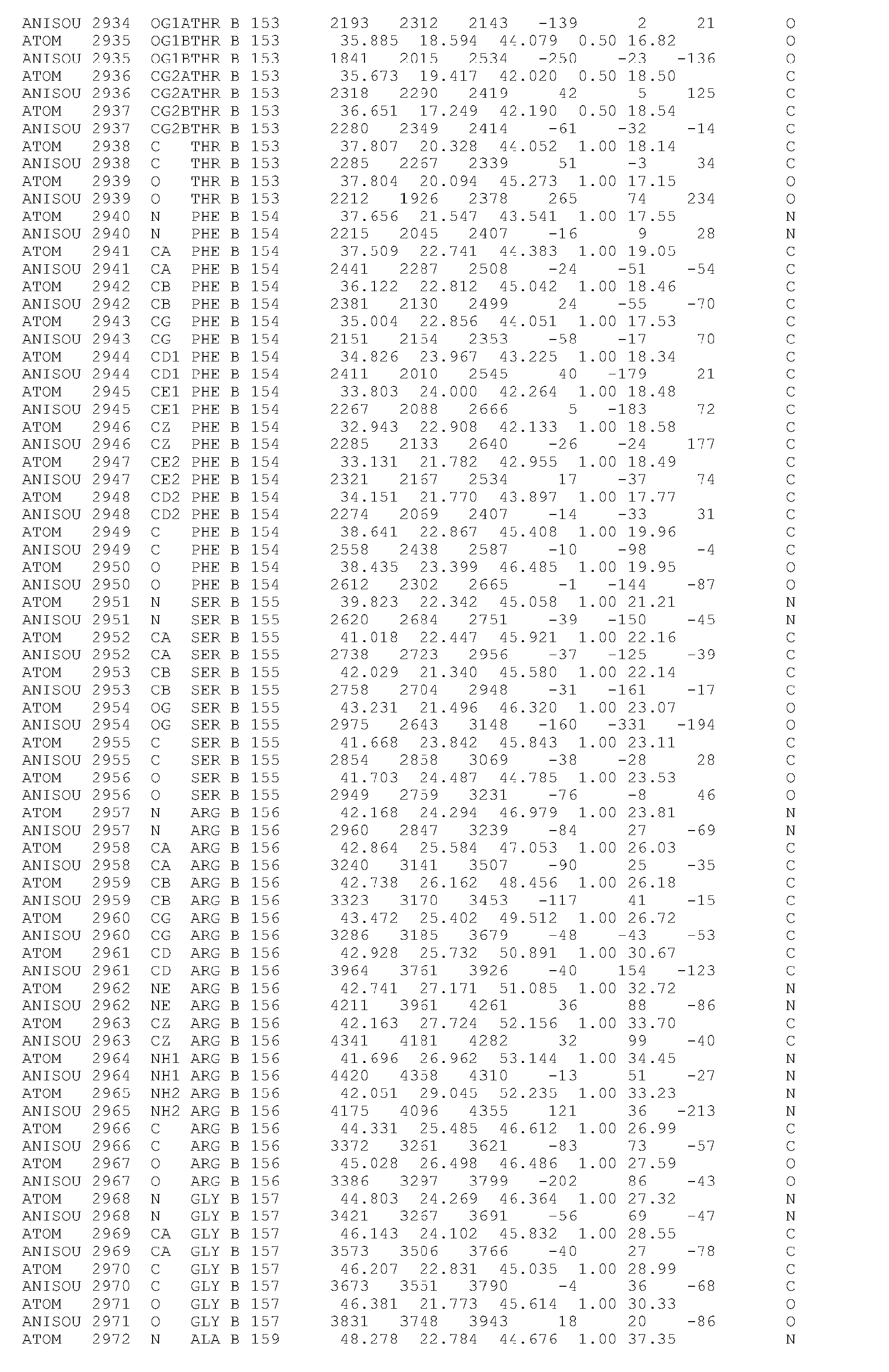

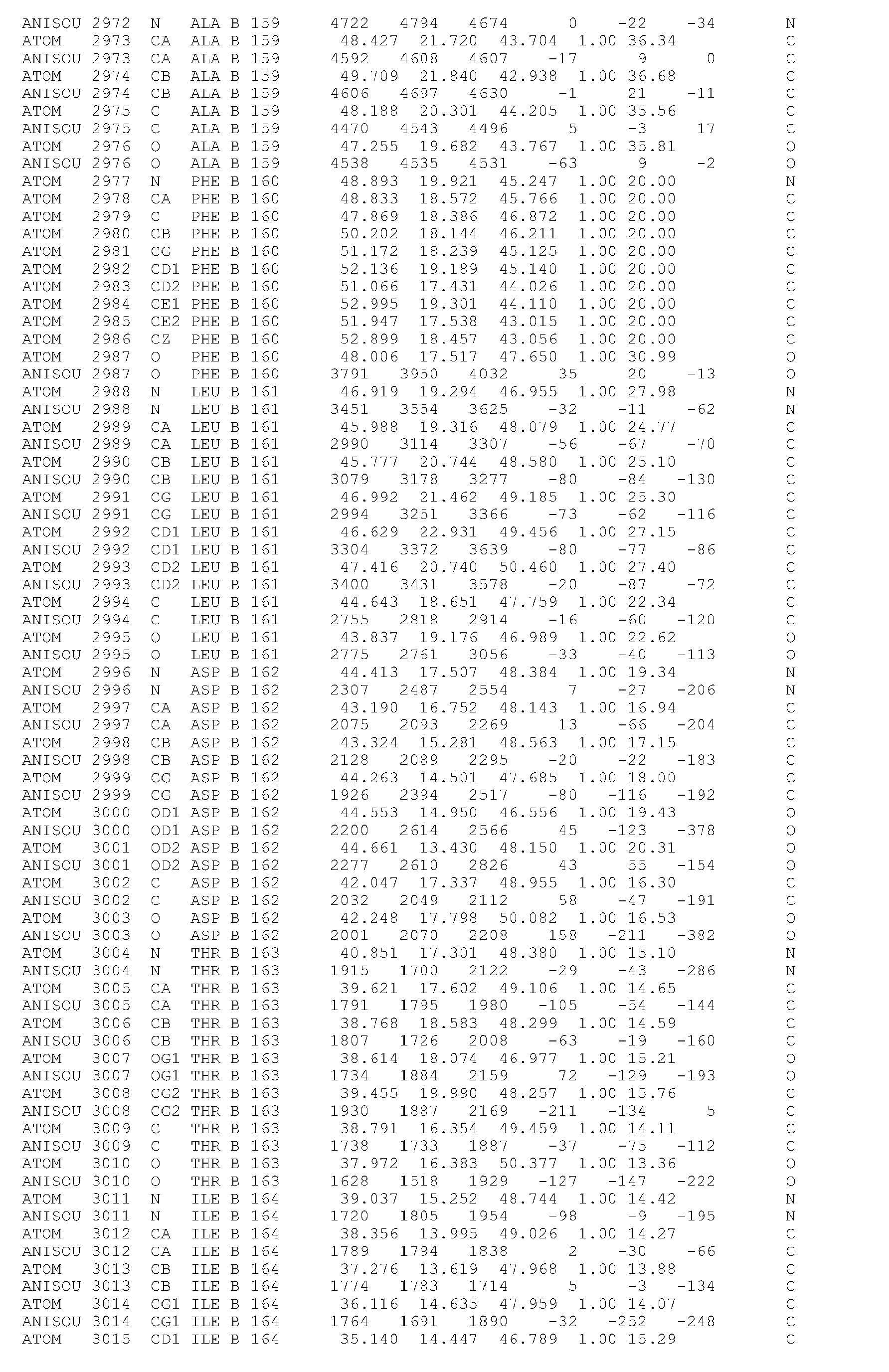

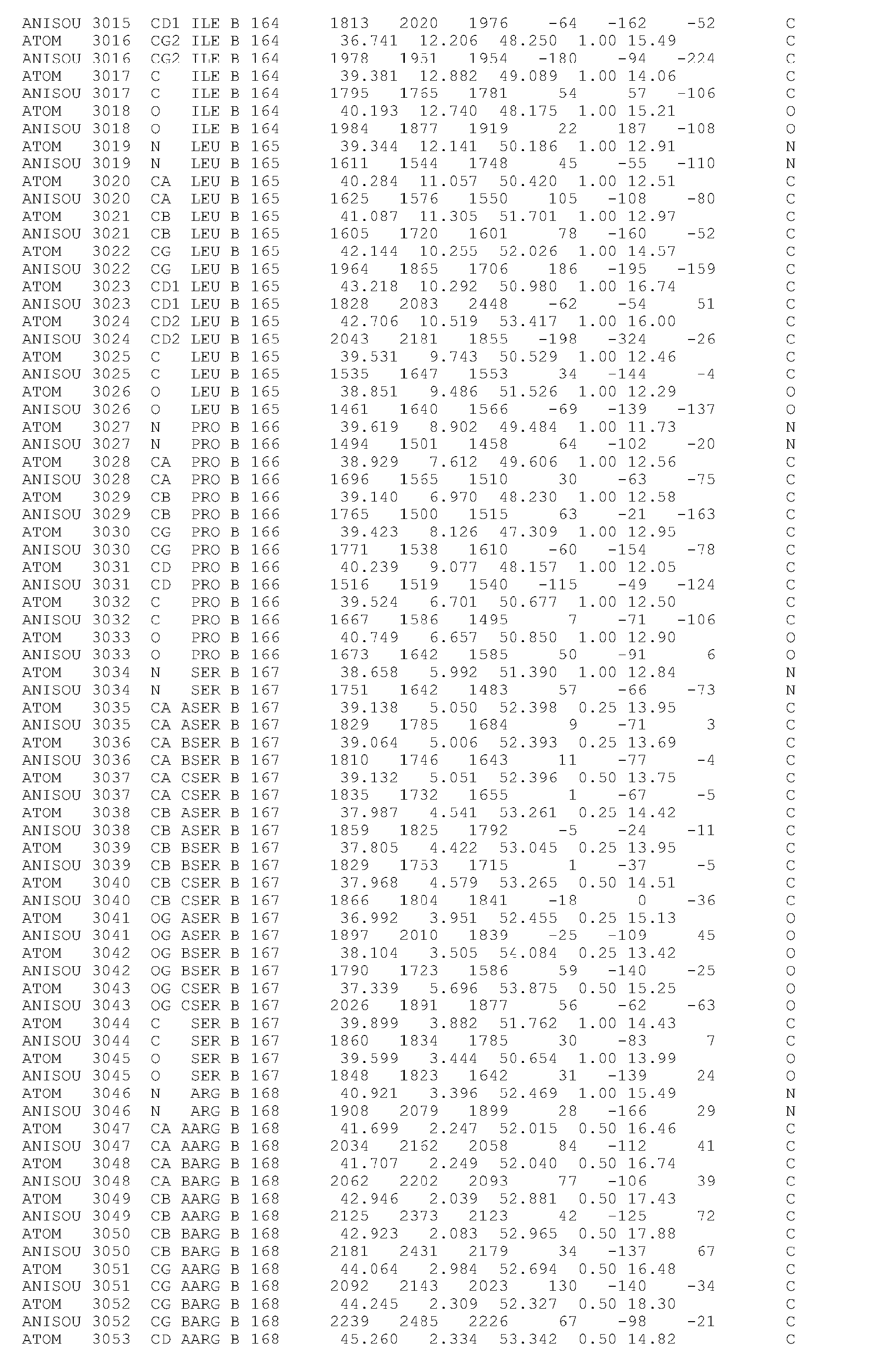

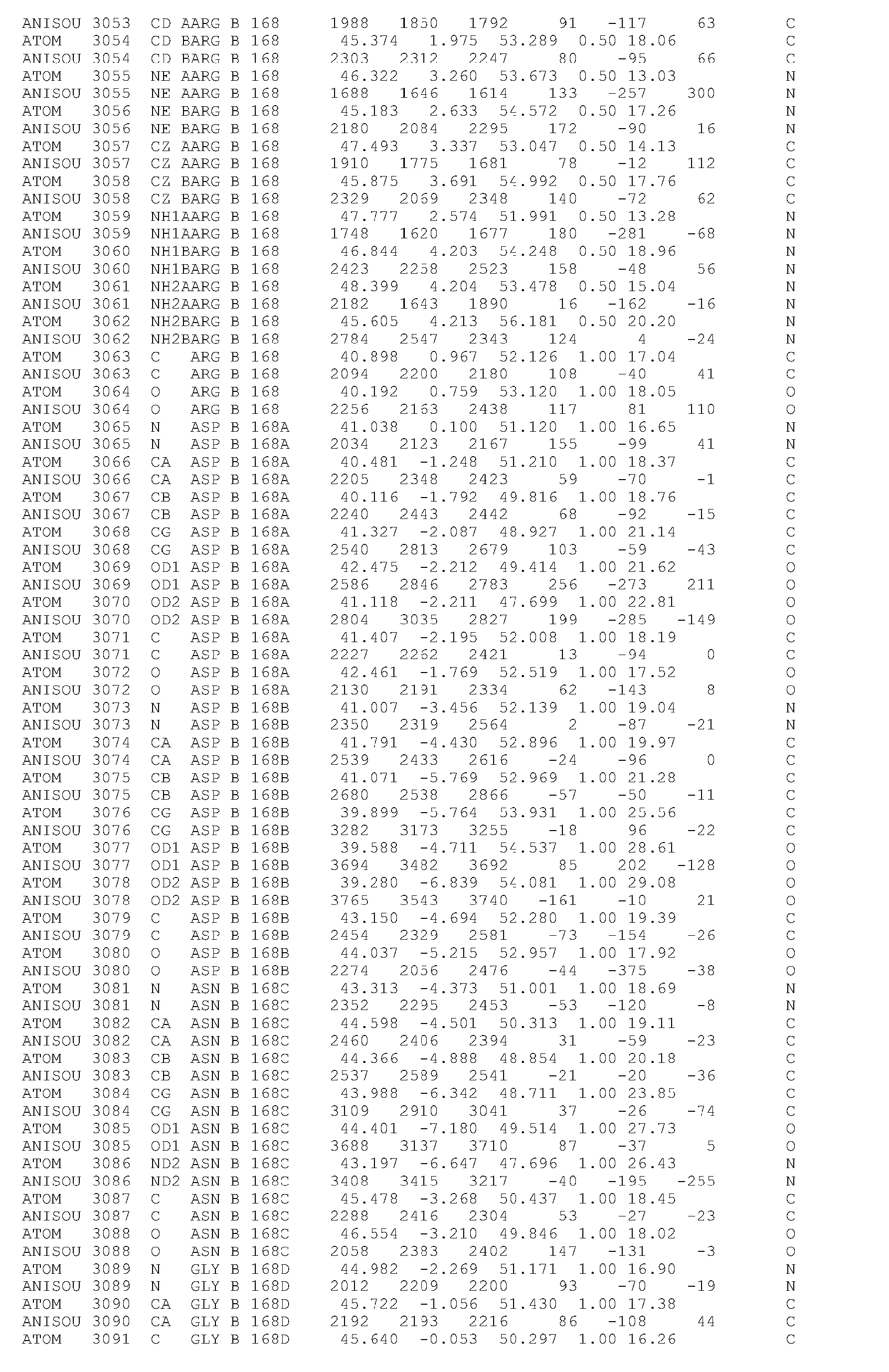

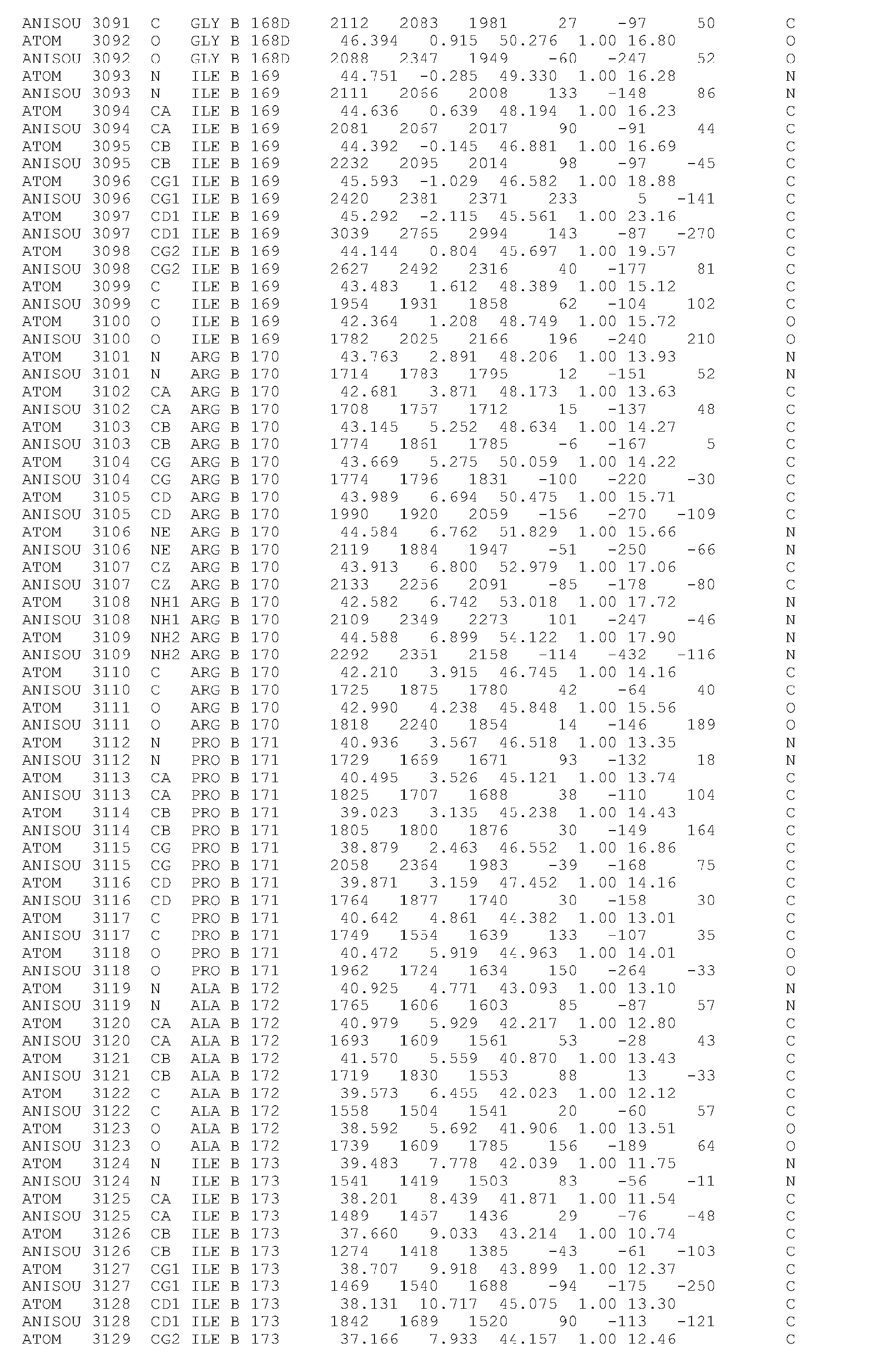

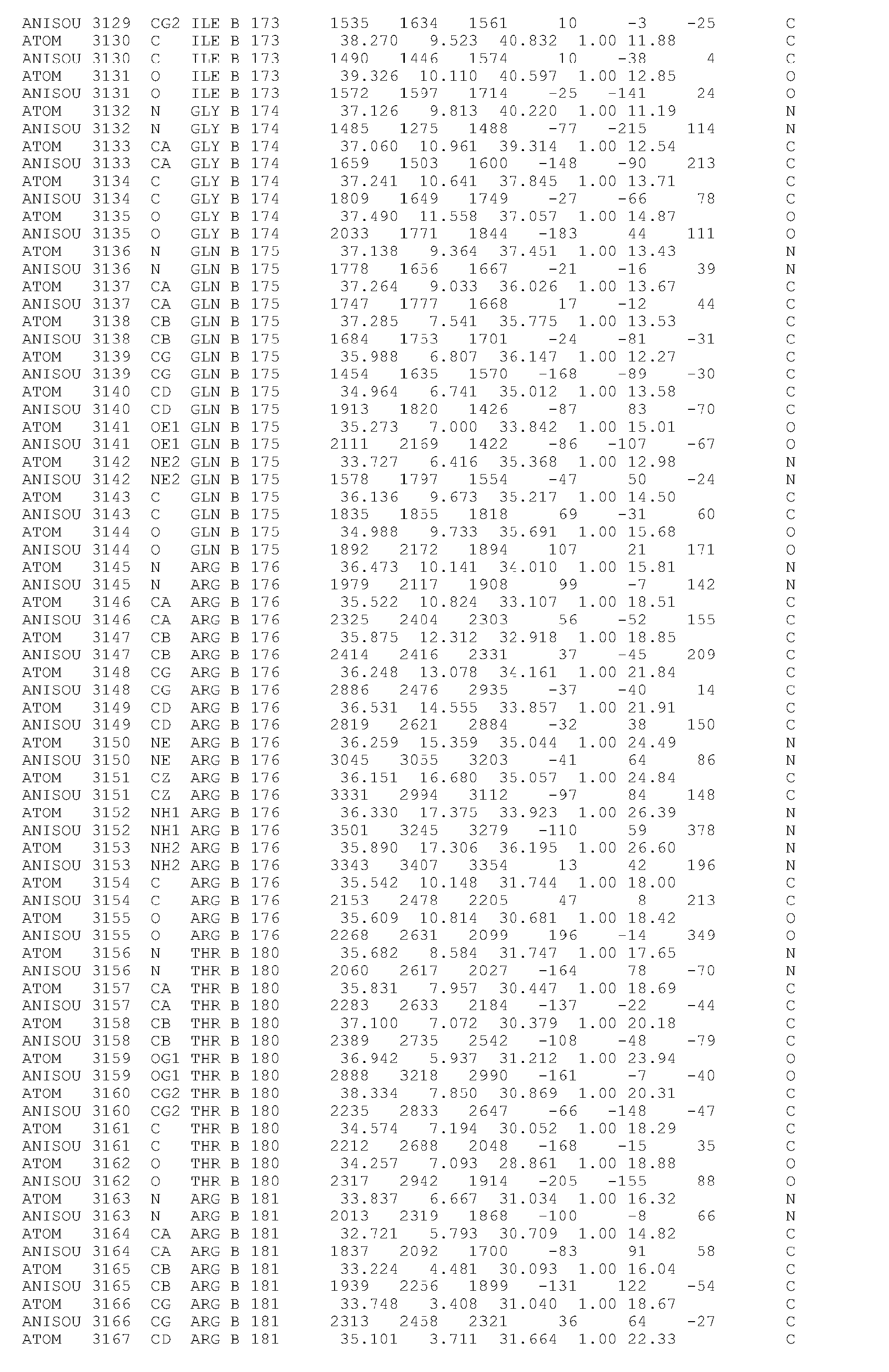

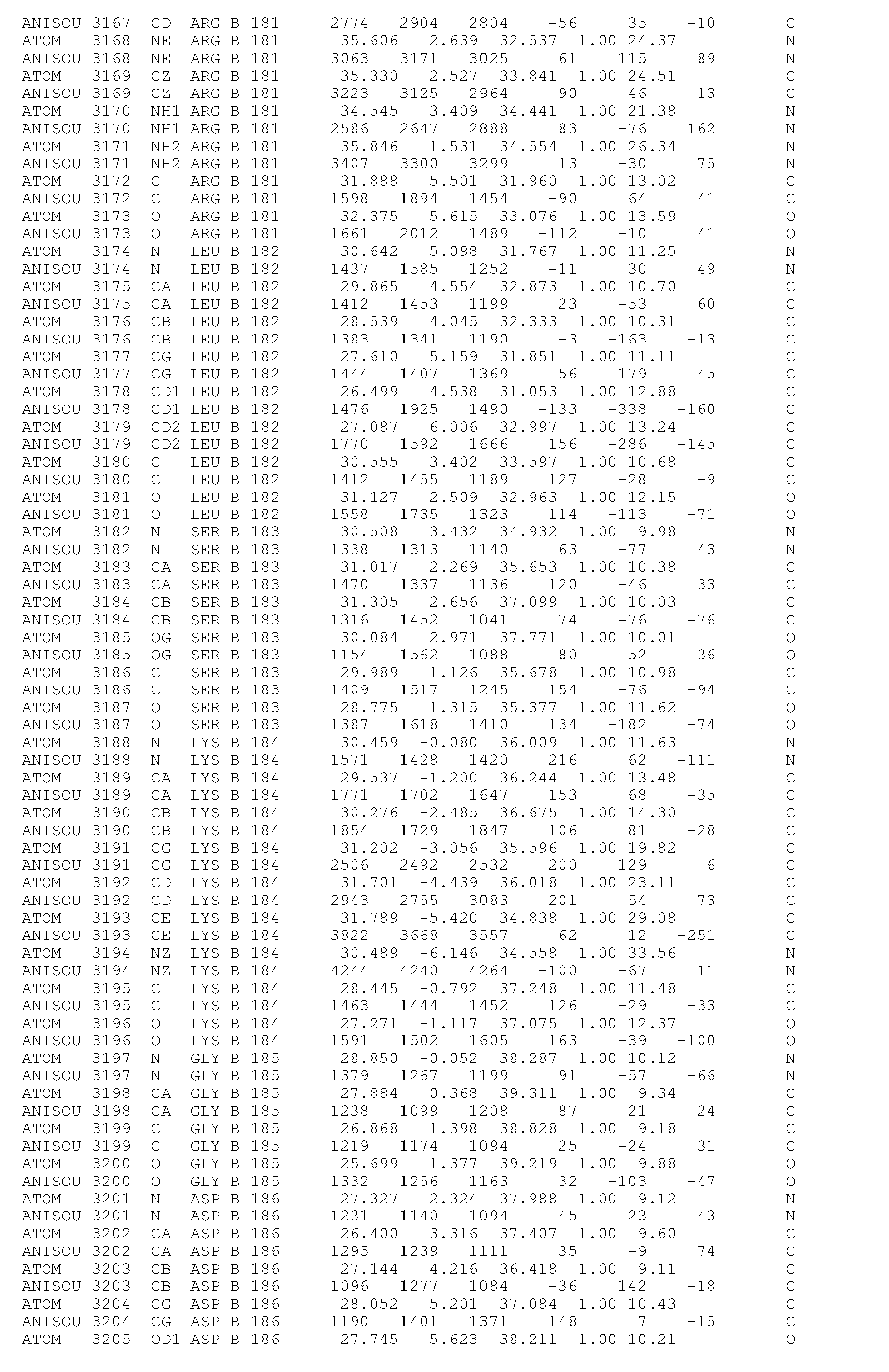

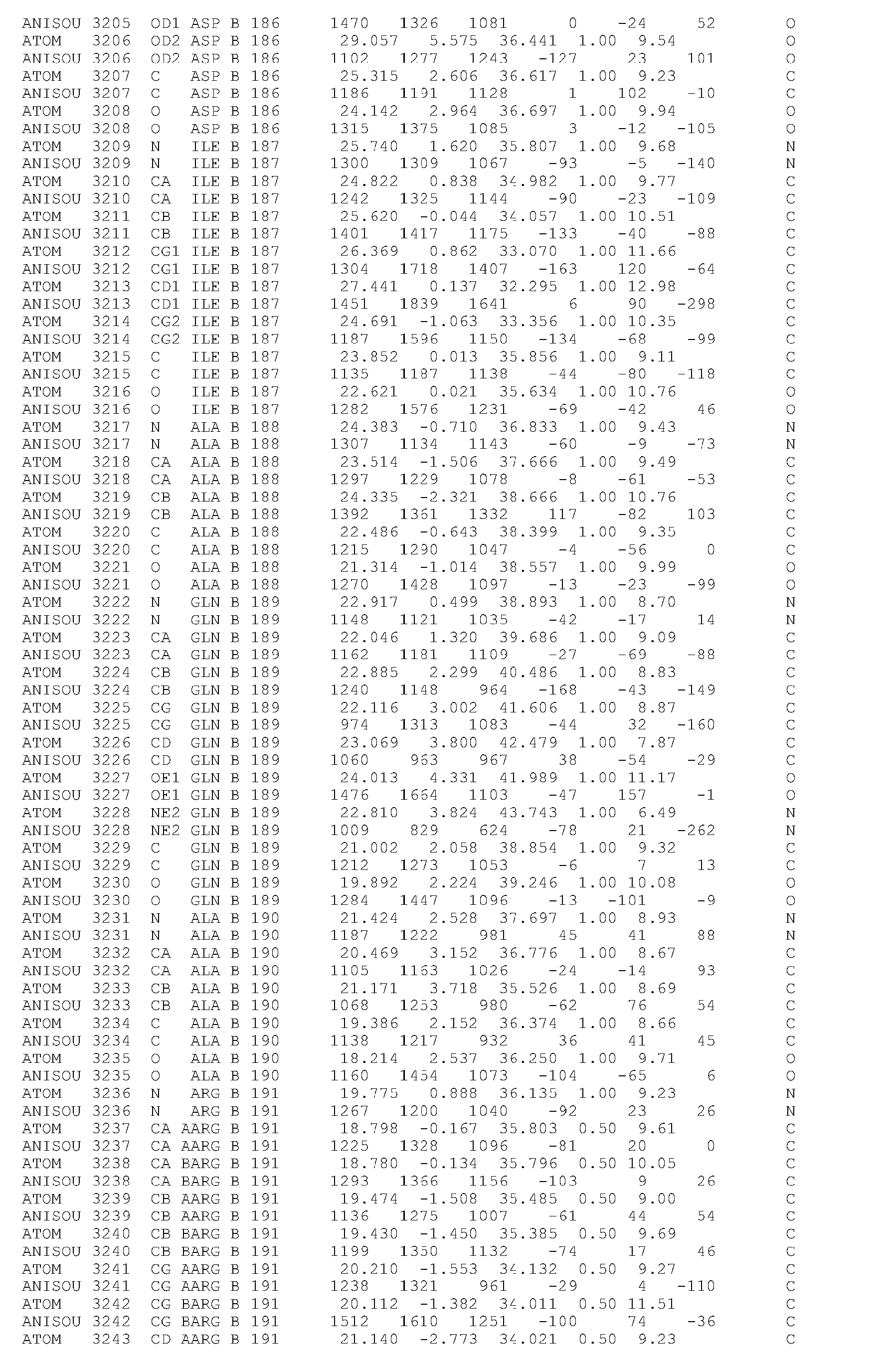

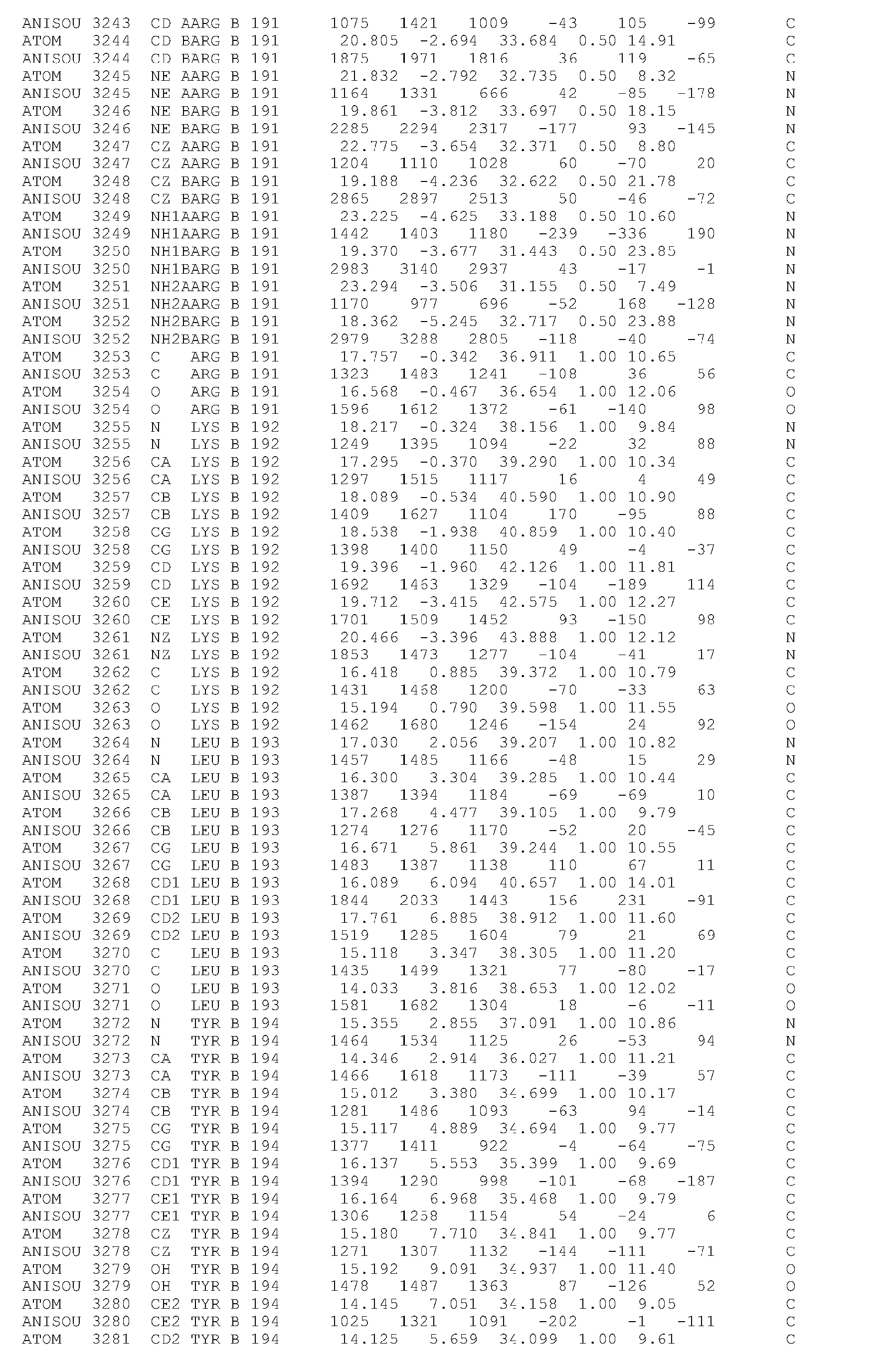

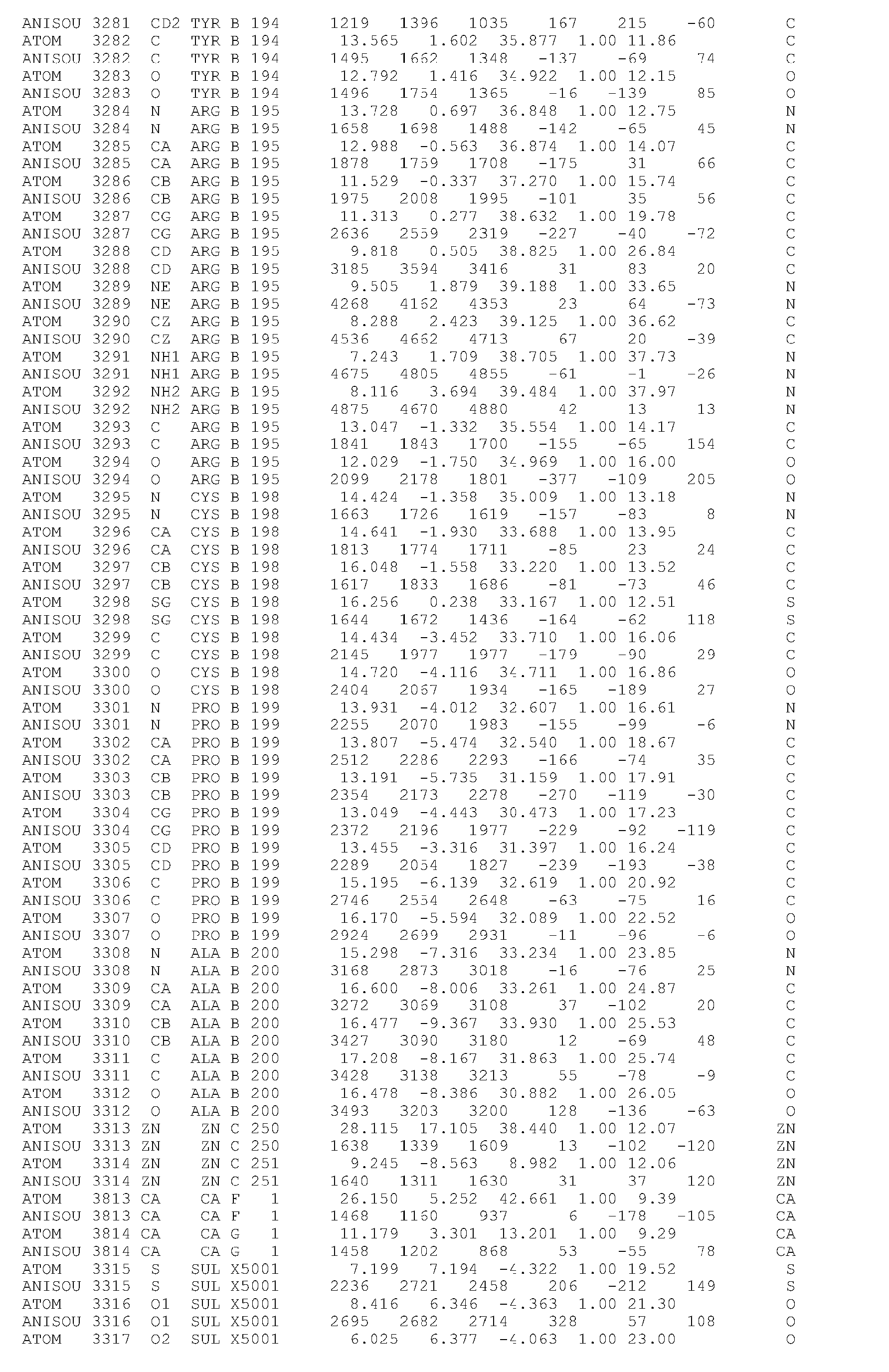

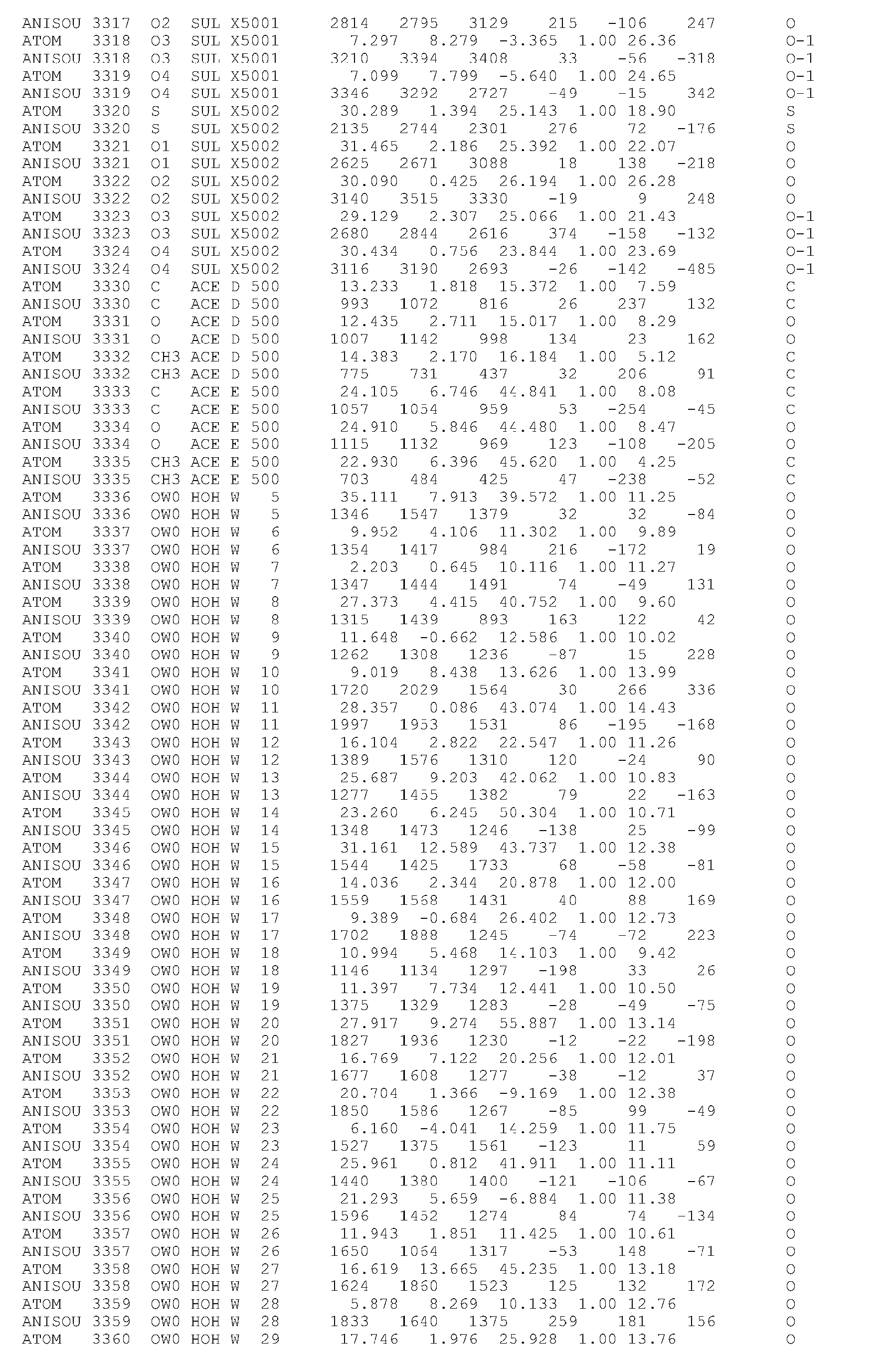

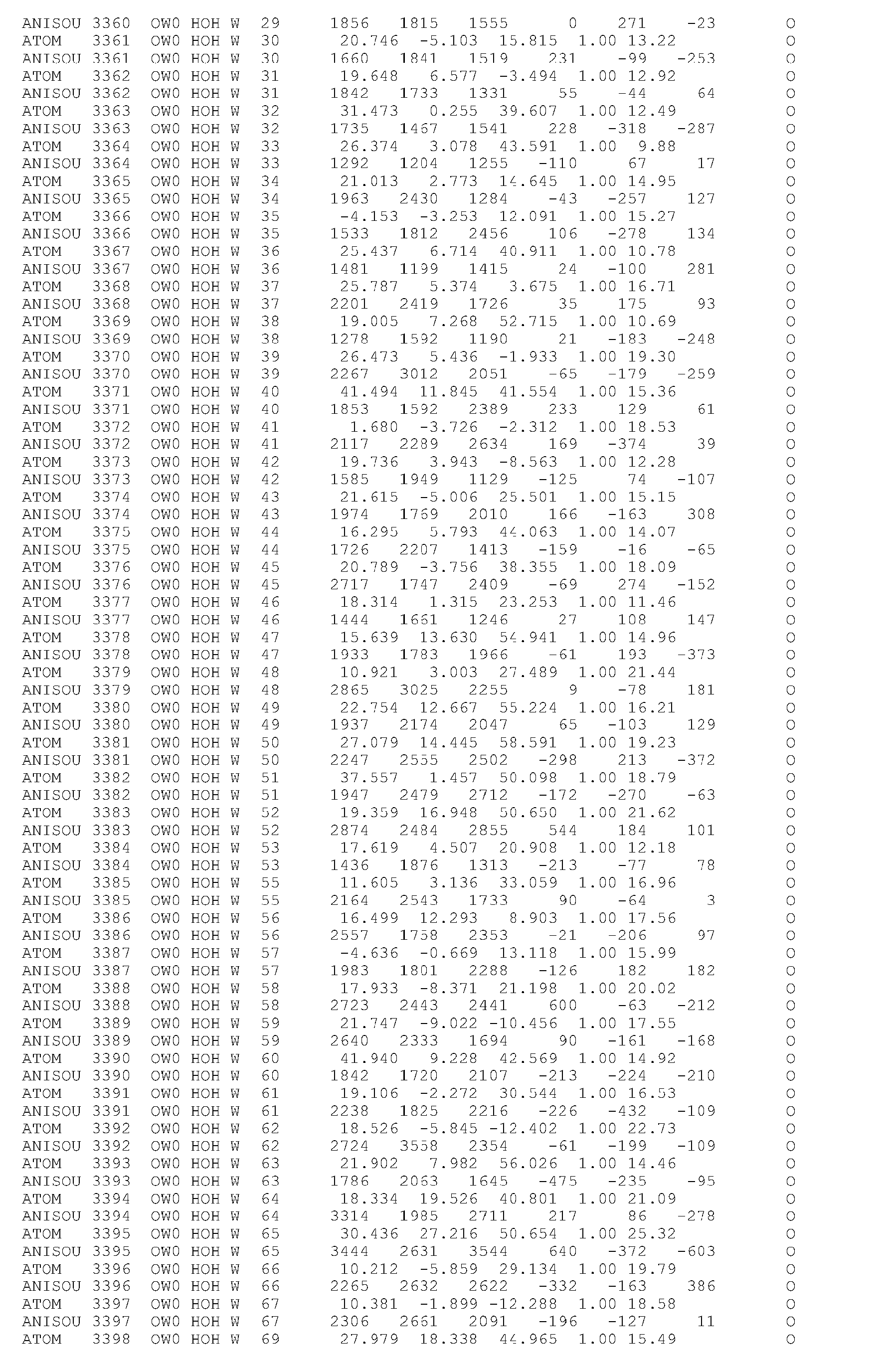

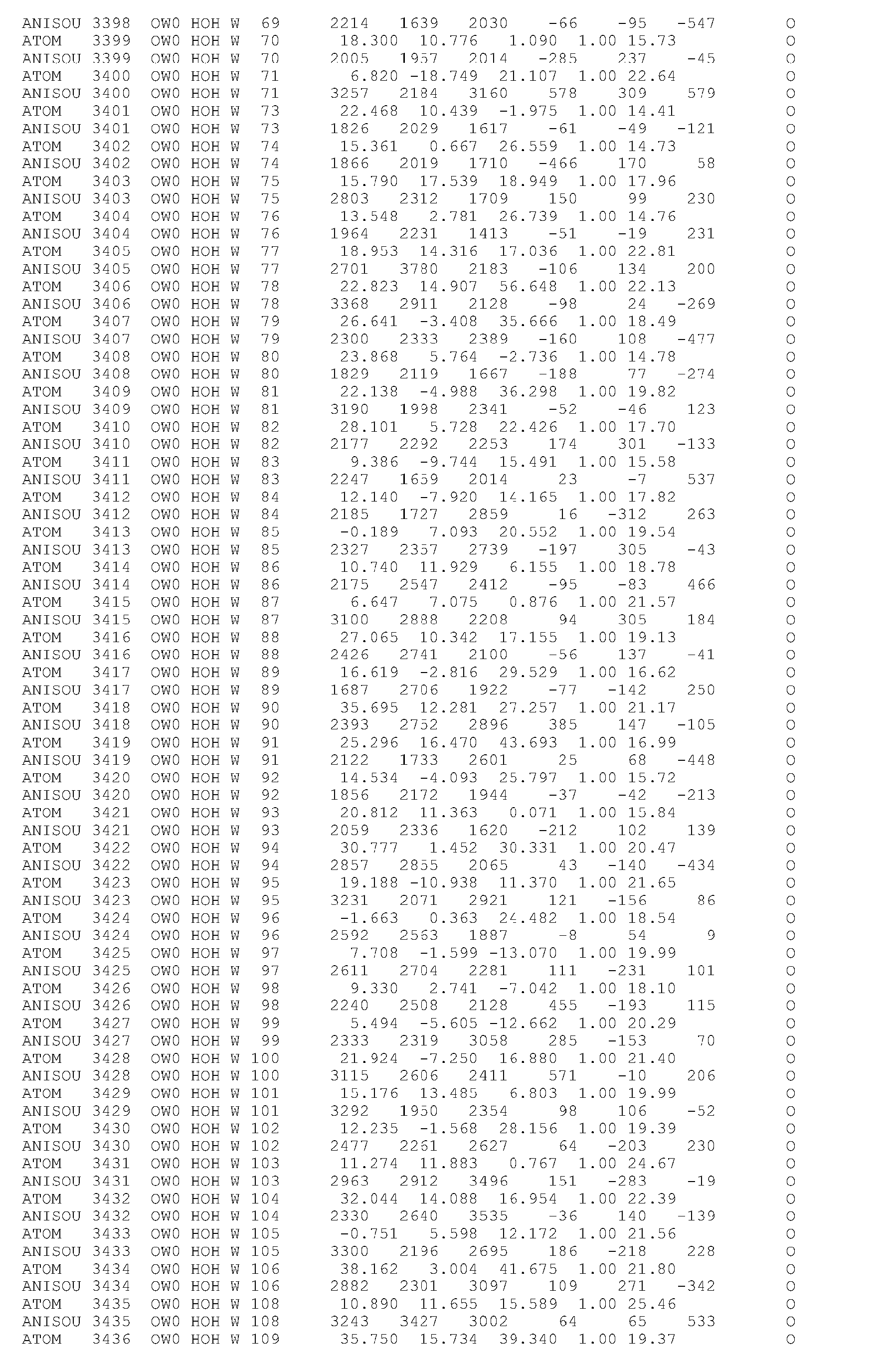

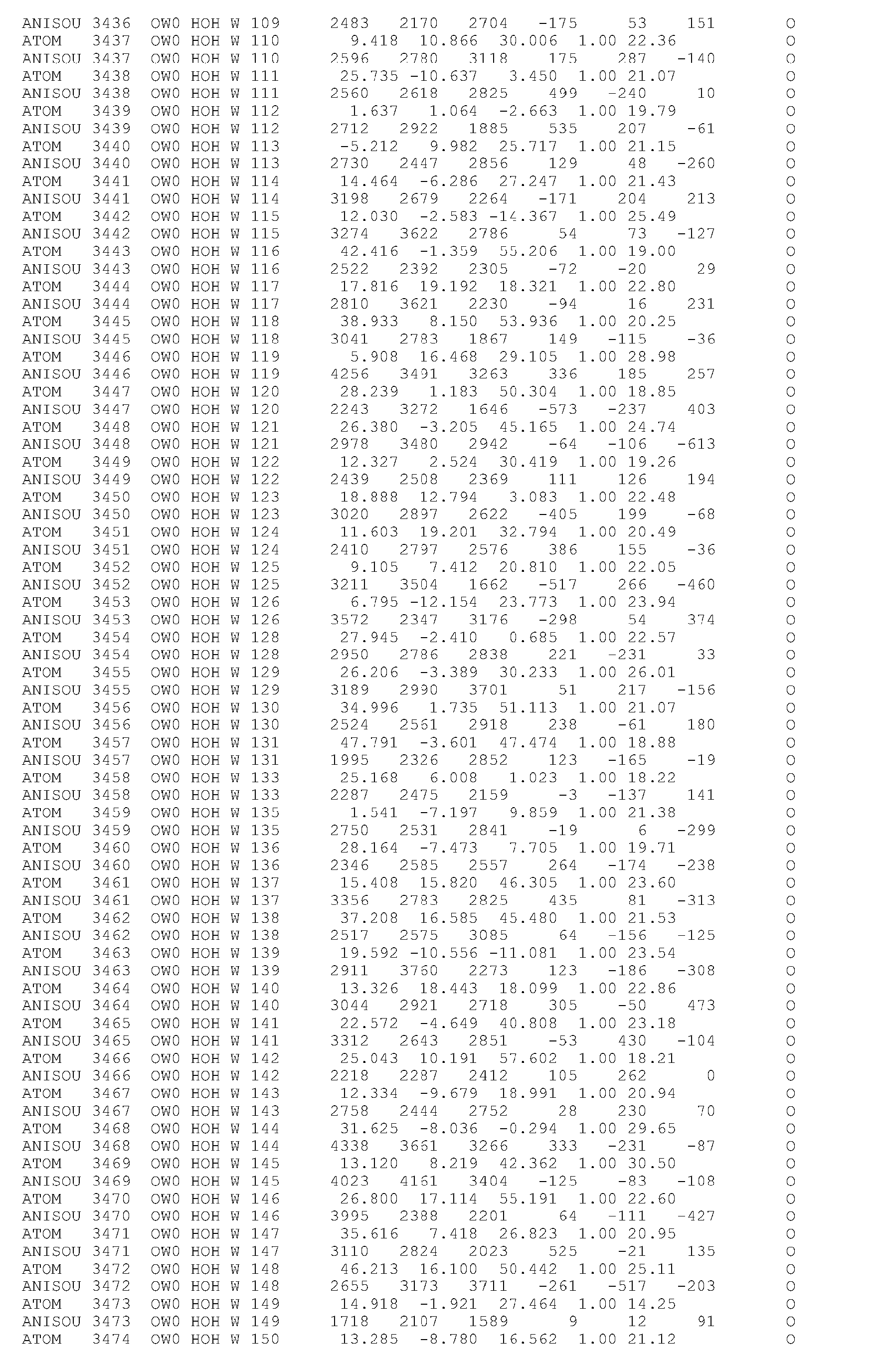

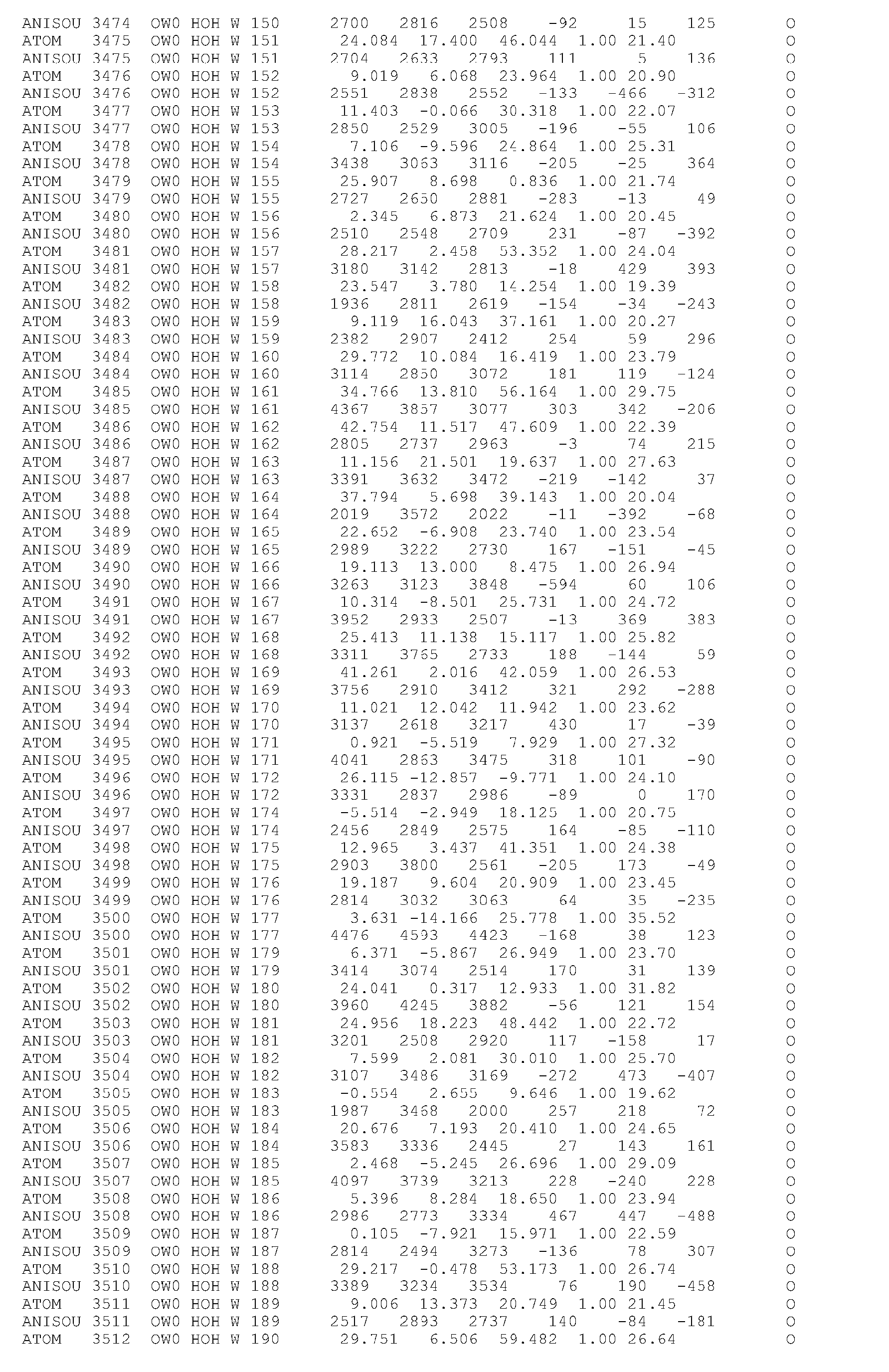

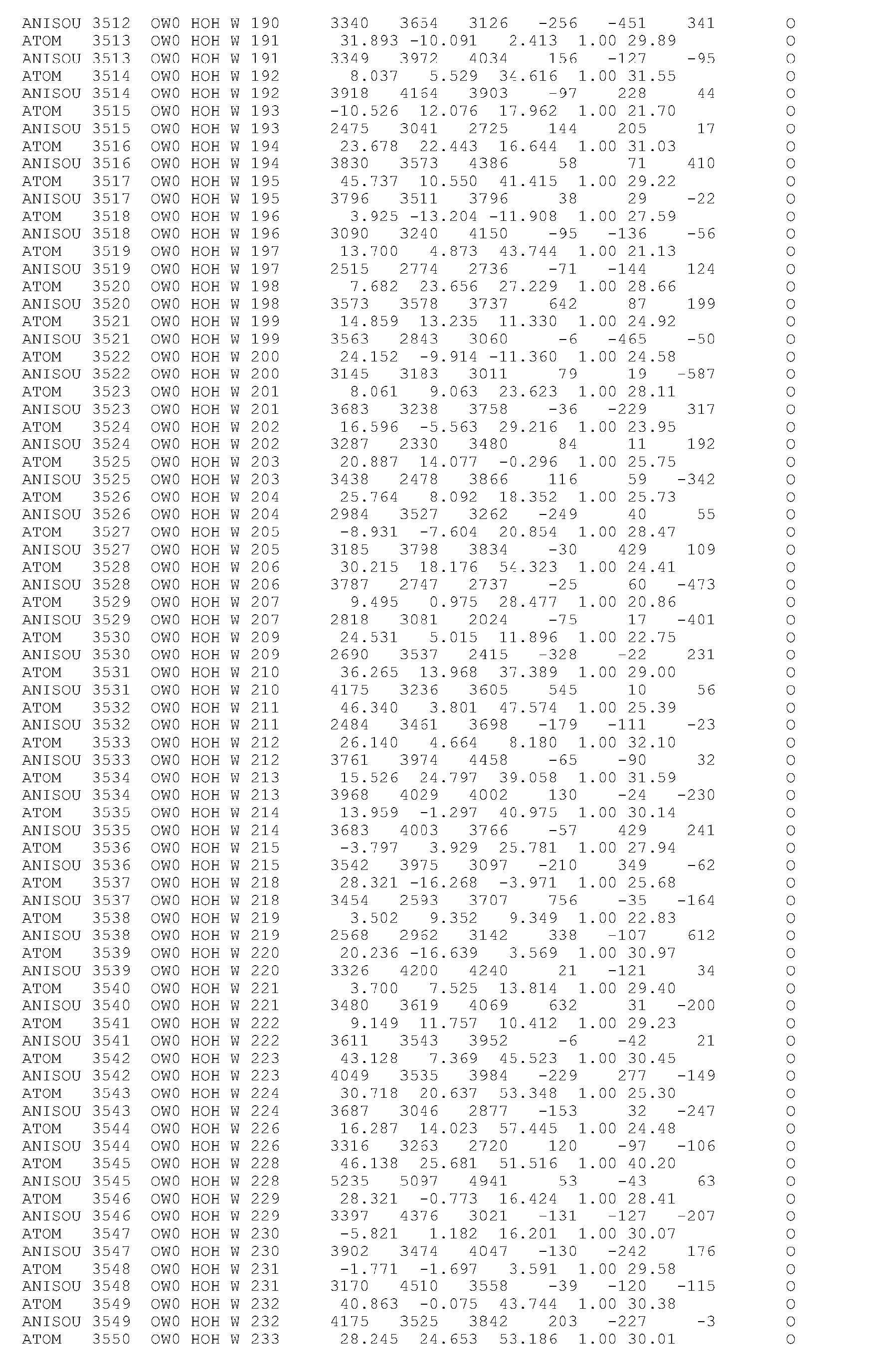

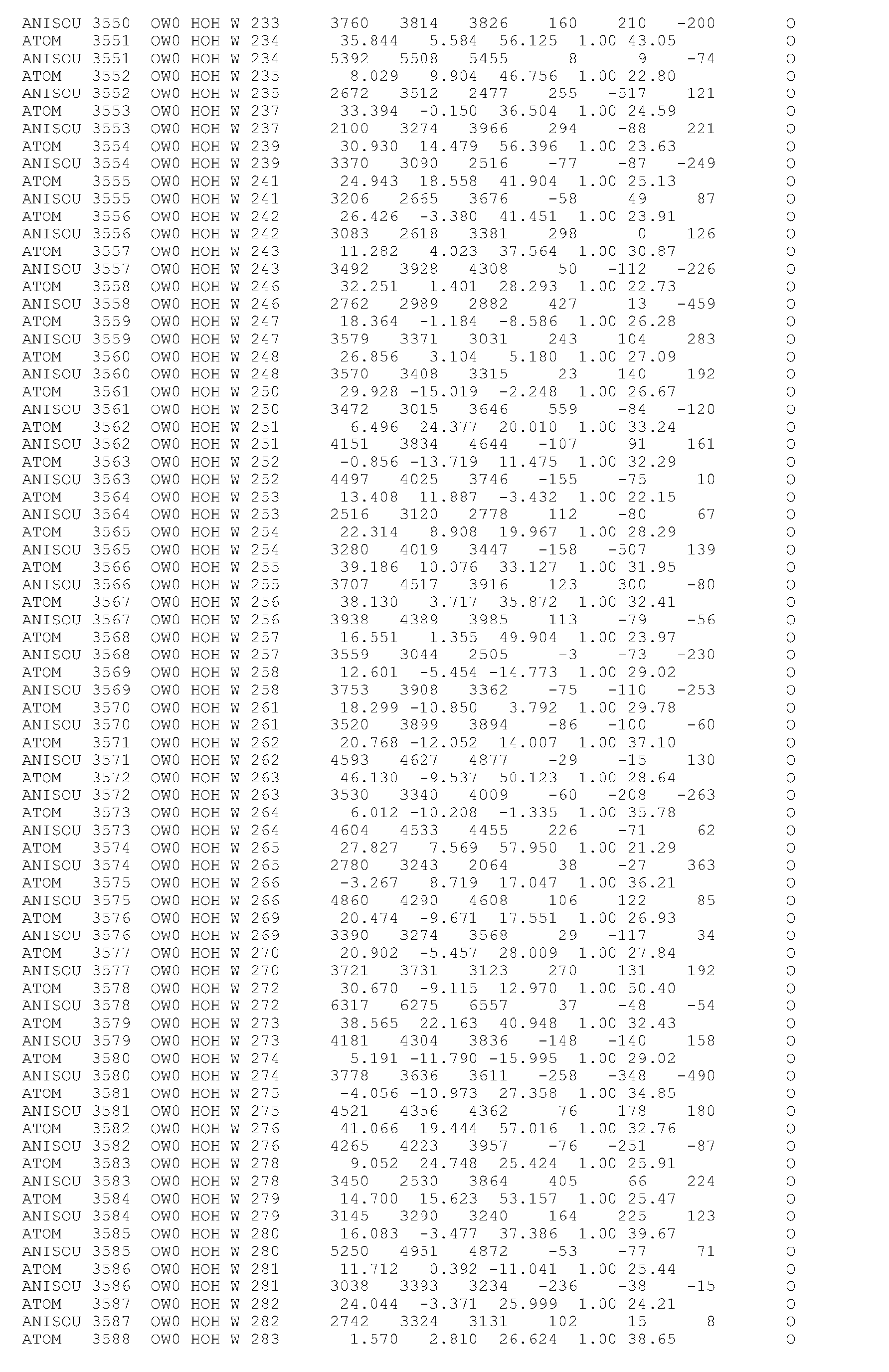

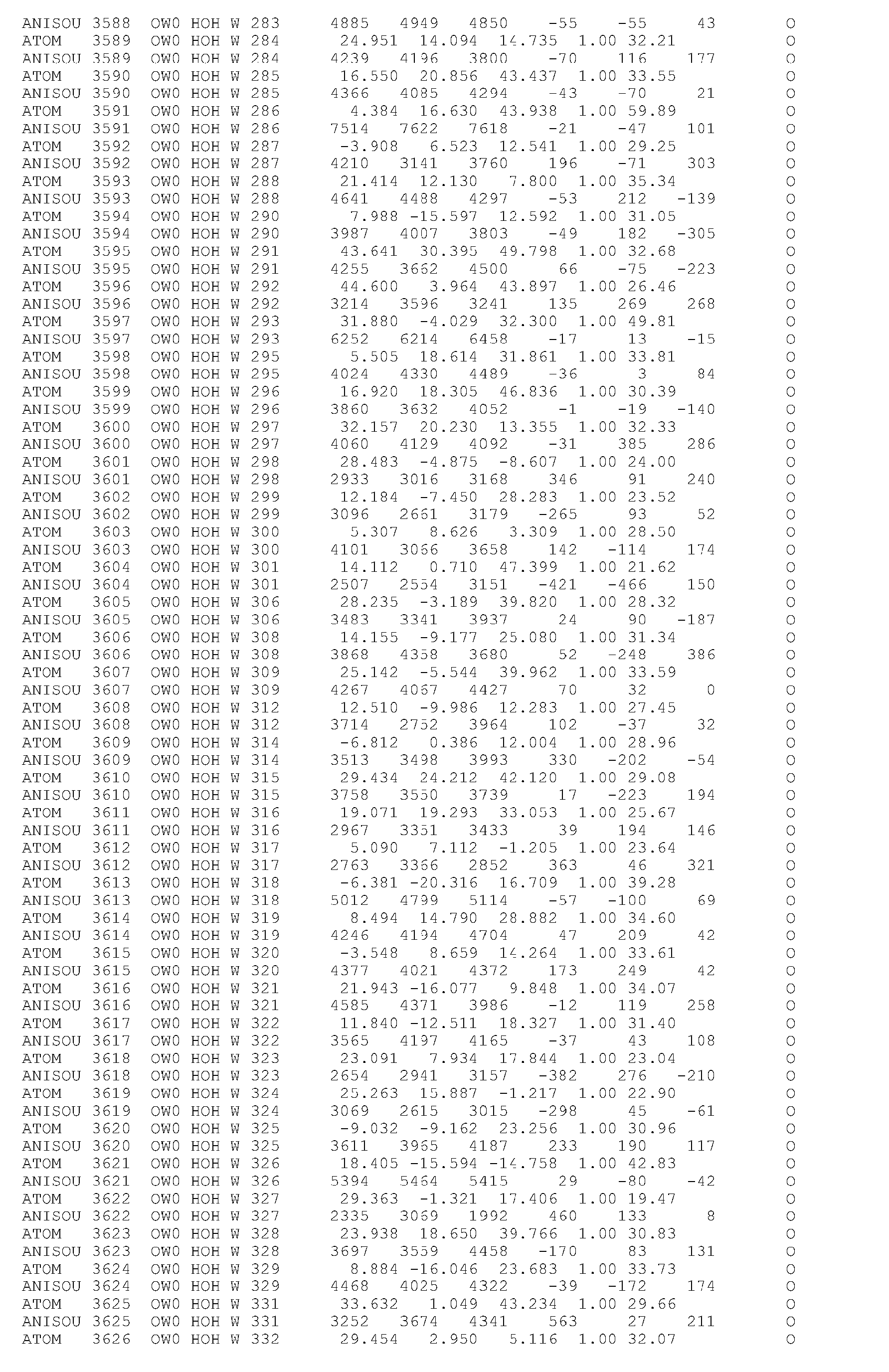

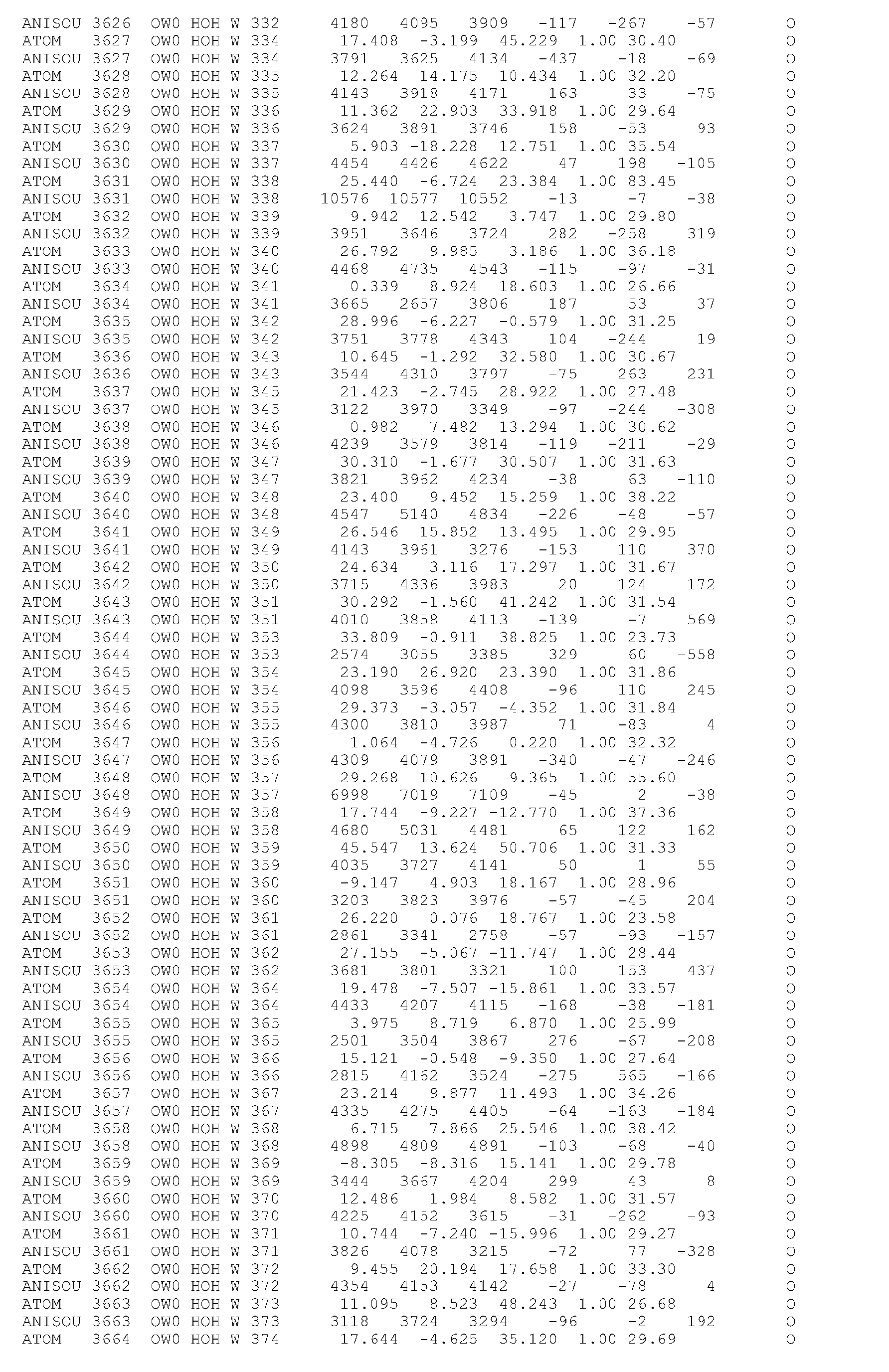

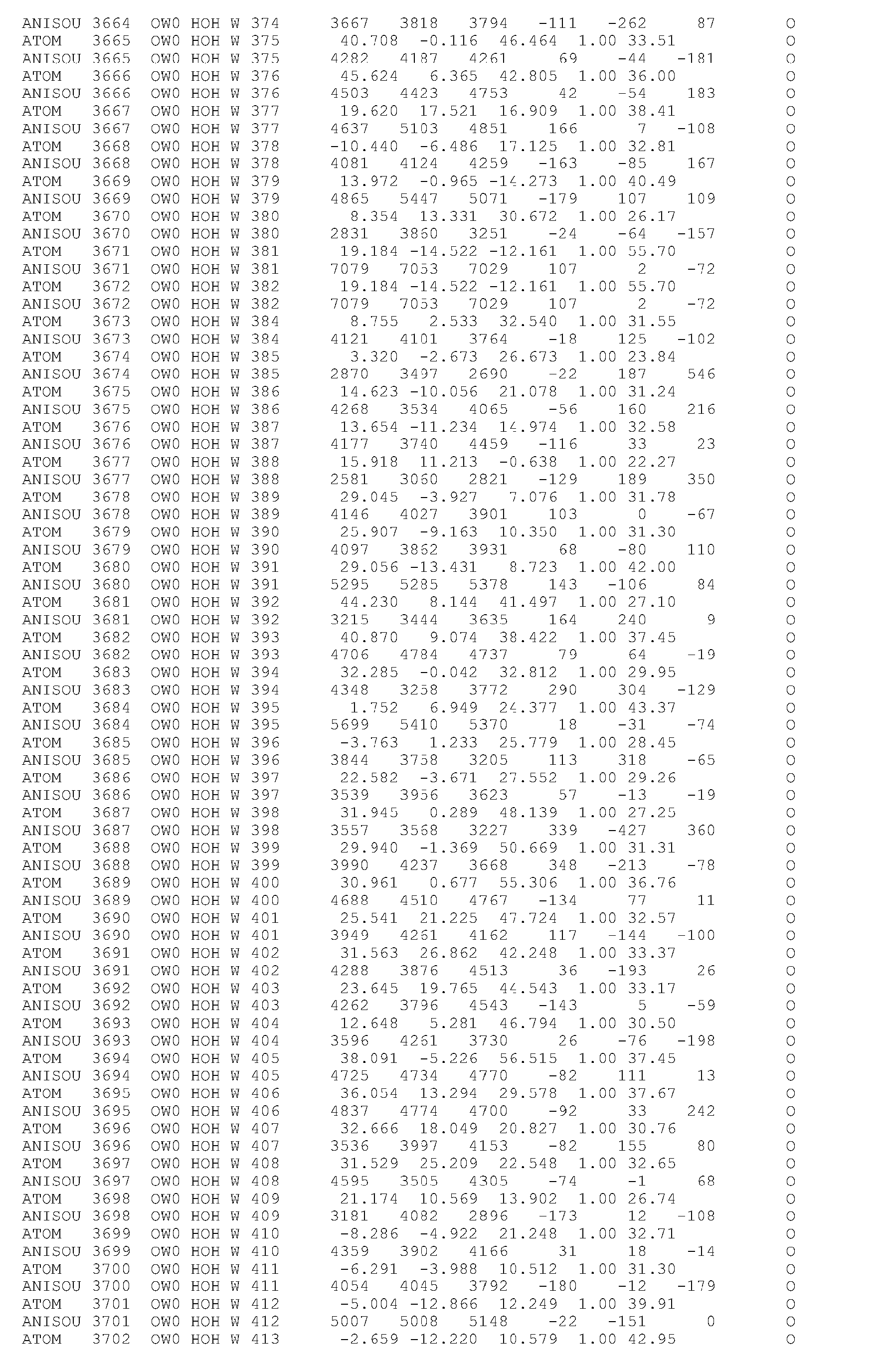

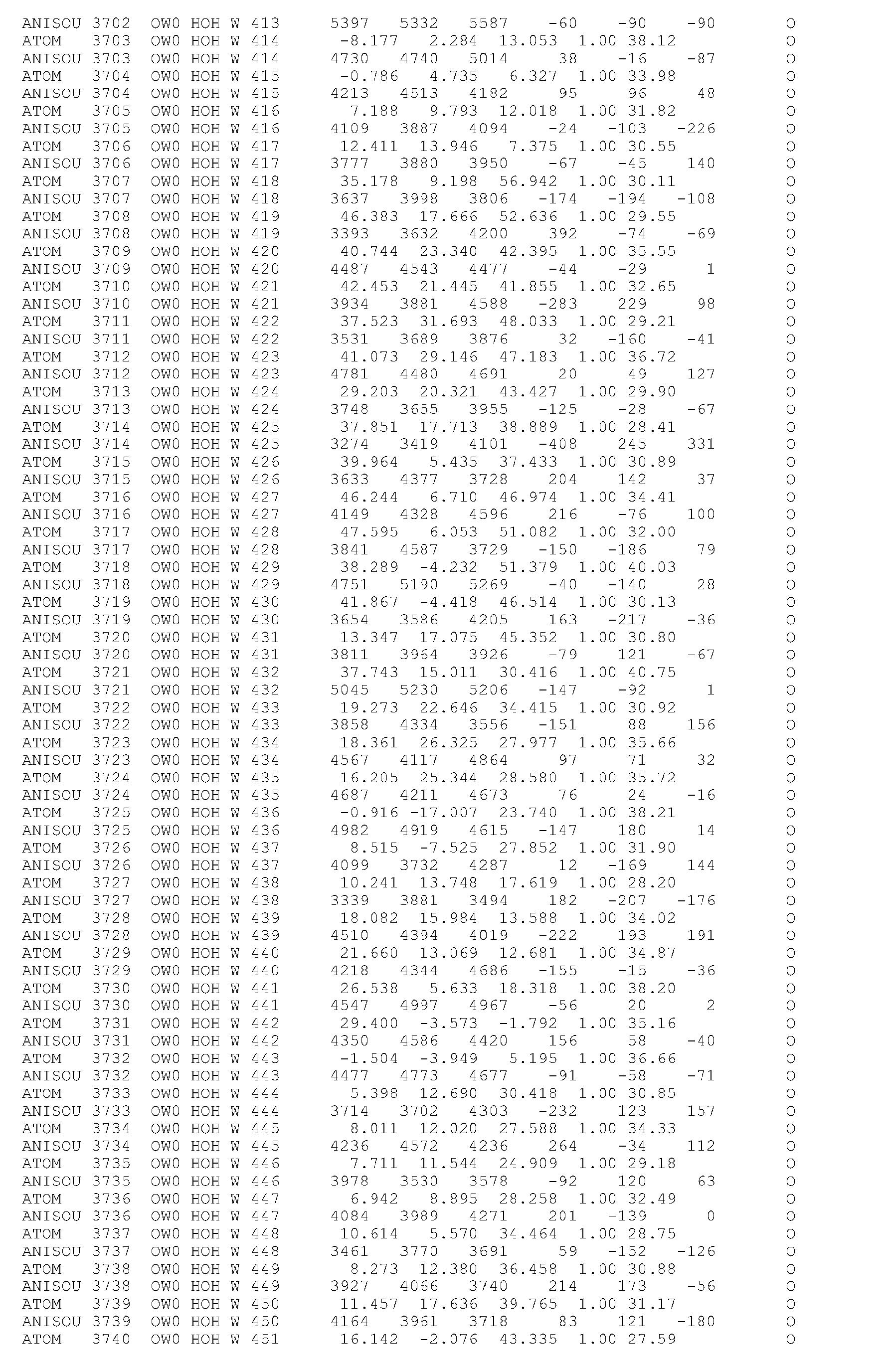

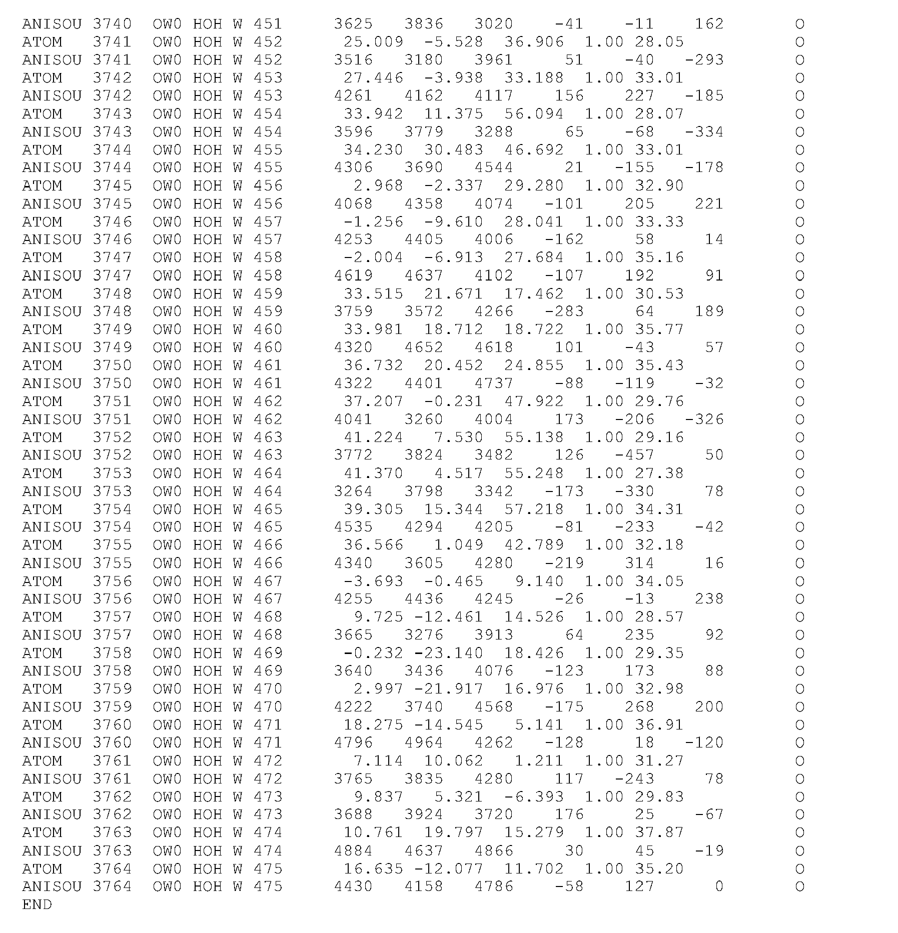

### References

Scott IC, Blitz IL, Pappano WN, et al (1999) Mammalian BMP-1/tolloid-related metalloproteinases, including novel family member mammalian tolloid-like 2, have differential enzymatic activities and distributions of expression relevant to patterning and skeletogenesis, Developmental Biology; 213 (2):283-300.
Kessler E, Takahara K, Biniaminov L, et al (1996) Bone morphogenetic protein-1: The type I procollagen C-proteinase, Science; 271 (5247):360-2.

Uzel MI, Scott IC, Babakhaniou-Chase H, et al (2001) Multiple bone morphogenetic protein 1-related mammalian metalloproteinases process pro-lysyl oxidase at the correct physiological site and control lysyl oxidase activation in mouse embryo fibroblast cultures, Journal of Biological Chemistry; 276 (25):22537-43.
McPherron AC, Lee SJ (1997) Double muscling in cattle due to mutations in the myostatin gene, Proceedings of the National Academy of Sciences of the United States of America; 94 (23):12457-61.
McPherron AC, Lawler AM, Lee SJ (1997) Regulation of skeletal muscle mass in mice by a new TGF-beta superfamily member, Nature; 387 (6628):83-90.
Lin J, Arnold HB, la-Fera MA, et al (2002) Myostatin knockout in mice increases myogenesis and decreases adipogenesis, Biochemical and Biophysical Research Communications; 291 (3):701-6.
Wolfman NM, McPherron AC, Pappano WN, et al (2003) Activation of latent myostatin by the BMP-1/tolloid family of metalloproteinases. Proceedings of the National Academy of Sciences of the United States of America; 100 (26):15842-6.
Bogdanovich S, Krag TOB, Barton ER, Morris LD, Whittemore LA, Ahima RS, Khurana TS. (2002) Functional improvement of dystrophic muscle by myostatin blockade. Nature, 420:418-421.
Kabsch W (1993) Automatic Processing of Rotation Diffraction Data from Crystals of Initially Unknown Symmetry and Cell Constants, Journal of Applied Crystallography; 26:795-800. Kroemer M, Dreyer MK, Wendt KU (2004) APRV - a program for automated data processing, refinement and visualization. Acta Crystallographica Section D-Biological Crystallography; 60:1679-82.
Vagin A, Teplyakov A (1997) MOLREP: an automated program for molecular replacement. Journal of Applied Crystallography; 30:1022-5.
Bode W, Gomisruth FX, Huber R, et al (1992) Structure of Astacin and Implications for Activation of Astacins and Zinc-Ligation of Collagenases. Nature; 358 (6382):164-7.
Emsley P, Cowtan K (2004) Coot: model-building tools for molecular graphics. Acta Crystallographica Section D-Biological Crystallography; 60:2126-32.
Engh RA, Huber R (1991) Accurate Bond and Angle Parameters for X-Ray Protein-Structure Refinement. Acta Crystallographica Section A; 47:392-400.
Laskowski RA, MacArthur MW, Moss DS, et al (1993) Procheck - A Program to Check the Stereochemical Quality of Protein Structures. Journal of Applied Crystallography; 26:283-91. Murshudov GN, Vagin AA, Dodson EJ (1997) Refinement of macromolecular structures by the maximum-likelihood method. Acta Crystallog.sect.D; 53:240-55.

Murshudov GN, Vagin A, Lebedev A, et al (1999) Efficient anisotropic refinement of macromolecular structures using FFT. Acta Crystallographica Section D-Biological Crystallography; 55:247-55.
Garrigue-Antar L, Barker C, Kadler KE. (2001) Identification of amino acid residues in bone morphogenetic protein-1 important for procollagen C-proteinase activity. Journal of Biological Chemistry, 276:26237-42.
Bhattacharyya R, Pal D, Chakrabarti P. (2004) Disulfide bonds, their stereospecific environment and conservation in protein structures. Protein Engineering Design & Selection, 17:795-808.
Gross E, Sevier CS, Vala A, Kaiser CA, Fass D. (2002) A new FAD-binding fold and intersubunit disulfide shuttle in the thiol oxidase Erv2p. Nature Structural Biology, 9:61-67. Rawlings ND, Morton FR, Barrett AJ. (2006) MEROPS: the peptidase database. Nucleic Acids Research 34:D270-D272.
Grams F, Dive V, Yiotakis A, Yiallouros I, Vassiliou S, Zwilling R, Bode W, Stocker W. (1996) Structure of astacin with a transition-state analogue inhibitor. Nature Structural Biology, 3:671-675.

## Claims

1. A crystal comprising the catalytic domain of tolloid-like protein 1 (TLL1cd) or a mutant thereof.

2. The crystal of claim 1 wherein said tolloid-like protein 1 is human TLL1cd (SEQ ID NO:1).

3. The crystal of claims 1 or 2 have a cell size of 41 x 48 x 59 Angstroms

4. The crystal of claims 1-3 having a three-dimensional structure **characterized by** the structure coordinates of Table 3.

5. The crystal of claims 1-4 further comprising a co-crystallised ligand.

6. A method for making a crystal of the TLL1cd protein according to claims 1-4, said method comprising the steps of:
(a) expressing a construct comprising a nucleotide sequence encoding for TLL1 cd;
(b) purifying a sufficient amount of the TLL1 cd protein produced at step (a) under appropriate conditions for crystallization; and,
(c) crystallizing the TLL1cd protein purified at step (b).

7. The method of claim 1 further comprising the step of adding a ligand to the purified protein of step (b).

8. A computer readable medium comprising data storage material encoded with computer readable data wherein said data comprises the structure coordinates of a crystal according to claims 1-5.

9. Use of a crystal according to any one of claims 1 to 5, for the generation of crystal structure data.

10. A method of identifying a ligand that binds to TLL1cd comprising the steps of:
(i) using the three dimensional structure data generated according to claim 9 to select and/or design a potential ligand that binds to TLL1cd,
(ii) identifying among the potential ligand selected in step (i), those ligands that bind to TLL1cd in an *in vitro, in vivo* or cell-based assay.

11. A method for determining the three-dimensional structure of a complex comprising the TLL1cd protein, a fragment or homologue thereof comprising:
(a) obtaining x-ray diffraction data for crystals of the complex,
(b) utilizing the atomic coordinates generated according to claim 9 to define the three-dimensional structure of the complex.

12. Use of an antagonist of TLL1 cd for the manufacture of a medicament to treat muscle-wasting related disorders.


SEQUENCE LISTING

<110>  Novartis AG

<120>  Crystal structure of the catalytic domain of tolloid-like
       protease 1 and uses thereof

<130>  50552

<160>  1

<170>  PatentIn version 3.3

<210>  1
<211>  202
<212>  PRT
<213>  Homo sapiens

<400>  1

Met Ala Ala Thr Ser Arg Thr Glu Arg Ile Trp Pro Gly Gly Val Ile
1               5                   10                  15

Pro Tyr Val Ile Gly Gly Asn Phe Thr Gly Ser Gln Arg Ala Met Phe
            20                  25                  30

Lys Gln Ala Met Arg His Trp Glu Lys His Thr Cys Val Thr Phe Ile
        35                  40                  45

Glu Arg Ser Asp Glu Glu Ser Tyr Ile Val Phe Thr Tyr Arg Pro Cys
    50                  55                  60

Gly Cys Cys Ser Tyr Val Gly Arg Arg Gly Asn Gly Pro Gln Ala Ile
65                  70                  75                  80

Ser Ile Gly Lys Asn Cys Asp Lys Phe Gly Ile Val Val His Glu Leu
                85                  90                  95

Gly His Val Ile Gly Phe Trp His Glu His Thr Arg Pro Asp Arg Asp
            100                 105                 110

Asn His Val Thr Ile Ile Arg Glu Asn Ile Gln Pro Gly Gln Glu Tyr
        115                 120                 125

Asn Phe Leu Lys Met Glu Pro Gly Glu Val Asn Ser Leu Gly Glu Arg
    130                 135                 140

Tyr Asp Phe Asp Ser Ile Met His Tyr Ala Arg Asn Thr Phe Ser Arg
145                 150                 155                 160

Gly Met Phe Leu Asp Thr Ile Leu Pro Ser Arg Asp Asp Asn Gly Ile
                165                 170                 175

Arg Pro Ala Ile Gly Gln Arg Thr Arg Leu Ser Lys Gly Asp Ile Ala
            180                 185                 190

Gln Ala Arg Lys Leu Tyr Arg Cys Pro Ala
        195                 200